# EUROPÄISCHE PATENTSCHRIFT

(11) **EP 1 314 783 B1**
(45) Veröffentlichungstag und Bekanntmachung des Hinweises auf die Patenterteilung: **19.11.2008**
(21) Anmeldenummer: 01127864.5
(22) Anmeldetag: 22.11.2001
(51) Int. Cl.: C12N 15/10, C12N 15/66, C12P 19/34

(54) **Nukleinsäure-Linker und deren Verwendung in der Gensynthese**
Nucleic acid linkers and their use in gene synthesis
Oligonucléotides et leur utilisation dans la synthèse de gènes

(43) Veröffentlichungstag der Anmeldung: 28.05.2003
(73) Patentinhaber: Sloning BioTechnology GmbH, 82178 Puchheim (DE)
(72) Erfinder: Schatz, Octavian, Dr., 85250 Altomünster (DE); O'Connell, Timothy, Dr., 82256 Fürstenfeldbruck (DE)
(74) Vertreter: Bohmann, Armin K.

(56) Entgegenhaltungen:
- EP-A- 0 245 130
- WO-A-00/75368
- WO-A-01/61036
- WO-A-01/75180
- WO-A-96/12014
- WO-A-99/47536
- SHIBATA Y ET AL: "Cloning full-length, Cap-Trapper-selected cDNAs by using the single-strand linker ligation method." BIOTECHNIQUES, Bd. 30, Nr. 6, Juni 2001 (2001-06), Seiten 1250-1254, XP002197302 ISSN: 0736-6205
- UNRAU PAUL ET AL: "Non-cloning amplification of specific DNA fragments from whole genomic DNA digests using DNA 'indexers'" GENE, ELSEVIER BIOMEDICAL PRESS. AMSTERDAM, NL, Bd. 145, Nr. 2, 1994, Seiten 163-169, XP002149819 ISSN: 0378-1119
- PADGETT K A ET AL: "Creating seamless junctions independent of restriction sites in PCR cloning" GENE, ELSEVIER BIOMEDICAL PRESS. AMSTERDAM, NL, Bd. 168, Nr. 1, 2. Februar 1996 (1996-02-02), Seiten 31-35, XP004042930 ISSN: 0378-1119
- KATO K: "DESCRIPTION OF THE ENTIRE MRNA POPULATION BY A 3' END CDNA FRAGMENT GENERATED BY CLASS IIS RESTRICTION ENZYMES" NUCLEIC ACIDS RESEARCH, OXFORD UNIVERSITY PRESS, SURREY, GB, Bd. 23, Nr. 18, 1. September 1995 (1995-09-01), Seiten 3685-3690, XP002008304 ISSN: 0305-1048
- VELCULESCU V E ET AL: "SERIAL ANALYSIS OF GENE EXPRESSION" SCIENCE, AMERICAN ASSOCIATION FOR THE ADVANCEMENT OF SCIENCE,, US, Bd. 270, Nr. 5235, 20. Oktober 1995 (1995-10-20), Seiten 484-487, XP001024449 ISSN: 0036-8075

## Beschreibung

Die vorliegende Erfindung betrifft einzelsträngige und doppelsträngige Nukleinsäuremoleküle zur Verwendung in einem Verfahren zur Herstellung einer Nukleinsäure, ein Verfahren zur Herstellung einer Nukleinsäure sowie einen Kit zur Herstellung einer Nukleinsäure.

Die Synthese von Nukleinsäuren findet in der modernen Biotechnologie vielfältige Anwendungen. Neben der Synthese vergleichsweise kurzer Nukleinsäuren wie Oligonukleotiden steht dabei die Synthese von mehreren Kilobasen großen Nukleinsäuren zunehmend im Vordergrund. Die dabei angewandten Methoden verwenden in der Regel für jedes Gen unterschiedliche synthetisch hergestellte Oligonukleotide von typischer Weise 40 bis 100 Nukleotiden Länge als Grundbausteine. Infolge der Vielzahl der notwendigen Reaktionsschritte enthalten diese, trotz vergleichsweise hoher Kopplungseffizienzen von ca. 98-99% pro Schritt sowohl Abbruchprodukte als auch Fehlsequenzen, die für die Qualität der zu synthetisierenden Nukleinsäuren nachteilig sind. Derartige Fehler sind vor allem dann nachteilig, wenn die zu synthetisierende Nukleinsäure eine codierende Sequenz darstellt und es infolge der Verschiebung des Leserasters zu verkürzten Transkriptions- oder Translationsprodukten kommt. Daher müssen die Oligonukleotidbausteine zusätzlich aufgereinigt werden, um diese Fehler auf ein vertretbares Maß zu reduzieren, da sonst komplexe Gensynthesen praktisch unmöglich sind.

Zu den im Stand der Technik bekannten Verfahren zählt beispielsweise das sogenannte "gap filling"-Verfahren, in welchem eine Vielzahl von Oligonukleotiden synthetisiert, aufgereinigt und anschließend paarweise oder in Untergruppen hybridisiert werden. Nach der Synthese der jeweiligen Gegenstränge mittels einer Klenow-Polymerase-Reaktion werden die einzelnen Fragmente miteinander ligiert. Die so entstandenen Ligationsprodukte können entweder als Teilfragmente kloniert oder zunächst mit außen liegenden Oligonukleotidprimern hybridisiert und in einer Polymerase-Kettenreaktion (PCR) amplifiziert werden. Alternativ können im Rahmen der sogenannten Kassettenmethode komplementäre Oligonukleotide miteinander hybridisiert und die so erhaltenen Genfragmente durch enzymatische oder chemische Ligation verknüpft werden. Nach Aufreinigung und/oder Klonierung können diese zu größeren Genabschnitten zusammengefügt werden. Beide Verfahren sind mit Nachteilen verbunden, so z. B. bedingt durch die mit zunehmender Länge der zu synthetisierenden Nukleinsäure zunehmend häufiger auftretenden Fehler in der Klenow-Polymerase-Reaktion oder in der Polymerase-Kettenreaktion.

Des Weiteren sind im Stand der Technik Verfahren bekannt, bei denen Oligonukleotide in einer Festphasensynthese miteinander verknüpft werden, um größere Nukleinsäuren aufzubauen. So beschreibt beispielsweise die internationale Patentanmeldung WO 99/47536 ein rekursives Verfahren, in dem einzelsträngige Oligo-nukleotide in einer definierten Orientierung sequenziell an ein immobilisiertes Startermolekül ligiert werden. Nachteilig ist bei diesem Verfahren die für größere Gensynthesen erforderliche hohe Anzahl der Einzelschritte, welche Prinzip bedingt zu geringen Ausbeuten sowie zur Anreicherung von Fehlsequenzen führen. Zudem müssen sämtliche für die Gensynthese eingesetzte Oligonukleotide für jede Synthese vorher neu synthetisiert werden. Eine Standardisierung dieses Verfahrens ist wegen des damit verbundenen enormen technischen Aufwands daher nur beschränkt möglich.

SHIBATA Y ET AL, BIOTECHNIQUES, Bd. 30, Nr. 6, Juni 2001 (2001-06), Seiten 1250-1254, beschreibt die Klonierung vollständiger cDNAs mittels einer einzelsträngigen Linker-Ligations-Methode.

Das US-Patent 5,710,000 beschreibt ein Verfahren zur Sequenzierung/Kartierung genetischer Marker in Polynukleotidsequenzen mittels Typ IIS-Restriktionsendonukleasen.

Die internationale Patentanmeldung WO 01/75180 betrifft ein sequenzabhängiges Verfahren zum Sortieren von cDNA-Molekülen.

UNRAU PAUL ET AL, GENE ESLEVIER BIOMEDICAL PRESS. AMSTERDAM, NL, Bd. 145, Nr. 2, 1994, Seiten 163-169, beschreibt ein Verfahren zur systematischen Amplifikation von spezifischen DNA-Fragmenten aus verdauter gesamt-DNA mittels DNA-"Indexer".

Die europäische Patentanmeldung EP 0 245 130 beschreibt Vektoren, welche Mittel zur enzymatischen Synthese von DNA-Fragmenten oder Nukleotidsequenzen umfassen, insbesondere tele-wirkende Restriktionszonen.

Die internationale Patentanmeldung WO 00/75368 beschreibt eine kombinatorische Festphasensynthese, in welcher in parallelen Reaktionsansätzen doppelsträngige Oligonukleotide, die eine Erkennungssequenz für ein Typ IIS-Restriktionsenzym enthalten, mit weiteren doppelsträngigen Oligonukleotiden, welche eine Erkennungssequenz für ein anderes Restriktionsenzym vom Typ IIS enthalten, ligiert werden und die Ligationsprodukte anschließend mit einem Typ IIS-Restriktionsenzym gespalten werden. Durch mehrfaches Wiederholen wird somit iterativ eine definierte Nukleinsäure aufgebaut. Dieses Verfahren hat gegenüber anderen, auf Ligation von Oligonukleotiden beruhenden Verfahren den Vorteil, dass die verwendeten Oligonukleotide Erkennungssequenzen für verschiedene Typ IIS-Restriktionsenzyme enthalten, die eine sequenzunabhängige Kombination parallel ligierter Teilfragmente erlauben. Dabei können beliebige Teilfragmente aus einer standardisierten Nukleinsäurebibliothek mit einer definierten Anzahl von Elementen generiert werden. Die Anzahl der diese Bibliothek aufbauenden Elemente hängt dabei von der Länge der durch das jeweilige Restriktionsenzym erzeugten Überhänge ab. Bei einem Überhang von beispielsweise vier Nukleotiden, besteht eine komplette Bibliothek aus insgesamt 65.536 Elementen. Diese Zahl ergibt sich aus der Anzahl der Sequenzvarianten, die bei einer Länge des Überhanges von vier Nukleotiden (4⁴ = 256) existieren, multipliziert mit der Anzahl der Sequenzvarianten für die vier direkt angrenzenden Nukleotide, die den Überhang beim nächsten Ligationsschritt bilden (4⁴ x 4⁴ = 65.536).

Obwohl dieses Verfahren eine sequenzunabhängige Verknüpfung beliebiger parallel hergestellter Genfragmente gestattet und somit die Grundlage für eine Automatisierung liefert, ist die Anzahl der erforderlichen Oligonukleotide zum Aufbau einer entsprechenden Bibliothek, auf die im Rahmen der Synthese zugegriffen wird, noch immer vergleichsweise hoch. Ein weiterer Aspekt, den es dabei zu berücksichtigen gilt, ist die Länge der Oligonukleotide, die in einem derartigen Verfahren verwendet werden. (typischer Weise etwa 20 bis 40 Nukleotide). Infolge dessen enthält eine komplette Bibliothek von Oligonukleotiden in dem geschilderten Fall insgesamt 65.536 x 40 ≈ 2.6 Mio. Nukleotide.

Trotz der mit dem Verfahren nach WO 00/75368 unmittelbar verbundenen Vorteile besteht demnach im Stand der Technik weiterhin der Bedarf, ein Verfahren zur Synthese von Nukleinsäuren und Mittel zur Durchführung desselben bereitzustellen, welche auf eine Oligonukleotid-Bibliothek geringerer Komplexität zurückgreifen. Insbesondere soll der Aufwand zur Erzeugung einer kompletten Bibliothek, vor allem hinsichtlich der Anzahl der dazu erforderlichen Nukleotide, gegenüber dem Stand der Technik deutlich verringert werden ohne dabei Nachteile wie eine geringere Ligationseffizienz (z.B. bei der Verwendung von Überhängen von nur ein oder zwei Nukleotiden Länge) in Kauf nehmen zu müssen. Es ist dabei eine weitere Aufgabe der Erfindung ein Verfahren bereitzustellen, welches einen noch geringeren Anteil an Abbruch- und Fehlsequenzen gewährleistet sowie die gleichzeitige Synthese mehrerer Genvarianten erlaubt.

Erfindungsgemäß wird diese Aufgabe durch den Gegenstand der unabhängigen Ansprüche gelöst. Berorzugte Ausführungsformen ergeben sich aus den abhängigen Ansprüchen.

Der vorliegenden Erfindung liegt die überraschende Erkenntnis zugrunde, dass die mit dem Verfahren nach WO 00/75368 mögliche sequenzunabhängige Verknüpfung beliebiger Oligoukleotide und damit die Synthese beliebiger Nukleinsäuren noch weiter verbessert werden kann, weil durch die geringere Komplexität der Oligonukleotidbibliothek (wie sie nach dem Verfahren der vorliegenden Anmeldung generiert werden kann) eine höhere Standardisierung der darin enthaltenen Oligonukleotide möglich ist. Insbesondere kann durch die nach dem vorliegenden Verfahren mögliche Verkürzung der Oligonukleotide eine höhere Ausbeute und eine größere Reinheit erreicht werden, wodurch die Genauigkeit der Gensynthese nochmals gesteigert werden kann. In dem Verfahren nach WO 00/75368 werden zwei verschiedene Klassen von Oligonukleotiden verwendet, wobei sich die beiden dort als Anchor- und Splinker-Oligonukleotid bezeichneten Klassen durch das Vorhandensein zweier unterschiedlicher Erkennungssequenzen für Restriktionsenzyme vom Typ IIS und die einzelnen Elemente innerhalb der Oligonukleotidklasse in der Sequenz des jeweiligen Überhanges unterscheiden. Um beliebige Gene nach diesem Verfahren herstellen zu können, muss eine Bibliothek von Oligonukleotiden zur Verfügung stehen, die alle möglichen Sequenzvarianten der zu Grunde liegenden Oligonukleotide enthält. Gemäß der vorliegenden Erfindung können die jeweils benötigten Anchor und Splinker Oligonukleotide unter Verwendung von insgesamt drei standardisierten Elementen bei Bedarf in einer Abwandlung der in der Anmeldung WO 00/75368 beschriebenen Methode hergestellt werden. Bei den drei Elementen handelt es sich zum einen um zwei Klassen von Oligonukleotiden, die sich im Wesentlichen durch die Anwesenheit von zumindest jeweils einer Erkennungssequenz (oder der dazu komplementären Sequenz) für ein Restriktionsenzym vom Typ IIS unterscheiden, und ein hierin im Folgenden auch als Linker bezeichnetes einzelsträngiges Oligonukleotid.

Restriktionsenzyme vom Typ IIS sind zeichnen sich dadurch aus, dass sie mit zwei diskreten Stellen einer doppelsträngigen DNA in Wechselwirkung treten. Eine der beiden Stellen ist die Erkennungsstelle, die typischer Weise eine Länge von 4 bis 7 Basenpaare aufweist. Die andere Stelle ist die Spaltungsstelle, die gewöhnlich 1 bis 20 Basenpaare von der Erkennungsstelle entfernt ist. Die Erkennungsstellen der Restriktionsenzyme sind entweder vollständig oder teilweise asymmetrisch.

Die beiden Klassen von jeweils mindestens eine Erkennungssequenz für ein Restriktionsenzym vom Typ IIS (oder der dazu komplementären Sequenz) aufweisenden Oligonukleotiden umfassen bevorzugter Weise jeweils die folgenden Strukturelemente in 3'-5'-Richtung: einen einzelsträngigen Bereich, einen doppelsträngigen Bereich und, optional, eine Schleife (engl. Loop). Diese Sekundärstruktur bildet sich in Folge der Primärstruktur der entsprechenden einzelsträngigen Nukleinsäure aus.

Dem einzelsträngigen Bereich kommt insoweit eine besondere Bedeutung bei, als dass dieser vollständig oder teilweise die Erkennungssequenz für ein Restriktionsenzym vom Typ IIS darstellt oder enthält. Alternativ kann der einzelsträngige Bereich auch eine Sequenz umfassen, die komplementär ist zu der vollständigen Erkennungssequenz für das Restriktionsenzym vom Typ IIS oder einen Teil derselben. Die minimale Länge des einzelsträngigen Bereichs kann dabei ein einzelnes Nukleotid sein. Die maximale Länge dieses Bereichs ist dabei grundsätzlich nicht beschränkt, jedoch ist es bevorzugt, wenn diese neben der Erkennungssequenz keine weiteren Nukleotide enthält, da sich dadurch die Länge des Oligonukleotids letztlich nur unnötig verlängert, was mit einem erhöhten Syntheseaufwand und damit einhergehend einem höheren Risiko von Fehlsequenzen verbunden ist. Andererseits sollte der verwendete Überhang eine stabile Hybridisierung mit dem einzelsträngigen Linker erlauben. Als optimal sind daher Längen zwischen 3 und 7 Nukleotiden anzusehen.

Der doppelsträngige Bereich kann aus der Rückfaltung des Oligonukleotids auf sich selbst entstehen. Die Länge des doppelsträngigen Bereichs beträgt bevorzugter Weise drei bis neun Nukleotide. Grundsätzlich ist die spezifische Abfolge der Nukleotide in diesem Bereich beliebig, sofern zumindest unter den Bedingungen der Nukleinsäuresynthese, bei der das Oligonukleotid verwendet werden soll, eine stabile Hybridisierung zwischen den komplementären Nukleotiden des Oligonukleotids erfolgt. Dabei ist die Ausbildung von GC-Paarungen in Folge der erhöhten Stabilität einer GC-Paarung gegenüber einer AT-Paarung bevorzugt. Wenn TypIIS Restriktionsenzyme mit weiter entfernten Schnittstellen zum Einsatz kommen, können die Erkennungssequenzen für diese Enzyme sowohl vollständig als auch teilweise in dem doppelsträngigen Bereich enthalten sein.

Die Schleife des Oligonukleotids kann aus einer beliebigen Abfolge von Nukleotiden gebildet werden. Bei der Auswahl der Abfolge gilt es jedoch zu gewährleisten, dass keine Wechselwirkung mit anderen Sequenzen erfolgt und damit die Ausbildung der Schleife oder der anderen das Oligonukleotid aufbauenden (Sekundär-)Strukturen gestört wird. Bevorzugt werden Pyrimidine und ganz besonders Thymidine verwendet, da diese relativ klein sind und die entstehende Schleifenstruktur stabil ist. Cytosin geht mit Guanosin eine stabilere Basenpaarung ein, wodurch die Ausbildung alternativer Sekundärstrukturen begünstigt wird. Die Verwendung von bevorzugter Weise vier Pyrimidinen und bevorzugt Thymidinen ergibt sich aus der Tatsache, dass die Ringspannung bei weniger als 4 Nukleotiden zu groß wird (wodurch die angrenzenden doppelsträngigen Regionen aufgelöst werden können). Mehr als 4 Nukleotide haben hingegen keine nennenswerte Auswirkung auf die Ringspannung und wären daher überflüssig.

Die vorstehend beschriebene Klasse von Oligonukleotiden wird hierin als einteiliges oder selbstkomplementäres Oligonukleotid bezeichnet. Eine hierzu alternative Klasse von Oligonukleotiden, die jedoch die gleiche Funktion aufweist, insbesondere im Rahmen ihrer Verwendung zur Gen- oder Nukleinsäuresynthese, zeichnet sich dadurch aus, dass die Schleife nicht vorhanden ist. Diese Klasse von Oligonukleotiden kann dadurch erzeugt werden dass zwei einzelsträngige Oligonukleotide miteinander hybridisiert werden, wobei es zur Ausbildung des doppelsträngigen und des einzelsträngigen Bereiches kommt. In Folge des Fehlens der Schleife sind bei der Auswahl und Ausgestaltung der beiden zu hybridisierenden einzelsträngigen Oligonukleotide sowohl hinsichtlich der Sequenz als auch der Modifikation am 3'- bzw. 5'-Ende eine Reihe von Überlegungen anzustellen bzw. Maßnahmen zu ergreifen. Wie im Folgenden noch erläutert wird, muss bei dieser Form von zweiteiligem Oligonukleotid gewährleistet sein, dass es zu keiner fehlerhaften Paarung mit dem Linker kommt. Weiterhin muss gewährleistet sein, dass die infolge des Fehlens der Schleife freiliegenden Enden der beiden hybridisierten Einzelstränge kein Substrat für eine Polymerase oder Ligase darstellen. Dies kann beispielsweise dadurch gewährleistet werden, dass eine Aminoverbindung, ein Succinylester, ein Fluoreszenzfarbstoff oder ein Digoxigeninrest an die endständigen 5' bzw. 3' Phosphatgruppen gekoppelt wird.

Beide Alternativen von Oligonukleotidklassen, d.h. sowohl die selbstkomplementäre Form wie auch die zweiteilige Form der Oligonukleotide, können über eine Modifikation verfügen, die es erlaubt, das Oligonukleotid an eine Festphase zu koppeln. Im Falle der selbstkomplementären Form erfolgt diese Modifikation bevorzugter Weise im Bereich der Schleife. Mit dieser Modifikation wird gewährleistet, dass das Oligonukleotid oder eine dieses umfassende Nukleinsäure von anderen Verbindungen abgetrennt werden kann. Die Modification selbst kann durch den Fachleuten auf dem Gebiet bekannte Maßnahmen erfolgen. Beispielhafte Modifikationen sind der Einbau niedermolekularer Verbindungen wie Biotin, Digoxigenin, Fluoresceinisothiocyanat (FITC), Aminoverbindungen oder Succinylester. Die Oberfläche wird in der Folge Moleküle aufweisen, die eine in der Regel spezifische Wechselwirkung mit der Modifikation zu Immobilisierungszwecken erlaubt.

Der Linker als drittes standardisiertes Element ist chemisch betrachtet ebenfalls ein Oligonukleotid. Der Linker besteht grundsätzlich aus zwei Teilsequenzen. Die erste (konstante) Teilsequenz, hierin auch als Teil A bezeichnet, umfasst mindestens die Erkennungssequenz eines Restriktionsenzyms vom Typ IIS oder einen Teil davon. Alternativ kann Teil A die zu der Erkennungssequenz des Restriktionsenzyms vom Typ IIS komplementäre Sequenz oder einen Teil davon umfassen. Die zweite (variable) Teilsequenz des Linkers, hierin auch als Teil B bezeichnet, stellt eine beliebige, aber definierte Folge von Nukleotiden dar. Die spezifische Ausgestaltung des konstanten Teils A des Linkers hängt dabei von den jeweiligen Restriktionsenzymen vom Typ IIS ab, die im Rahmen des Syntheseverfahrens verwendet werden bzw. auf die die beiden Klassen von Oligonukleotiden abstellen (in der Regel ist dieser vollständig komplementär zu dem einzelsträngigen Bereich mindestens eines der oben beschriebenen Oligonukleotide) .

Im Folgenden wird die Ausgestaltung eines Oligonukleotids und des entsprechenden Linkers unter der Annahme dargestellt, dass der einzelsträngige Bereich des Oligonukleotids die Erkennungssequenz des Restriktionsenzyms vom Typ IIS vollständig umfasst. Infolge der Eigenschaft dieser Restriktionsenzyme, dass die Schnittstelle außerhalb der Erkennungsstelle liegt, d.h. die Erkennungsstelle durch die enzymatische Aktivität nicht zerstört wird, und darüber hinaus der Schnitt des Restriktionsenzyms in einer definierten Entfernung von seiner Erkennungsstelle unabhängig von der zu schneidenden Sequenz erfolgt, kann der Linker so gestaltet werden, dass der konstante Teil A komplementär ist zu der Erkennungssequenz des Restriktionsenzyms, welches den einzelsträngigen Bereich des Oligonukleotids ausbildet. In Folge dieser Komplementarität kann eine Hybridisierung von Oligonukleotid und Linker erfolgen. Nachdem der Linker neben Teil A noch Teil B umfasst, bildet Teil B des Linkers nach der Hybridisierung mit dem Oligonukleotid einen Überhang oder ein überstehendes Ende. Die gleiche Struktur aus Oligonukleotid und Linker bildet sich aus, wenn das Oligonukleotid in seinem einzelsträngigen Bereich eine Sequenz aufweist, die komplementär ist zur Erkennungssequenz des Restriktionsenzyms und Teil A des Linkers die Erkennungssequenz des Restriktionsenzyms darstellt. Es ist dabei nicht erforderlich, dass entweder der einzelsträngige Bereich des Oligonukleotids oder der konstante Teil A des Linkers die vollständige Erkennungssequenz bzw. deren komplementäre Sequenz umfasst. Vielmehr ist es auch im Rahmen der Erfindung, wenn die Erkennungssequenz oder die dazu komplementäre Sequenz des Restriktionsenzyms durch Teile des doppelsträngigen Bereiches und des einzelsträngigen Bereiches insgesamt ausgebildet wird. In diesem Fall wird beispielsweise Teil A des Linkers nur den Teil der Erkennungssequenz des Restriktionsenyms umfassen, der komplementär zu dem im einzelsträngigen Bereich des Oligonukleotids enthaltenen Teil ist.

Die Länge von Teil B des Linkers wird durch das jeweils verwendete Restriktionsenzym und genauer durch die Länge des von ihm erzeugten Überhanges bestimmt. Die nachfolgende Tabelle 1 gibt eine Übersicht über verschiedene Restriktionsenzyme vom Typ IIS mit ihren Erkennungssequenzen und den erzeugten Überhängen wider. Dabei stellt die Tabelle die Paare von Restriktionsenzymen dar, die vorteilhafter Weise in dem erfindungsgemäßen Nukleinsäuresyntheseverfahren zusammen mit den standardisierten Elementen verwendet werden.

**Tabelle 1: Beispielhafte Ausgestaltung von Oligonukleotid 1 und Oligonukleotid 2 sowie des erfindungsgemäßen Linkers in Abhängigkeit von dem spezifisch verwendeten Restriktionsenzympaar vom Typ IIS.**

| **Restriktionsenzympaar** | **Oligonukleotid 1-(5'-3')** | **Oligonukleotid 2 (5'-3')** | **Linker (5'-3')** |
|---|---|---|---|
| Eeo31I/Esp3I | CGN₁₋₉X₁₋₉N'₁₋₉*CGTCT*CN | CCN₁₋₉X₁₋₉N' ₁₋₉*GGTCTC*N | NNNNN' GAGA |
| | (SEQ. ID.No 14) | (SEQ. ID.No 16) | (SEQ. ID.No.18) |
| BbsI/Acc36I | TTCN₁₋₉X₁₋₉N'₁-₉*GAAGAC*NN | CAGGTN₁₋₉X₁₋₉N'₁-₉*ACCTGC*N₄ | a) NNNNN'₂GTC |
| | (SEQ. ID.No. 15) | (SEQ. ID.No. 17) | (SEQ ID No. 19) |
| | | | b) NNNNN'₄G |
| | | | (SEQ ID No. 20) |
| ECo31I/Esp3I (bipartite) | N₁-₉CGTCTCN | CCN'₁₋₉ | N₁₋₉CGAGA |
| | (SEQ. ID.No.21) | (SEQ. ID.No.23) | (SEQ. ID.No. 25) |
| | CGN'₁₋₉ | N₁₋₉GGTCTCN | |
| | (SEQ. ID.No.22) | (SEQ. ID.No. 24) | |
| BbsI/Acc36I (bipartite) | N₁-₉GAAGACNN | CAGGTN₁₋₉ | NNNNNNGTC |
| | (SEQ. ID.No. 26) | (SEQ. ID.No. 28) | (SEQ. ID.No. 30) |
| | TTCN₁₋₉ | N'₁₋₉ACCTGCNNNN | NNNNN'₄G |
| | (SEQ.ID.No. 27) | (SEQ. ID.NO 29) | (SEQ. ID.No. 31) |

| | | | |
|---|---|---|---|
| Dabei bedeutet: N ein beliebiges der Nukleotide A, G, C oder T; N' das jeweils zu N an der korrespondierenden Position im Gegenstrang komplementäre Nukleotid X ein beliebiges nukleotidisches oder nichtnukleotidisches Element (ggf. mit einer entsprechenden Modifikation), welches zu einer Kettenbildung befähigt ist. | | | |

Die Subskriptnummern geben die Anzahl der jeweiligen Elemente an.

**Tabelle 2: Beispielhafte Kombination der Erkennungssequenzen von Restriktionsenzymen vom Typ IIS in den Oligonukleotiden der Klasse 1 und 2**

| **Erkennungssequenz der Klasse 1** | **Erkennungssequenz der Klasse 2** |
|---|---|
| CGTCTCN^NNNN_ (Esp3I, BsmBI) (SEQ.ID.No1) | GGTCTCN^NNNN_(BsaI, Eco31I...) |
| GGTCTCN^NNNN_(BsaI, Eco31I,..) (SEQ.ID.No. 2) | CGTCTCN^NNNN_(Esp3I, BsmBI) |
| GAAGACNN^NNNN_(BbsI, BpiI...) (SEQ.ID.No.3) | ACCTGCNNNN^NNNN_(BspMI, Acc36I) |
| ACCTGCNNNN^NNNN_(BspMI, Acc36I) (SEQ.ID.No.4) | GAAGACNN^NNNN_(BbsI, BpiI...) |
| GCAGTG_NN^ (BtsI) (SEQ.ID.No.5) | GCAATG_NN^ (BsrDI, Bse3DI, ..) |
| GCAATG_NN^ (BsrDI, Bse3DI, ..) (SEQ.ID.No.6) | GCAGTG_NN^ (BtsI) |
| GTATCCNNNNN_N^ (BciVI, BfuI) (SEQ.ID.No.7) | ACTGGGNNNN_N^ (BfiI, BmrI) |
| ACTGGGNNNN_N^ (BfiI, BmrI) (SEQ.ID.No.8) | GTATCCNNNNN_N^ (BciVI, Bful) |
| GGCGGANNNNNNNNN_NN^ (EciI) (SEQ.ID.No.9) | GAGGAGNNNNNNNN_NN^ (BseRI) |
| GAGGAGNNNNNNNN_NN^ (BseRI) (SEQ.ID.No.10) | GGCGGANNNNNNNNN_NN^ (EciI) |
| CACCTGCNNNN^NNNN_(AarI) <SEQ.ID.No.11 | CAGCTCNNNNNNNNN^NNNN_(AceIII) |
| CAGCTCNNNNNNN^NNNN_(AceIII) (SEQ.ID.No.12) | CACCTGCNNNN^NNNN_(AarI) |
| GCTCTTCN^NNN_(SapI) (SEQ.ID.No.13) | - (Adapter Linker erforderlich) |

| | |
|---|---|
| Dabei bedeutet: N ein jedes der Nukleotide A, G, C oder T; ^ die Schnittstelle im "oberen" Strang, d.h. 5'->3' von links nach rechts _die Schnittstelle im "unteren" Strang, d.h.5'->3' von rechts nach links | |

Bevorzugte Paarungen eines ersten und eines zweiten Restriktionsenzym vom Typ IIS zu Zwecken der Verwendung der beiden Klassen von Oligonukleotiden und des Linker-Moleküls für die Synthese einer Nukleinsäure, bevorzugter Weise einer DNA sind die folgenden: Eco31I/Esp3I (37°C), BsaI/BsmBI (50°C), BsmBI/BsaI (55°C), BbsI/BspMI (37°C), BspMI/BbsI (37°C) BsrDI/BtsI (65°C), BtsI/BsrDI (37°C), BciVI/Bmrl (37°C), AarI/AceIII (37°C), EciI/BseRI (37°C) und BmrI/BciVI (37°C). (Die in Klammern angegebenen Temperaturen sind die bei der jeweiligen Paarung verwendeten Inkubationstemperaturen.). Die Isoschizomeren zu diesen Enzymen (BsaI: Bo31, Eco31I; BsmB1: Esp3I; BbsI: Bpil,BpuAI; BspMI: Acc36I; BsrDI:Bse3DI, BseMI; BmrI: BfiI) stellen potenzielle Ausweichkandidaten dar; z.T. werden diese aus klonierten Vektoren überexprimiert und in höherer Ausbeute bzw. Reinheit produziert. Isoschizomere werden auch dann bevorzugt verwendet, wenn die Lagerfähigkeit eines Enzyms verglichen mit seinem Isoschizomeren beschränkt ist.

Wird nun beispielsweise BsaI als Restriktionsenzym verwendet, wird ein Überhang aus vier Nukleotiden erzeugt, welcher eine beliebige Sequenz aufweisen kann. Da an einer jeden der vier Nukleotidpositionen im Prinzip alle vier Nukleotide (A, G, C, T) stehen können, kann mit insgesamt 256 Linkern eine jede aus vier Nukleotiden bestehende Sequenz dargestellt werden. Ein derartiger Linker kann sodann mit einem Oligonukleotid infolge der Komplementarität der Sequenzen von Teil A des Linkers mit dem einzelsträngigen Teil des Oligonukleotides hybridisiert werden. Beträgt der durch das Restriktionsenzym erzeugte Überhang zwei Nukleotide, wird die entsprechende Linker-Bibliothek 16 Elemente, bei einem Überhang von drei Nukleotiden 64 Elemente, bei einem Überhang von fünf Nukleotiden 1024, bei einem Überhang von sechs Nukleotiden 4096 und bei einem Überhang von sieben Nukleotiden 16384 Elemente umfassen.

Bei derartigen Bibliotheken ist anzumerken, dass der Teil B bei Betrachtung des einzelnen Linkers scheinbar eine beliebige Sequenz aufweist, jedoch in der Gesamtheit die Linker einer entsprechenden Bibliothek den gesamten, durch die Länge des Überhanges definierten Sequenzraum abdecken und in einem jeden Fall eine definierte, d. h. **nicht randomisierte** Abfolge von Nukleotiden umfassen.

Die vorstehende Konzeption der Paarbildung aus einem Oligonukleotid der ersten Klasse, hierin auch als erstes Oligonukleotid bezeichnet, und einem entsprechenden Linker, definiert durch ein spezifisches Restriktionsenzym, wobei das die Klasse des Oligonukleotids definierende Restriktionsenzym das gleiche ist, wie das die Klasse des Linkers definierende Restriktionsenzym vom Typ IIS, kann nun in gleicher Art und Weise durchgeführt werden für ein Paar aus Linker und Oligonukleotid einer zweiten Klasse, die durch ein anderes, zweites Restriktionsenzym vom Typ IIS definiert werden, und sodann Komplexe aus Linker und Oligomer hergestellt werden. Nachdem in diesem Falle ein anderes Restriktionsenzym vom Typ IIS verwendet wird, wird sich Teil A des Linkers von dem vorstehend im Zusammenhang mit dem ersten Restriktionsenzym vom Typ IIS beschriebenen Linker unterscheiden. Teil B wird jedoch, wiederum in Abhängigkeit von der Länge des durch das Restriktionsenzym vom Typ IIS erzeugte Ende ausgestaltet sein und insgesamt einen entsprechenden Sequenzraum definieren.

Durch das vorstehend beschriebene Vorsehen liegen somit typischer Weise zwei Linker-Bibliotheken vor, die bedingt durch die Spezifität bzw. Komplementarität der Erkennungsstelle des jeweiligen Restriktionsenzyms vom Typ IIS mit jeweils einem korrespondierenden Oligonukleotid hybridisieren können. Nach der Hybridisierung und ggf. Ligierung des Linkers mit dem Oligonukleotid wird dieser typischer Weise phosphoryliert und der durch Teil B des Linkers generierte Überhang mittels einer Polymerase aufgefüllt, so dass der Komplex aus Oligonukleotid und Linker nun als glattendiges Oligonukleotid vorliegt. Dieser Vorgang wird für das Oligonukleotid der zweiten Klasse und den entsprechenden Linker (der zweiten Klasse) wiederholt. Anschließend werden die beiden um den Linker verlängerten glattendigen Oligonukleotide miteinander ligiert und anschließend mit einem der beiden Restriktionsenzyme vom Typ IIS gespalten. Infolgedessen kommt es zu einer Verlängerung des einen oder des anderen Oligonukleotids. Die Anzahl der angefügten Nukleotide wird dabei durch die Länge des Überhanges bestimmt, welcher von dem jeweils für die Spaltung der beiden ligierten glattendigen Oligonukleotide verwendeten Enzym produziert wird.

Der gezielte Aufbau einer definierten Nukleinsäure wird durch Anwendung und Wiederholung des oben beschriebenen schematischen Reaktionsgeschehens dadurch möglich, dass aus der Linker-Bibliothek jene Linker gewählt werden, die als Teil B die Sequenz enthalten, die zu einer bereits bestehenden oder aufzubauenden Nukleinsäure hinzugefügt werden soll. Nach Spaltung des Ligationsproduktes aus den beiden glattendigen Oligonukleotiden erhält man ein abgespaltenes Oligonukleotid, welches in der Gensynthese nach dem Sloning-Verfahren, wie sie Gegenstand der internationalen Patentanmeldung WO 00/75368 ist, eingesetzt werden kann. In diesem Verfahren werden größere Gene dadurch hergestellt, dass zunächst in parallelen Reaktionsansätzen Teilfragmente durch sequenzielle Ligationen von unmodifizierten doppelsträngigen Oligonukleotiden (sogenannten Splinkern) an ein über eine Modifikation immobilisierbare Oligonukleotide (sogenannte Anchor) aufgebaut werden. Die dabei entstehenden Ligationsprodukte werden nach jedem Schritt mit dem Restriktionsenzym gespalten, dessen Erkennungssequenz in den anligierten Splinker-molekülen enthalten ist. Dadurch verbleibt jeweils nur der variable Teil der Splinker an dem Anchor Molekül, während der konstante Teil durch das Restriktionsenzym abgetrennt und durch einen Waschschritt aus dem Reaktionsansatz entfernt wird. Je nach der zu synthetisierenden Teilsequenz wird für jeden Einzelschritt der hierfür benötigte Splinker aus einer Bibliothek aller Splinker ausgewählt. Anschließend wird die eine Hälfte der so erhaltenen Fragmente mit dem Anchor-spezfischen Restriktionsenzym behandelt, die andere Hälfte der Fragmente mit dem Splinker-spezifischen Enzym. Jedes dieser Fragmente weist nun einen einzelsträngigen Überhang auf, welcher komplementär zu dem Überhang des in der Abfolge der zu synthetisierenden Gensequenz nächsten Fragments ist. Durch Ligation der nebeneinander liegenden Fragmente (sogenannte Transposition) verdoppelt sich die Länge der nunmehr vorliegenden Fragmente, während sich die Anzahl der Fragmente halbiert. Mit jeder weiteren Transposition verdoppelt sich die Länge der Teilfragmente bis schließlich nur noch ein Fragment übrig ist, welches im Normalfall die gesamte zu synthetisierende Gensequenz enthält.

In seiner Allgemeinheit formuliert, umfasst dieses Verfahren, das hierin auch allgemein als das Sloning-Verfahren bezeichnet wird, somit die folgenden Schritte:
a) Bereitstellen eines Oligonukleotids, das hergestellt ist durch die folgenden Schritte:
   aa) Kopplung eines Oligonukleotids mit einem Ende an eine feste Matrix, wobei die Kopplung über eine Modifikation erfolgt, und das Oligonukleotid eine Erkennungssequenz für ein TypIIS Restriktionsenzym enthält, welches außerhalb seiner Erkennungssequenz schneidet,
   ab) Zugabe eines weiteren, mindestens teilweise doppelsträngigen Oligonukleotids mit einer anderen Erkennungssequenz für ein TypIIS Restriktionsenzym, welches außerhalb seiner Erkennungssequenz schneidet, als in Schritt aa), wobei dieses Oligonukleotid nicht an die Matrix binden kann,
   ac) Ligation der Oligonukleotide aus Schritt aa) und ab) in der durch die Blockierung der nicht zu ligierenden Enden festgelegten Orientierung,
   ad) Entfernen nicht verbrauchter Reaktanden sowie Enzyme,
   ae) Spaltung des Ligationsprodukts aus Schritt ac) mit einem TypIIS Restriktionsenzym, welches außerhalb seiner Erkennungssequenz schneidet, wobei die Spaltung in der Nukleinsäuresequenz des Oligonukleotids aus Schritt ab) stattfindet,
   af) Abtrennen des Reaktionsgemisches vom in Schritt ae) erhaltenen verlängerten Oligonukleotid aus Schritt aa),
   ag) mindestens einmaliges Wiederholen der Schritte ab) bis af),
b) Bereitstellen eines weiteren Oligonukleotids, das hergestellt ist durch die Schritte:
   ba) Koppelung eines Oligonukleotids mit einem Ende an eine feste Matrix, wobei die Kopplung über eine Modifikation erfolgt, und das Oligonukleotid eine Erkennungssequenz für ein TypIIS Restriktionsenzym enthält, welches außerhalb seiner Erkennungssequenz schneidet,
   bb) Zugabe eines weiteren, mindestens teilweise doppelsträngigen Oligonukleotids mit einer anderen Erkennungssequenz für ein TypIIS Restriktionsenzym, welches außerhalb seine Erkennungssequenz schneidet, als in Schritt ba), wobei dieses Oligonukleotid nicht an die Matrix binden kann,
   bc) Ligation der Oligonukleotide aus Schritt ba) und bb) in der durch die Blockierung der nicht zu ligierenden Enden festgelegten Orientierung,
   bd) Entfernen nicht verbrauchter Reaktanden sowie Enzyme,
   be) Spaltung des Ligationsproduktes aus Schritt bc) mit einem TypIIS Restriktionsenzym, welches außerhalb seine Erkennungssequenz schneidet, wobei die Spaltung im Oligonukleotid in Schritt bb) stattfindet,
   bf) Abtrennen des so verlängerten Nukleinsäuremoleküls vom Reaktionsgemisch,
   bg) mindestens einmaliges Wiederholen der Schritte bb) bis bf), wobei im Anschluss an die letzte Ligation in Schritt bc) und Entfernen nicht verbrauchter Reaktanden sowie Enzyme das Ligationspordukt mit einem TypIIS Restriktionsenzym geschnitten wird,
   wobei die Spaltung in Oligonukleotid in Schritt ba) stattfindet,
c) Ligation der Oligonukleotide aus Schritt a) und b) in der durch die Blockierung der nicht zu ligierenden Enden festgelegten Orientierung,
d) Entfernen nicht verbrauchter Reaktanden sowie Enzyme,
e) Spaltung des Ligationsproduktes aus Schritt c) mit einem TypIIS Restriktionsenzym, welches außerhalb seiner Erkennungssequenz schneidet, wobei die Spaltung im Oligonukleotid aus Schritt a) oder b) stattfindet,
f) Abtrennen des so verflängerten Nukleinsäuremoleküls vom Reaktionsgemisch.

Der Begriff der festen Matrix, wie er hierin verwendet wird, bezeichnet allgemein eine jegliche Oberfläche, an der eine Kopplung mindestens einer der Reaktanten stattfinden kann. Insbesondere zählen hierzu Oberflächenformen wie Filter, Folien, Membranen, Chips, Platten, Kügelchen (engl. beads) und Säulen. Diese Oberflächenformen können aus einem der folgenden Materialien hergestellt sein: Polymere wie Kunststoffe, beispielsweise Polystyrol, Polyacetat, Polyacrylamide, Polyvinylidenfluorid, Agarose, Sepharose, Cellulose; Silizium, Glas (Silikatglas) und Kieselgel. Diese Materialien können in einer oder mehreren dem Fachmann bekannten Art und Weisen modifiziert werden.

Die Kopplung kann auf Seiten der Oligonukleotide dadurch erfolgen, das eine Modifizierung intern, d.h. an einem nicht-terminalen Nukleotid des Polynukleotids, oder terminal, d.h. an einem terminalen Nukloetid, vorliegt. Letzteres wird insbesondere dann möglich sein, wenn das Oligonukleotid als eine zweiteilige (engl. bipartite) Struktur vorliegt. Derartige Modifizierungen, die eine Kopplung eines Oligonukleotids an eine Oberfläche, insbesondere eine modifizierte Oberfläche erlauben, sind den Fachleuten auf dem Gebiet bekannt und umfassen beispielsweise Biotin, Iminobiotin, Digoxygenin, Sulfhydrylgruppen, Diclycohexylcarbodiimid, Fluorescein, Acridin und Rhodamin.

Die Kopplung kann auf Seiten der festen Matrix durch eine oder mehrere der folgenden Modifizierungen erfolgen: Avidine, wie Streptavidin, monomeres Avidin, an den Tyrosinresten modifiziertes Avidin; Antikörper, insbesondere solche, die gegen die vorstehend genannten Verbindungen gerichtet sind, und Sulfhydrylgruppen.

Es ist im Rahmen der Fähigkeiten der Fachleute auf dem Gebiet, geeignete Kombinationen der vorstehenden Modifizierungen der Reaktionspartner zu bestimmen.

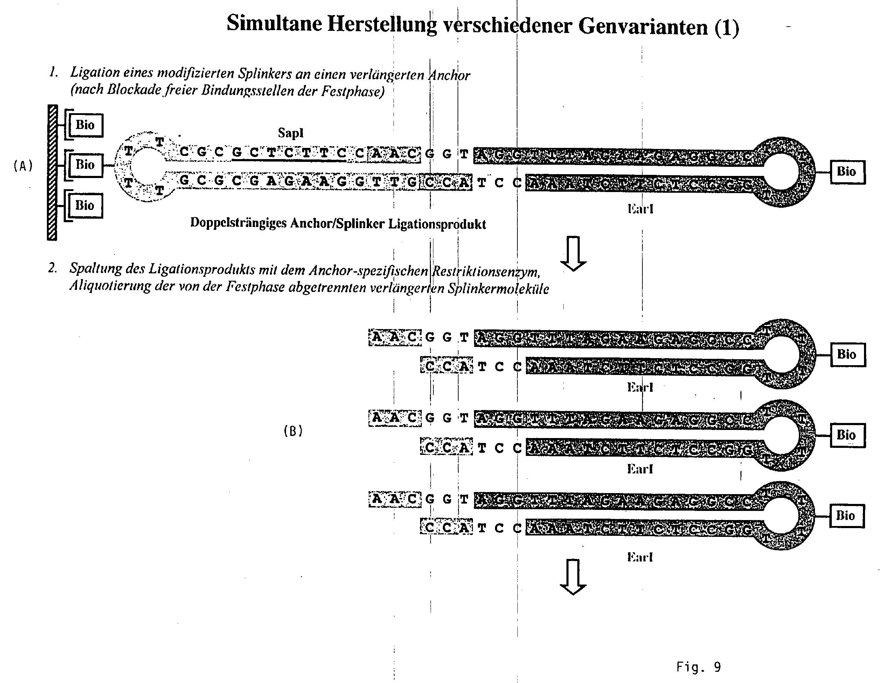

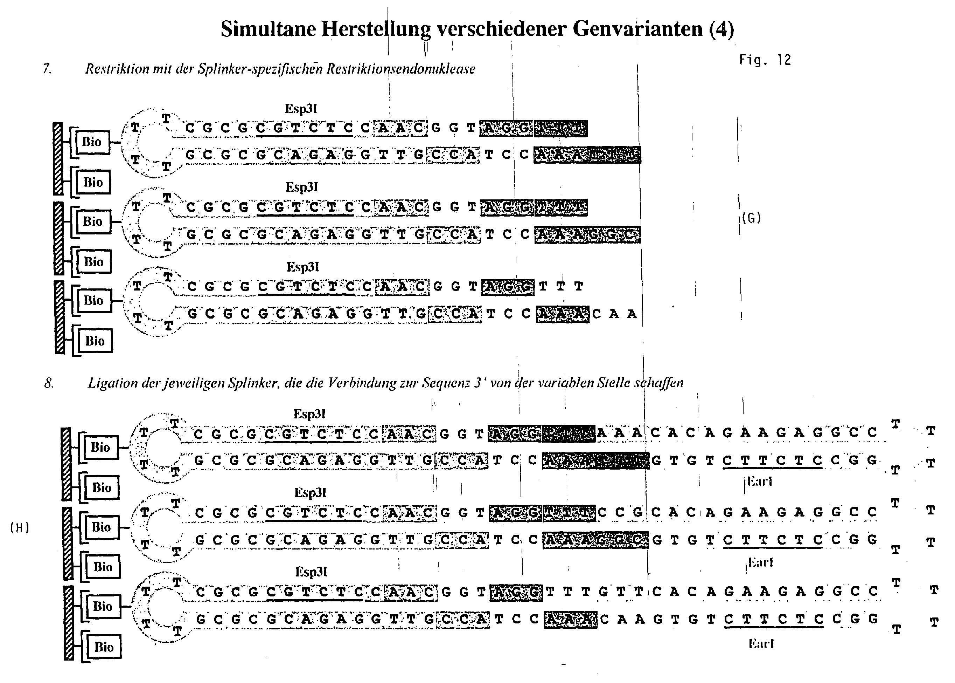

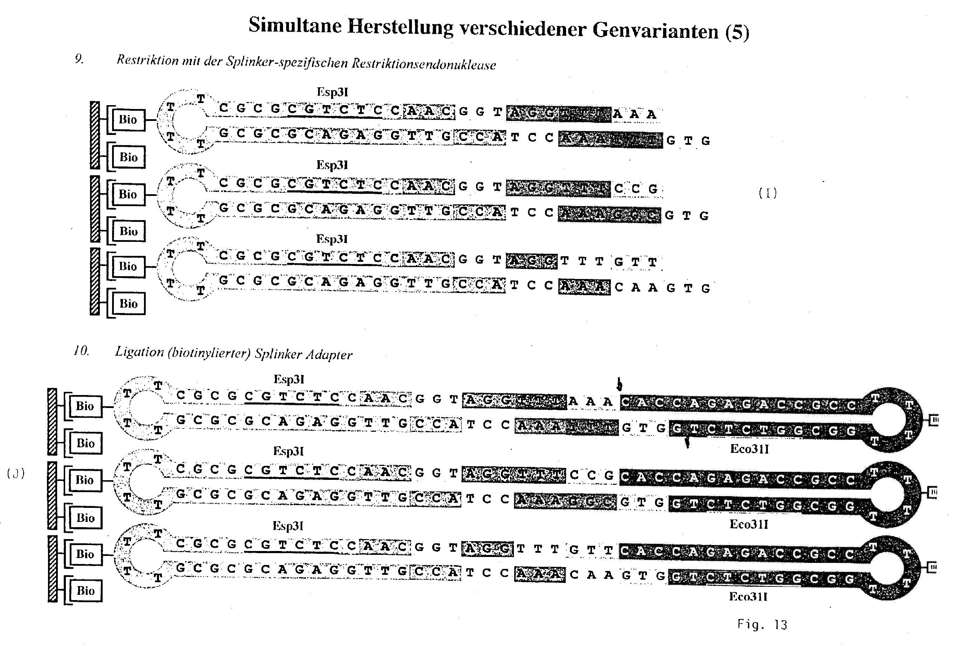

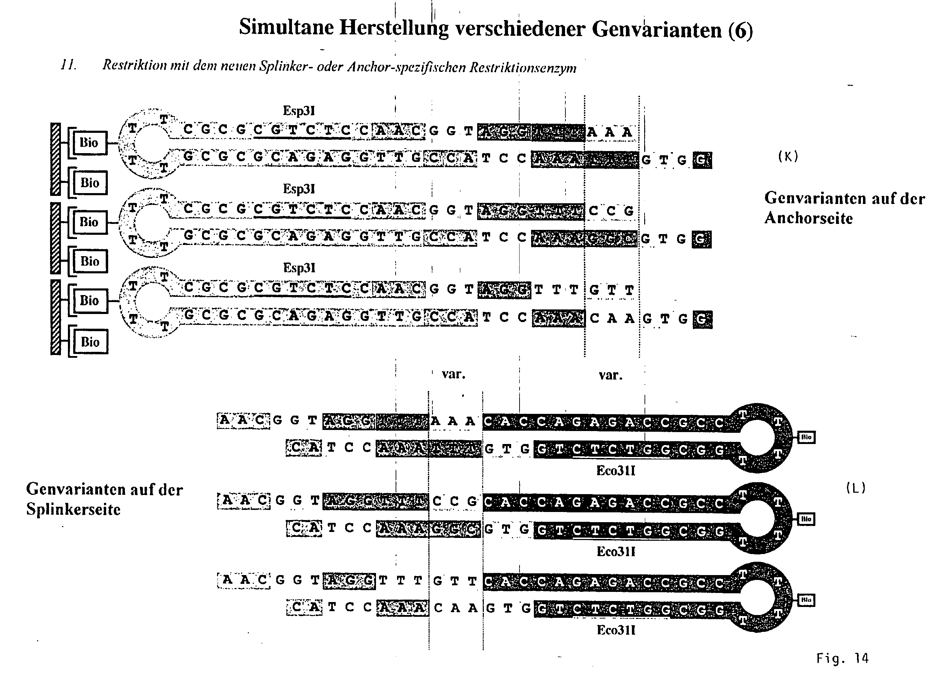

In einer Weiterentwicklung des Verfahrens nach WO 00/75368 können aber auch mehrere Genvarianten gleichzeitig hergestellt werden. Um Teilfragmente mit terminal verschiedenen Sequenzen herstellen zu können, ist dazu folgende Modifikation des Protokolls notwendig: Nach der Ligation eines modifizierten Splinkers an einen verlängerten Anchor (nach vorheriger Blockierung der vorhandenen Bindungsstellen an der Festphase) wird das entstandene Anchor-Splinker Ligationsprodukt nicht mit der Splinker-spezifischen Restriktionsendonuklease geschnitten, sondern mit dem Anchor-spezifischen Restriktionsenzym. Hierdurch entstehen doppelsträngige DNA Moleküle, welche an einem Ende einen einzelsträngigen Überhang aufweisen, jedoch trotz ihrer Modifikation nicht mehr an die feste Matrix gebunden sind. Diese DNA Moleküle können daher jetzt auf verschiedene Reaktionsgefäße aufgeteilt und an eine Festphase gebunden werden. Nach erneuter Blockierung freier Bindungsstellen, Ligation neuer Anchormoleküle sowie Spaltung mit der Splinker-spezifischen Restriktionsendonuklease können nun verschiedene Splinker ligiert anligiert werden, die sich in der Überhangsequenz unterscheiden, nicht aber in den an den Überhang unmittelbar angrenzenden Nukleotiden, welche nach der folgenden Restriktion den nächsten Überhang bilden. Auf diese Weise können mehrere verschiedene Teilfragmente aufgebaut werden. Diese Fragmente werden im nächsten Schritt mit Splinkern ligiert, welche im Anschluss an die Überhangsequenzen wieder die gleiche Sequenz aufweisen, so dass alle Varianten Fragmente nach der folgenden Spaltung mit dem in der aufzubauenden Gensequenz folgenden Fragment verknüpft werden können. Um eine annähernd äquimolare Aufteilung dieser verschiedenen Fragmente zu erreichen, müssen auch alle anderen, parallel hergestellten Ligationsprodukte entsprechend behandelt und portioniert werden. Hierzu ist es notwendig, dass diejenigen Fragmente, die nach dem ursprünglichen Verfahren am Anchor verbleiben nach dem Schneiden mit der Anchor-spezifischen Restriktionsendonuklease erst wieder mit einem Anchormolekül ligiert werden müssen, um die Reaktion fortführen zu können. Dieser Aspekt der Erfindung wird in den Figs. 9 bis 14 illustriert.

Nach Bedarf kann sich nach der letzten Transposition eine Polymerase Kettenreaktion (PCR) anschließen, in welcher als Primer Oligonukleotide verwendet werden, die zu den konstanten Teilen der Anchor bzw. Splinker komplementär sind. Auf diese Weise können Verluste durch die Aufteilung der Reaktionen wieder kompensiert werden. Vorzugsweise werden hierbei thermostabile Polymerasen mit Proofreading Funktion eingesetzt, um möglichst wenige zusätzliche Fehler einzuführen.

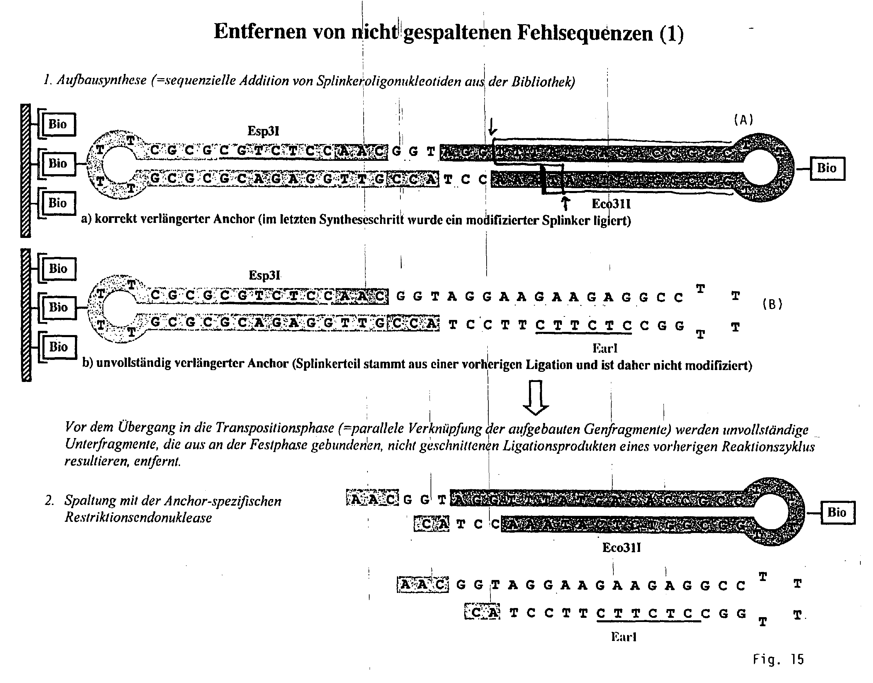

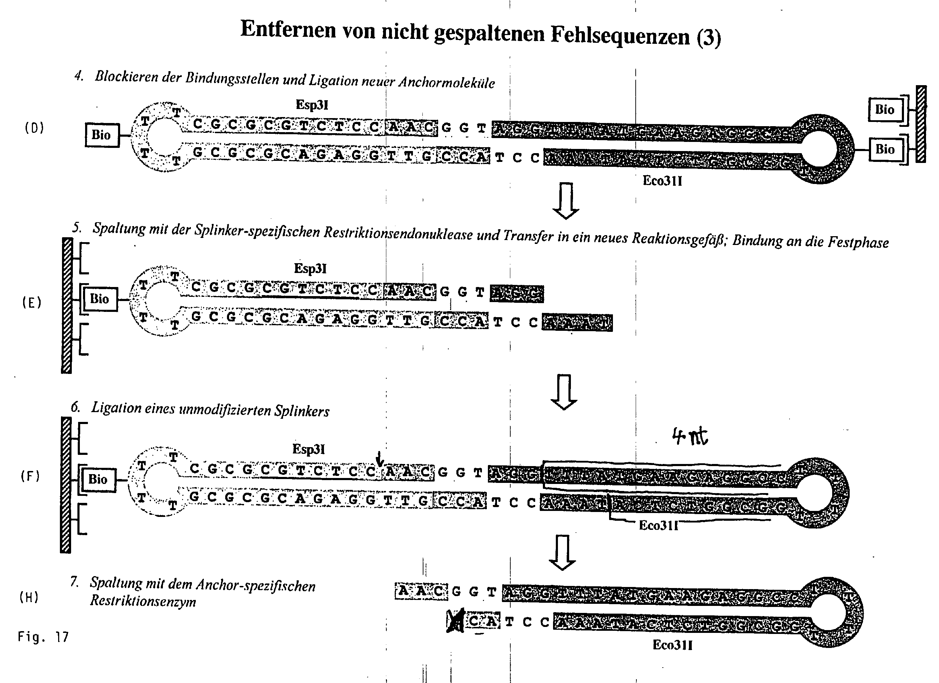

Ein ähnliches Verfahren kann angewendet werden, um zu verhindern, dass verkürzte Ligationsprodukte, welche durch inkomplette Spaltungen mit der Splinker-spezifischen Restriktionsendonuklease entstehen können, nicht in die folgenden Transpositionsreaktionen (= Übertragung von verlängerten Splinkermolekülen nach dem Schneiden mit der Anchor-spezifischen Restriktionsendonuklease) verschleppt werden und so zu Deletionsmutationen führen können. Hierbei werden an Stelle der jeweils letzten Splinkermoleküle am Ende des Ligation/Restriktion-Zyklus Anchormoleküle ligiert, welche jedoch eine Erkennungssequenz für das Splinker-spezifische Restriktionsenzym enthalten (sogenannte Splinker-Anchor). Durch eine Blockierung noch vorhandener freier Bindungsstellen der Festphase wird gewährleistet, dass die Splinker-Anchor nur mittels Ligation an die verlängerten Anchor an die Festphase gekoppelt werden. Nach der Spaltung der so entstandenen Ligationsprodukte mit dem Anchor spezifischen Restrüktionsenzym weisen nur diejenigen Moleküle, welche im letzten Schritt einen modifizierten Splinker-Anchor erhalten haben, die entsprechende Modifikation auf und können daher in einem nächsten Schritt nach Überführung in ein neues Reaktionsgefäß durch Bindung an eine Festphase von nicht modifizierten Teilfragmenten abgetrennt werden. Dieser Aspekt der Erfindung wird in den Figs. 15 bis 17 hierin weiter erläutert.

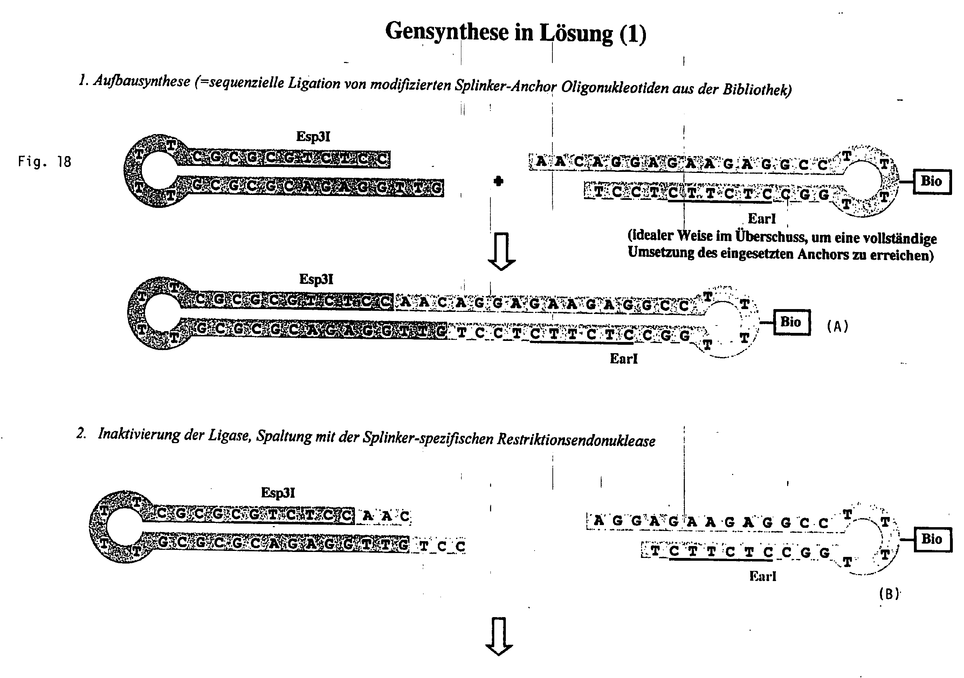

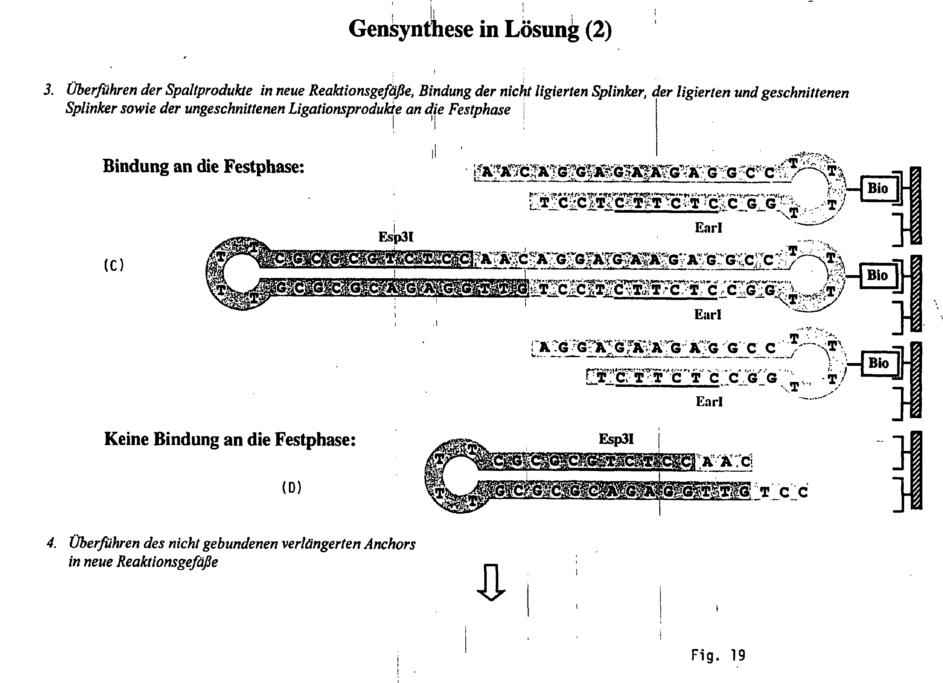

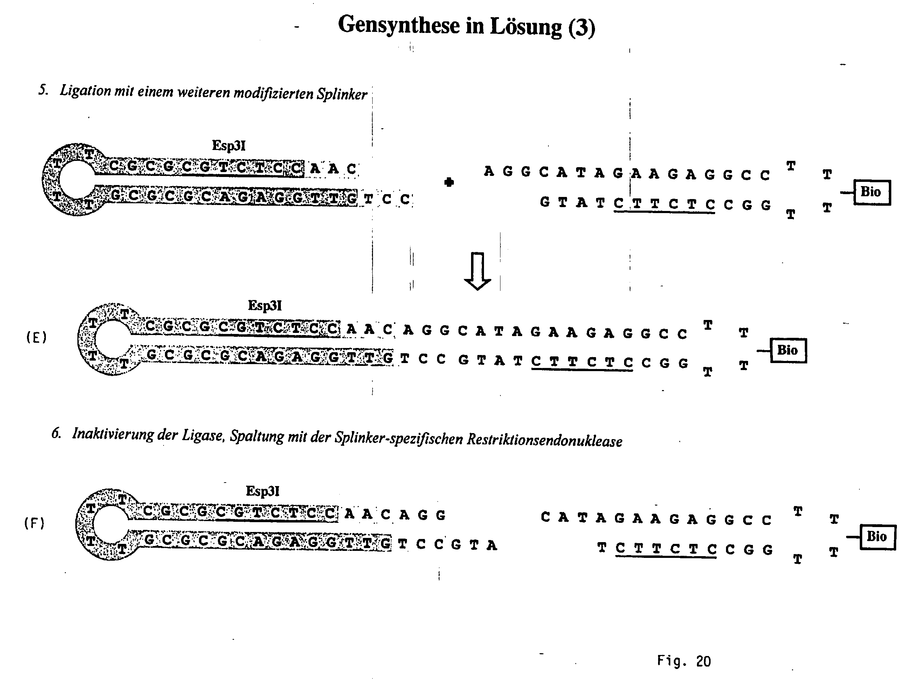

Des Weiteren ist auch die Umkehrung des in der Anmeldung WO 00/75368 beschriebenen Vorgehens möglich: anstatt die Genfragmente an den an eine Festphase gekoppelten Anchor Oligonukleotide aufzubauen, können die Ligationen prinzipiell auch in Lösung erfolgen, wobei jeweils modifizierte Splinker-Anchor statt Splinker Oligonukleotide ligiert werden. Nach der Restriktion mit der Splinker-spezifischen Restriktionsendonuklease verbleiben nunmehr die nicht geschnittenen Ligationsprodukte an der Festphase während die durch die Restriktion frei gesetzten Fragmente in die weiteren Reaktionsansätze überführt werden können. Bevorzugter Weise können bei dieser Vorgehensweise Enzyme eingesetzt werden, die selbst eine Modifikation tragen und somit ebenfalls in den Reaktionsansätzen zurückgehalten werden. Eine Hitzedenaturierung der Enzyme ist damit nicht mehr notwendig. Dieser Aspekt der Erfindung wird in den Figs. 18 bis 20 im Detail beschrieben.

Prinzipiell können aufsynthetisierte Teilfragmente durch Kombination der in den letzten beiden Absätzen beschriebenen Verfahren nach Belieben zwischen Anchor und Splinker-Anchor hin und her transferiert werden (Transfer-Reaktion). Eine identische Überhangsequenz vorausgesetzt, können so beliebige Genfragmente aus verschiedenen Synthesen miteinander kombiniert werden. Dieses "Mini-Exon-Shuffling" eignet sich beispielsweise hervorragend für die Herstellung von Designerproteinen oder zur Optimierung enzymatischer Eigenschaften durch die Kombination von Mutanten mit erhöhter Aktivität bzw. Stabilität.

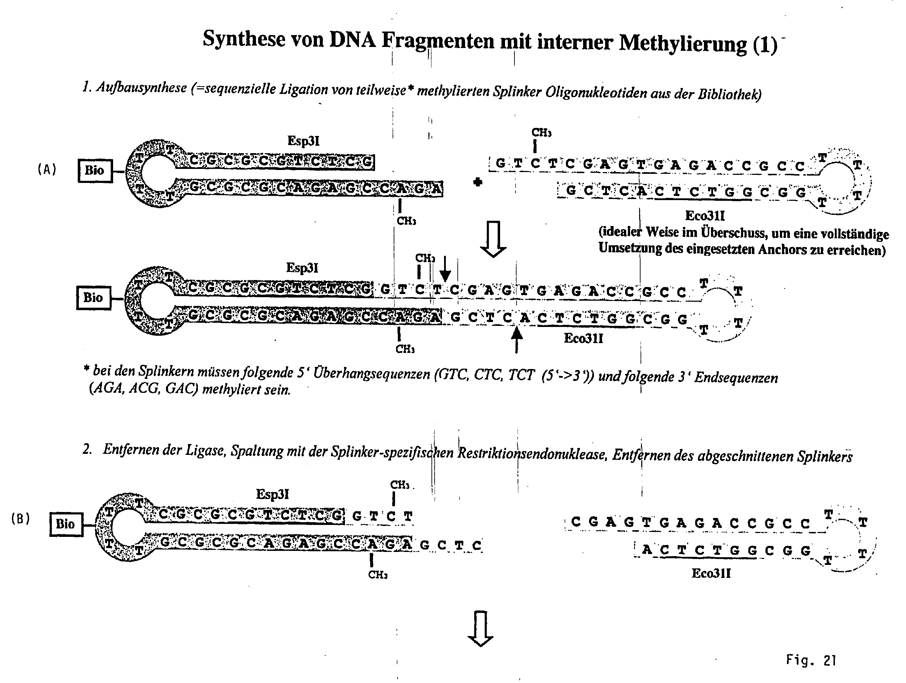

Das in der Anmeldung WO 00/75368 beschriebene Gensyntheseverfahren war in der Auswahl der zu synthetisierenden Nukleinsäuren insoweit eingeschränkt als dass die Erkennungssequenzen der verwendeten Anchor und Splinker spezifischen Restriktionsendonukleasen in der zu synthetisierenden Sequenz nicht auftreten durften, da diese zu internen Spaltungen der Teilfragmente führen würde. Diese Beschränkung könnte dadurch umgangen werden, dass beim Zusammensetzen einer solchen Sequenz ein Splinker mit einer Erkennungssequenz für eine alternative Restriktionsendonuklease verwendet wird und das Ligationsprodukt dann anschließend mit der entsprechenden Methylase behandelt wird. Die methylierten internen Sequenzen sind dann vor der Spaltung mit der entsprechenden Restriktionsendonuklease geschützt, während die Anchor und Splinker nach wie vor abgetrennt werden können. Dieser Aspekt der Erfindung wird in den Figs. 21 bis 22 weiter ausgeführt.

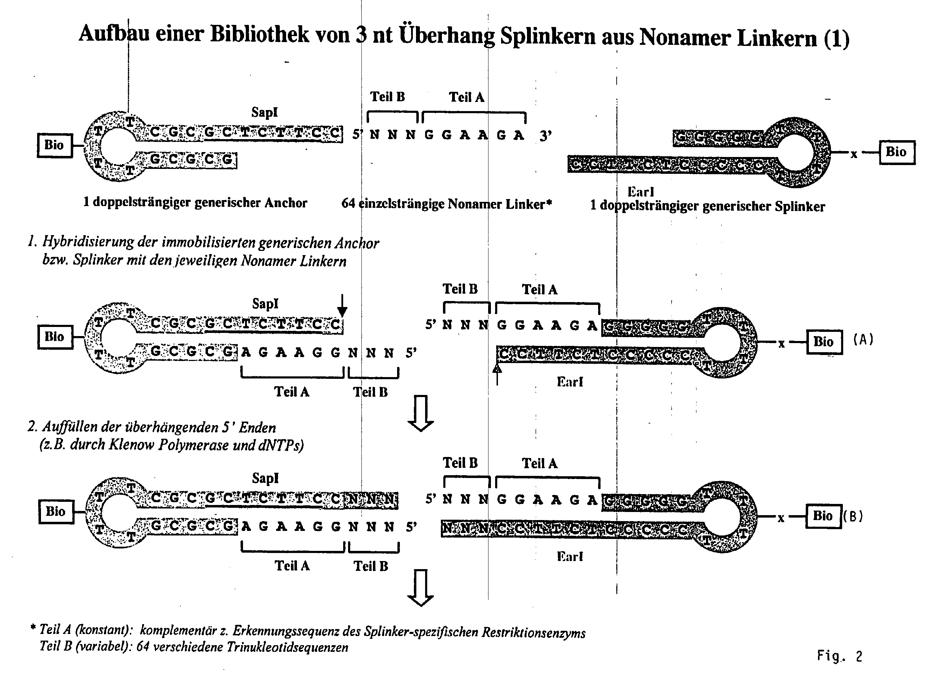

Schließlich kann eine Verbesserung des Verfahrens der ursprünglichen Anmeldung dadurch erreicht werden, dass der Einsatz von Splinker Oligonukleotiden mit einem nur drei Nuleotide großem Überhang möglich ist, obwohl bislang kein Paar von Restriktionsendonukleasen bekannt ist, welche einen drei Nukleotide langen Überhang produzieren und darüber hinaus voneinander unterscheidbare Erkennungssequenzen aufweisen. Das Problem, dass die Genfragmente dann nicht durch Spaltungen an entweder dem einen oder dem anderen Ende in den Transpositionen zusammen gesetzt werden können, kann nämlich dadurch umgangen werden, dass der letzte hinzuzufügende Splinker ein Adapter ist, d.h. zwar ein drei Nukleotide langes überstehendes Ende aufweist, aber eine Erkennungssequenz für ein Restriktionsenzym beinhaltet, welches einen vier Nukleotide langen Überhang erzeugt. Der Einsatz solcher Splinker-Adapter ist insoweit von Vorteil, als dass nur eine Splinkerbibliothek kleinerer Komplexität notwendig ist (4096 statt 65536) und drei Nukleotide lange Überhänge niemals selbstkomplementär sein können. Da zudem die Gene dann im Triplettraster aufgebaut werden können, ist für codierende Regionen eine weitere Einschränkung der Komplexität möglich, da nicht für alle Codons Splinker bereit gestellt werden müssen. Insgesamt werden 256 verschiedene Splinker-Adapter-benötigt, um alle Sequenzvarianten abdecken zu können; beschränkt man sich auf die 30 häufigsten Codons, würden 120 ausreichen. Dieser Aspekt der Erfindung wird in den Figs. 2 bis 8 näher erläutert.

Der Hauptaspekt der vorliegenden Erfindung beruht darauf, dass es mit Hilfe der hier beschriebenen Verfahren möglich ist, die Größe der für die Synthesen von beliebigen Genen notwendigen Oligonukleotid-Bibliothek durch eine kombinatorische Herstellung aus zwei kleineren Bibliotheken entscheidend zu verringern. Aus dieser Vorgehensweise resultieren gegenüber dem bereits sehr vorteilhaften Sloning-Verfahren weitere deutliche Vorteile, werden doch nicht mehr standardisierte Elemente mit Längen von 30 bis 40 Nukleotiden verwendet, sondern vielmehr Linkermoleküle, welche Längen von typischer Weise 6 bis 11 Nukleotiden aufweisen. Hierdurch wird der erreichbare Aufreinigungsgrad der standardisierten Oligonukleotidbausteine extrem verbessert und somit die Grundlage für eine sehr zuverlässige und automatisierbare Nukleinsäuresynthese geschaffen.

Ein weiterer Vorteil des erfindungsgemäßen Verfahrens, wie er sich aus der vorstehenden Darstellung ergibt, besteht darin, dass die Größe der Bibliothek der Linker im Falle eines Überhanges von vier Nukleotiden lediglich 256 beträgt, wobei diese für jedes Restriktionsenzym vom Typ IIs, welches auf Seiten der Oligonukleotide verwendet wird, entsprechend hergestellt werden muss. Somit ergibt sich, dass insgesamt im vorstehend genannten Beispiel lediglich 512 verschiedene Linker sowie zwei Oligonukleotide (jeweils eines für eine jede Klasse) hergestellt werden müssen, gegenüber insgesamt 65.536 Oligonukleotiden (bei der Verwendung von Restriktionsenzymen, die einen Überhang gegenüber ihrer Erkennungssequenz von vier Nukleotiden erzeugen).

Eine ganz besonders bevorzugte weitere Ausführungsform sowohl hinsichtlich der Linker, wie auch der sich daraus ergebenden Bibliotheken kann dann realisiert werden, wenn sich wie im Falle des Restriktionsenzym-Paares Eco31I/Esp3I, die Erkennungssequenz lediglich in einem Nukleotid unterscheidet. Unter diesen Umständen ist es möglich, dass statt der erforderlichen 512 (256 Linker für Klasse 1 und 256 Linker für Klasse 2) verschiedenen Elementen lediglich einmal 256 verschiedene Linker hergestellt werden müssen, wenn das eine Nukleotid, in dem sich die beiden Restriktionsenzyme des Restriktionsenzympaares unterscheiden nicht in Teil A des Linkers, sondern im terminalen Teil des doppelsträngigen Bereiches des Oligonukleotides angeordnet ist.

In der folgenden Tabelle 2 werden Sequenzen für Vertreter der beiden Klassen von Oligonukleotiden angegeben sowie das zu ihnen korrespondierende Restriktionsenzym vom Typ IIS, deren Erkennungssequenz entweder vollständig oder teilweise in dem 3'-OH-Überhang vorhanden ist.

**Tabelle 2: Sequenzbeispiele für selbstkomplementäre und bipartite Oligonukleotide**

| Restriktionsenzympaar | Oligonukleotid 1 (5' -3') | Oligonukleotid 2 (5'-3') | Linker (5'-3') |
|---|---|---|---|
| Eco31I/Esp3I | CGCCCCTTTTGGGGCGTCTCG (SEQ. ID.No. 9) | CCCGGGTTTTCCCGGGTCTCG (SEQ. ID.No. 11) | NNNNCGAGA (SEQ. ID.No. 13) |
| BbsI/Acc36I | TTCGGGTTTTCCCGAAGACGC (SEQ. ID.No. 10) | CAGGTGGGTTTTCCCACTGGGACGC (SEQ. ID.No. 12) | NNNNGCGTC (SEQ. ID.No. 14) |
| Eco31I/Esp3I (bipartite) | GGGGCGTCTCG CGCCCC (SEQ. ID.No.17) | CCCGGG CCCGGGTCTCG (SEQ. ID.No. 19) | NNNNCGAGA (SEQ. ID.No. 15) |
| BbsI/Acc36I (bipartite) | CCCGAAGACGC TTCGGG (SEQ. ID.No. 18) | CAGGTG CCCACTGGGACGC (SEQ. ID.No. 20) | NNNNGCGTC (SEQ. ID.No. 16) |

Die hierin offenbarten Nukleinsäuremolekülbibliotheken bestehen aus einer Mehrzahl der erfindungsgemäßen einzelsträngigen Nukleinsäuremoleküle, wie sie hierin offenbart werden. Der Begriff einzelsträngiges Nukleinsäuremolekül und Linker werden hierin, sofern nicht anders angegeben, synonym verwendet. Bevorzugter Weise umfassen diese Nukleinsäuremolekülbibliotheken den gesamten Sequenzraum, wie er durch die Länge des Überhanges (Teil B des Linkers) definiert ist. Es ist jedoch auch im Umfang der vorliegenden Erfindung, dass lediglich ein Teil der entsprechenden Linker und somit ein Teil des Sequenzraumes in der Nukleinsäuremolekülbibliothek enthalten ist. Darüber hinaus kann das relative Verhältnis der einzelnen Moleküle in einer derartigen Bibliothek zueinander sowohl gleich als auch unterschiedlich ausgebildet sein. Beispielsweise ist es im Rahmen der vorliegenden Erfindung, dass solche Sequenzen, die vergleichsweise selten in den zu synthetisierenden Sequenzen oder natürlichen Sequenzen vorkommen, verglichen mit anderen, häufiger vorkommenderen Sequenzen entsprechend unterrepräsentiert sind

Wie bereits anhand der vorstehenden Beschreibung offenbart wurde, können sowohl die einzelsträngigen Nukleinsäuremoleküle, d. h. Linker, als auch die NukleinsäuremolekülBibliotheken im Rahmen eines Verfahrens zur Herstellung eines Nukleinsäuremoleküls verwendet werden. Bevorzugter Weise handelt es sich bei diesem Verfahren um ein Verfahren mit sequenzieller Ligation von Oligonukleotiden auf sequenzunabhängige Weise, wie es in der internationalen Patentanmeldung WO 00/75368 beispielhaft beschrieben ist. Die hierin als Oligonukleotide der ersten Klasse definierten Oligonukleotide, wobei die Zuordnung dadurch erfolgt, dass ein Oligonukleotid der ersten Klasse eine Erkennungssequenz eines ersten Restriktionsenzym vom Typ IIS oder einen Teil davon oder eine dazu komplementäre Sequenz umfasst und ein Oligonukleotid der zweiten Klasse eine Erkennungssequenz eines zweiten, vom ersten Restriktionsenzym verschiedenen Restriktionsenzym vom Typ IIS oder einen Teil davon oder eine dazu komplementäre Sequenz umfasst, können dabei ein sogenanntes "Anchor"-Oligonukleotid sein, d.h. eine Modifikation tragen, die eine Immobilisierung des Oligonukleotids an einer Festphase erlaubt, und die andere Klasse ein sogenanntes "Splinker"-Oligonukleotid, das eine gleich- oder andersartige spaltbare Modifikation aufweist. Ansonsten entsprechen "Anchor"- und "Splinker"-Oligonukleotide dem hierin für die Oligonukleotide beschriebenen Aufbau aus einem einzelsträngigen und einem doppelsträngigen Bereich sowie optional einer Schleife.

Das erfindungsgemäße Verfahren sieht dabei vor, dass ein Oligonukleotid einer durch das Vorhandensein der Erkennungssequenz, deren komplementären Sequenz oder jeweils eines Teils davon, eines ersten Restriktionsenzyms vom Typ IIS definierten Klasse 1 bereitgestellt wird, wobei dieses Oligonukleotid hinsichtlich der Erkennungssequenz für das erste Restriktionsenzym wie oben offenbart ausgebildet sein kann. Dieses im Folgenden als erstes Oligonukleotid bezeichnete Oligonukleotid kann eine Modifikation aufweisen, die eine Befestigung oder Immobilisierung des ersten Oligonukleotids an einer Oberfläche, bevorzugter Weise an einer festen Matrix, erlaubt. Bevorzugter Weise ist diese Modifikation so ausgebildet, dass eine Abspaltung des an die Oberfläche gebundenen Oligonukleotids erfolgen kann. Zu diesem ersten Oligonukleotid wird ein erfindungsgemäßer Linker unter geeigneten Bedingungen hinzugegeben, so dass eine Hybridisierung zwischen dem ersten Oligonukleotid und dem Linker erfolgt. Die Hybridisierung beruht auf der Komplementarität von Teil A des Linkers mit dem einzelsträngigen Teil des Oligonukleotids. Durch die Hybridisierung wird ein Doppelstrang gebildet, der die vollständige Erkennungssequenz des besagten Restriktionsenzyms vom Typ IIS enthält. Die Mengenverhältnisse zwischen dem ersten Oligonukleotid und dem Linker werden- entsprechend den Erfordernissen einer effizienten Ligation ausgestaltet, wobei typischer Weise vorgesehen ist, dass der vergleichsweise kleinere Linker im Überschuss zum ersten Oligonukleotid hinzugegeben wird.

Als nächster Schritt kann nach Behandlung mit einer Kinase der 5'-überhängende Teil des ersten Oligonukleotides aufgefüllt und somit glattendig gemacht werden. Bevorzugter Weise wird vor dem Auffüllen, typischer Weise unter Verwendung des Klenow-Fragmentes der T4 DNA-Polymerase, der Überschuss des Linkers entfernt. Diese Entfernung kann in dem Fall, dass das erste Oligonukleotid an einer festen Matrix immobilisiert ist, durch entsprechende Waschschritte erfolgen. Alternativ kann vorgesehen sein, dass eine Auftrennung der verschiedenen Moleküle, insbesondere eine Abtrennung des Linkers für den Fall, dass das erste Oligonukleotid nicht immobilisiert ist, mittels geeigneter Trenntechniken, wie beispielsweise Gelelektrophorese oder Gelfiltration, erfolgen. Neben der bevorzugter Weise erfolgenden Entfernung des nicht ligierten Überschusses an Linkern werden entweder gleichzeitig oder in verschiedenen Schritten auch die weiteren Komponenten des Reaktionsansatzes, d. h. Kinase, Klenow-Fragment und die bei der Auffüllung nicht umgesetzten Nukleosid-triphosphate entfernt.

Parallel dazu oder im Nachgang dazu wird anschließend ein Oligonukleotid der zweiten Klasse, hierin auch als zweites Oligonukleotid bezeichnet, bereitgestellt, wobei sich dieses Oligonukleotid bzw. die Klasse dadurch auszeichnet, dass sie vollständig oder teilweise die Erkennungssequenz eines von der Klasse 1 unterschiedlichen Restriktions-enzyms vom Typ IIS bzw. die komplementäre Sequenz hierzu umfassen und diese nun mit einem entsprechenden Linker umgesetzt wird, dessen Teil A zu dem einzelsträngigen Bereich des Oligonukleotids komplementär ist, und nach Ligation und Auffüllen am Ende auch hier ein glattendiges Oligonukleotid vorhanden ist. Auch das zweite Oligonukleotid kann, nun versehen mit dem Linker und entsprechend aufgefüllt, an einer Oberfläche immobilisiert vorliegen.

In einem nächsten Schritt werden die glattendigen Oligonukleotide miteinander in Kontakt gebracht, wobei bevorzugter Weise entweder das erste Oligonukleotid oder das zweite Oligonukleotid an einer Oberfläche immobilisiert vorliegen. Es ist jedoch auch im Rahmen des erfindungsgemäßen Verfahrens, dass beide glattendigen Oligonukleotide in Lösung vorliegen. Die beiden glattendigen Oligonukleotide, welche gegenüber den jeweiligen Ausgangsoligonukleotiden jeweils um die Länge des Linkerüberhangs an ihrem 5'-Ende verlängert sind, werden unter Verwendung einer Ligaseaktivität ligiert. Die nicht umgesetzten Moleküle sowie die verwendeten Enzyme können nach den den Fachleuten auf dem Gebiet bekannten Verfahren leicht entfernt werden, so beispielsweise für den Fall, dass die gesamte Reaktion in Lösung erfolgt ist, durch Gelelektrophorese, für den Fall, dass eines der Oligonukleotide an einer Oberfläche immobilisiert vorliegt und somit auch das Ligationsprodukt, durch Waschen unter Verwendung geeigneter Waschlösungen.

In einem weiteren Schritt wird unter Verwendung eines der beiden Restriktionsenzyme vom Typ IIS ein Oligonukleotid vom Ligationsprodukt zwischen den beiden glattendigen, aufgefüllten Ausgangsoligonukleotiden abgetrennt. Dieses unterscheidet sich gegenüber dem ursprünglich eingesetzten Oligonukleotid dadurch, dass dieses die variablen Nukleotide (Teil B) des zuvor ligierten Linkers enthält, ebenso wie die variablen Nukleotide des zweiten Linkers welcher mit dem zweiten Oligonukleotid verbunden wurde.

Bei der Ausführungsform des erfindungsgemäßen Verfahrens, bei dem wenigstens eines der Oligonukleotide an eine feste Phase immobilisiert ist, schließt sich vor dem Schneideschritt durch das Restriktionsenzym vom Typ IIS noch der Schritt an, das aus dem glattendigen und aufgefülltem ersten Oligonukleotid und dem glattendigen und aufgefüllten zweiten Oligonukleotid entstandene Ligationsprodukt von der Oberfläche durch Spaltung der durch die in dem Oligonukleotid vorhandenen Modifikation ausgebildeten Verbindung zwischen dem Ligationsprodukt und der festen Oberfläche abgetrennt wird.

Der erfindungsgemäße Kit umfasst wenigstens eines der erfindungsgemäßen einzelsträngigen Nukleinsäuremoleküle, d. h. Linker. Bevorzugter Weise umfasst ein derartiger Kit eine der erfindungsgemäßen Nukleinsäurebibliotheken, oder einen Teil davon. In einer Ausführungsform umfasst der Kit darüber hinaus noch geeignete Puffer, Enzymaktivitäten wie Ligasen, Topoisomerasen, 3'-5'-Exonukleasen, Phosphatasen, Typ IIS-Restriktionsendonukleasen, oder geeignete Oberflächen. Bevorzugter Weise umfasst der Kit zwei verschiedene Restriktionsenzyme vom Typ IIS, die bevorzugter Weise Überhänge von der gleichen Länge erzeugen. Dabei kann vorgesehen sein, dass die Oberflächen bereits eines oder mehrere der standardisierten Oligonukleotide aufweisen.

Ein derartiger Kit dient typischer Weise zur Herstellung einer Nukleinsäure

Der Begriff Nukleinsäure umfasst hierin bevorzugter Weise-Desoxyribonukleinsäure

Die vorliegende Erfindung wird nun anhand der folgenden Figuren und Beispiele weiter erläutert, aus denen sich weitere Merkmale, Ausführungsformen und Vorteile der Erfindung ergeben. Dabei zeigt bzw. zeigen
- Fig. 1: den Ablauf des erfindungsgemäßen Verfahrens;
- Figs. 2 und 3: die Erzeugung einer Bibliothek von Splinker-Molekülen mit einem Überhang von drei Nukleotiden;
- Figs. 4 und 5: ein Verfahren zum Aufbau einer Bibliothek, welche den Übergang von Splinker bzw. Anchor-Molekülen mit einem drei Nukleotide langen Überhang zu solchen mit einem vier Nukleotide langen Überhang erlauben;
- Figs. 6 bis 8: eine Ausführungsform des Sloning-Verfahrens unter Verwendung von Anchor- und Splinker-Molekülen mit einem drei Nukleotide langen Überhang;
- Figs. 9 bis 14: die wesentlichen Schritte des erfindungsgemäßen Verfahrens zur simultanen Herstellung verschiedener Genvarianten unter Anwendung des Sloning-Verfahrens;
- Figs. 15 bis 17: die verschiedenen Schritte bei der Entfernung von nicht gespaltenen Fehlsequenzen;
- Figs. 18 bis 20: die verschiedenen Schritte bei der Gensynthese in Lösung, bei der es sich um eine weitere Ausführungsform des Sloning-Verfahrens handelt;
- Figs. 21 und 22: die wesentlichen Schritte bei der Synthese von DNA-Fragmenten mit interner Methylierung gemäß der vorliegenden Erfindung; und
- Fig. 23: das Verfahren der (Zwischen)- Produktamplifikation, wie es im Rahmen von verschiedenen Schritten des Sloning-Verfahrens durchgeführt werden kann.

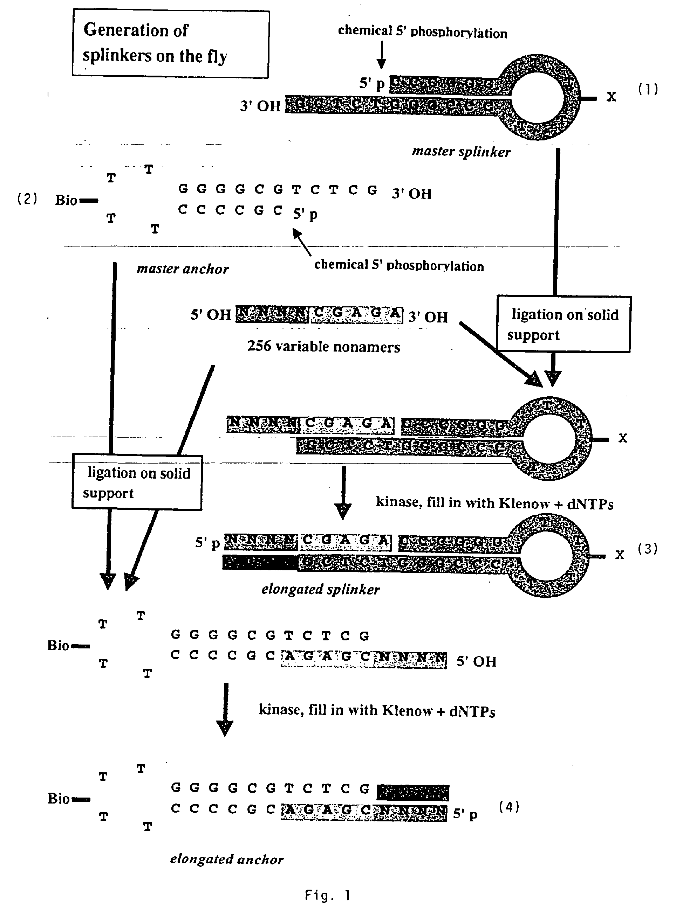

Fig. 1 zeigt den Ablauf des erfindungsgemäßen Verfahrens, bei dem zur Verringerung der Größe der Bibliothek aus Anchor und Splinker Molekülen ein einzelsträngiges Linker-Molekül verwendet wird. Dabei wird in einem ersten Schritt ein Oligonukleotid 1 bereitgestellt, das auch als generischer Splinker oder Master Splinker bezeichnet wird und einen einzelsträngigen Bereich umfassend fünf Nukleotide sowie einen doppelsträngigen Bereich umfassend sieben Nukleotide sowie eine Schleife bestehend aus vier Nukleotiden umfasst. Die Schleife trägt eine Modifikation X, welche geeignet ist, Oligonukleotid 1 an eine feste Oberfläche zu binden. Bevorzugter Weise handelt es sich dabei um eine reversible Bindung. Das Oligonukleotid 1 weist ein überstehendes 3'-OH-Ende auf. Das 5'-Ende wird chemisch oder enzymatisch phosphoryliert

In einem zweiten Schritt wird Oligonukleotid 2 bereigestellt, welches hierin auch als generischer Anchor oder Master anchor bezeichnet wird. Auch Oligonukleotid 2 besteht aus einem einzelsträngigen Bereich umfassend fünf Nukleotide, einem doppelsträngigen Bereich umfassend sechs Nukleotide sowie einer Schleife umfassend vier Nukleotide. Die Schleife trägt eine Biotinylierung, die eine Bindung des Oligonukleotides 2 an eine Oberfläche erlaubt. Ähnlich wie Oligonukleotid 1 steht das 3'-OH-Ende um fünf Nukleotide über und das 5'-Ende ist chemisch oder enzymatisch phosphoryliert. Das überstehende 3'-OH-Ende von Oligonukleotid 1 stellt dabei die Erkennungssequenz von Restriktionsenzym X dar.

Sowohl Oligonukleotid 1 als auch Oligonukleotid 2 werden unabhängig voneinander an einen festen Träger, im Fall von biotinylierten Oligonukleotiden an Streptavidin-beschichtete Beads oder Mikrotiterplatten nach den Herstellerangaben gebunden. Zu den immobilisierten Oligonukleotiden 1 und 2 wird sodann jeweils ein Linker hinzugegeben. Im vorliegenden Falle umfasst Teil A des Linkers die Sequenz CGAGA und entspricht damit dem Komplementärstrang der letzten 4 Nukleotide der Typ IIs Restriktionsenzyme Eco31I und Esp3I und hybridisiert mit dem entsprechenden Einzelstrang des Oligonukleotids 1. Anschließen erfolgt eine Ligation unter Verwendung einer Ligase-Aktivität und es kommt zur Ausbildung der vollständigen Erkennungssequenz von Restriktionsenzym Eco31I. Gleiches erfolgt an dem ebenfalls an einer Oberfläche immobilisierten Oligonukleotid 2. Nach Ligation des Linkers an Oligonukleotid 1 bzw. Oligonukleotid 2 steht Teil B des Linkers in beiden Fällen über und definiert den Bereich bzw. die Nukleinsäuresequenz, der bzw. die im Rahmen der Synthese aufgebaut werden soll. Dieses überstehende Ende wird nach Kinasebehandlung und Klenow-Polymerisebehandlung mittels entsprechender Nukleosidtriphosphate spezifisch aufgefüllt, so dass am Ende ein aufgefülltes Oligonukleotid (1) bzw. Oligonukleotid (2) an der Oberfläche immobilisiert ist. In Abhängigkeit von der zu synthetisierenden Nukleinsäure werden dabei jene Linker gewählt, deren Teil B die gewünschte Sequenz aufweist. In dem in Fig. 1 dargestellten Verfahren wurde durch geeignete Wahl des Restriktionsenzympaares gewährleistet, dass sowohl für das Verlängern bzw. Auffüllen von Oligonukleotid 1 als auch von Oligonukleotid 2 die gleiche Linker-Bibliothek verwendet werden kann. Dies wurde dadurch möglich, dass die Sequenz des Oligonukleotides 1 bzw. Oligonukleotides 2 am Übergang zwischen dem Oligonukleotid und dem Linker so ausgestaltet wurde, dass nach Ligation die für die beiden Restriktionsenzyme unterschiedlichen Erkennungssequenzen ausgebildet wurden.

Als nächster Schritt kann die Entfernung von Oligonukleotid 1 und/oder Oligonukleotid 2 von der Oberfläche erfolgen. Anschließend kommt es zur Ligation der beiden aufgefüllten Oligonukleotide 1 und 2. In einem weiteren Reaktionsschritt wird sodann das Ligationsprodukt mit einem der beiden Restriktionsenzyme vom Typ IIS, im vorliegenden Fall mit Esp3I, gespalten. Auf diese Weise können komplette Splinkeroligonukleotide mit sämtlichen 65536 möglichen Oktamerendsequenzen erzeugt werden.

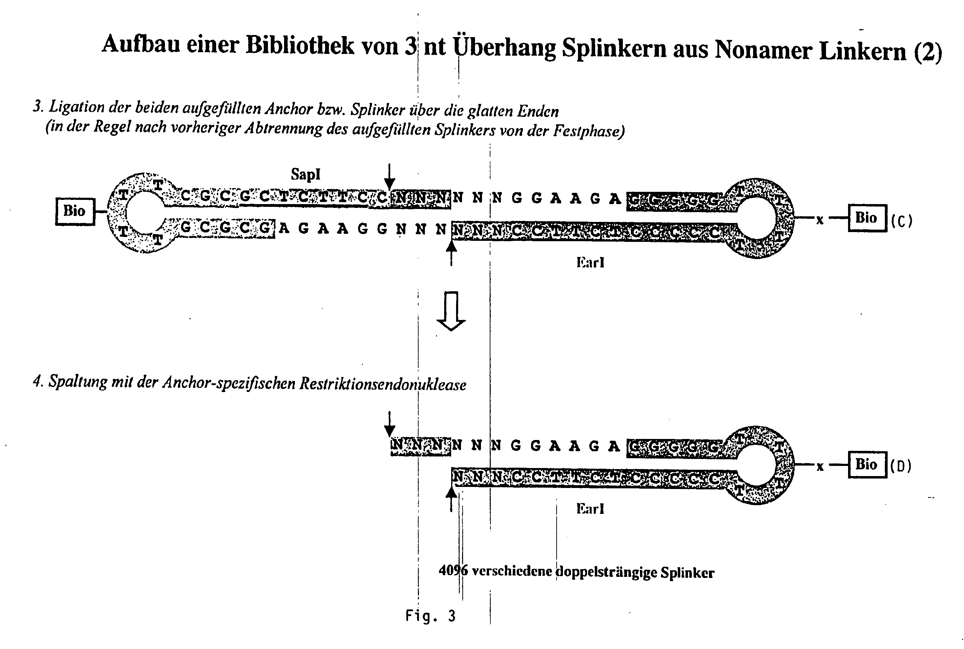

Die Figuren 2 und 3 zeigen die Erzeugung einer Bibliothek von Splinker-Molekülen mit einem Überhang von drei Nukleotiden. Wie hierin offenbart und aus den vorstehend genannten Aspekten offensichtlich, werden beispielsweise bei der Erzeugung von Genvarianten und insbesondere von Genvarianten, die codierende Nukleinsäuren betreffen, bevorzugter Weise Anchor- und Splinker-Moleküle verwendet mit einem Überhang von drei Nukleotiden. Insoweit ist es ein Aspekt der Erfindung, ein Verfahren bereitzustellen zur Erzeugung einer Bibliothek von Splinker-Molekülen bzw. Anchor-Molekülen mit einem Überhang von drei Nukleotiden. Beispielhaft für die Erzeugung von derartigen Molekülen ist in den Figuren 2 und 3 die Erzeugung einer Bibliothek mit Splinker-Molekülen mit einem Überhang von drei Nukleotiden dargestellt. Im Zusammenhang mit der Beschreibung der Figuren 2 und 3 bezeichnet dabei der Begriff des Anchors oder Anchor-Moleküls ein Oligonukleotid gemäß aa) bzw. ba) des Sloning-Verfahres bzw. der Begriff des Splinkers oder Splinker-Moleküls ein Oligonukleotid gemäß ab) bzw. bb) des Sloning-Verfahrens.

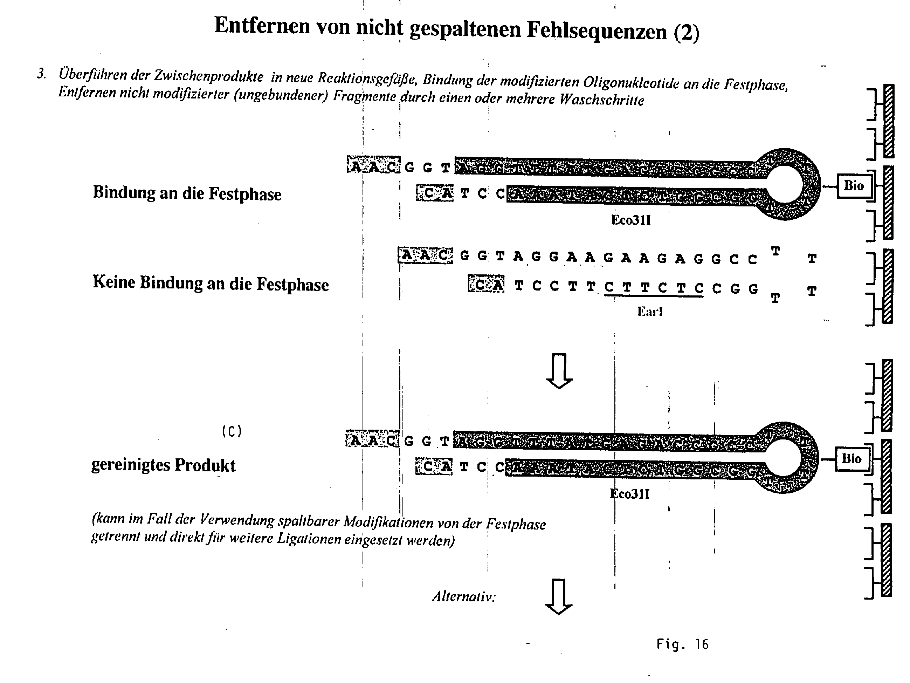

Der Aufbau der Bibliothek beginnt dabei ausgehend von einem Anchor-Molekül, welches eine Modifizierung trägt, welche eine Kopplung an eine feste Matrix erlaubt, sowie eines Splinker-Moleküls, welches ebenfalls eine Modifizierung trägt, die eine Kopplung an eine feste Matrix erlaubt, wobei diese Splinker-Modifizierung bevorzugter Weise spaltbar ist. Zu einem Anchor-Molekül, genauer dem generischen Anchormolekül (ist für alle zu synthetisierenden Splinker bzw. Anchor Oligonukleotide gleich) wird ein einzelsträngiges Nukleinsäuremolekül, hierin auch als Linker bezeichnet, - hinzugegeben, welches im vorliegenden Falle aus einem Nonamer besteht, umfassend einen Teil B und einen Teil A. Der Teil A ist dabei komplementär zu dem 5'-Überhang des Anchor-Moleküls. Der Teil B umfasst drei Nukleotide, die eine beliebige Sequenz umfassen. In gleicher Weise wird einem bevorzugter Weisegenerischen Splinker-Molekül ein einzelsträngiges Nukleinsäuremolekül vorgegangen, d.h. es wird ein Linker, zugegeben, der ebenfalls aus einem Teil A und Teil B besteht, wobei die Teile A und B prinzipiell genauso ausgebildet sind wie im Falle des zu dem Anchor-Mölekül hinzugegebenen Linkers. Mit Blick darauf, dass an jeder der drei, in Fig. 16 (A) mit N bezeichneten Positionen eines der vier Nukleotide stehen kann, kann mittels 64 verschiedener einzelsträngiger Linker der gesamte Sequenzraum, d.h. alle möglichen Moleküle, die sich in diesen drei Nukleotidpositionen unterscheiden können, abgebildet werden. Bevorzugter Weise ist die Länge des Teils A des Linkers sechs Nukleotide, wobei jedoch auch Linker mit größeren und kleineren Längen von Teil A im Schutzumfang enthalten sind.

Durch Hybridisieren des Anchor-Moleküls bzw. des Splinker-Moleküls mit jeweils einem der insgesamt 64 Linkern, können sodann insgesamt 64 verschiedene Anchor-Moleküle bzw. Splinker-Moleküle erzeugt werden, die sich jeweils im Teil B unterscheiden. Teil A des Linkers ist dabei typischer Weise komplementär zur Erkennungssequenz, oder eines Teils davon, Anchor- bzw. des Splinker-spezifischen Restriktionsenzyms oder der dazu komplementären Sequenz. Die überhängenden Enden der mit dem Linker aufgefüllten Anchor bzw. Splinker-Molekülen wird sodann durch eine Polymerase, beispielsweise durch Klenow Polymerase aufgefüllt und somit Anchor-Moleküle und Splinker-Moleküle mit glatten Enden erzeugt.

Bevorzugter Weise ist das Anchor-Molekül ebenso wie das Splinker-Molekül an eine Oberfläche oder eine feste Matrix gekoppelt. Die Modifikation des Splinker-Moleküls kann bevorzugter Weise unter milden Bedingungen gespalten werden, so dass der aufgefüllte Splinker von der Oberfläche gelöst und einem Ligationsansatz mit einem geeigneten glattendigen Anchor-Molekül hinzugefügt werden kann.

In einem nächsten Schritt wird sodann das Ligationsprodukt aus Schritt (C) mit einer Anchor-spezifischen Restriktionsendonuklease vom Typ IIS gespalten, wodurch Splinker-Moleküle mit einem drei Nukleotid langen Überhang entstehen. In gleicher Weise kann prinzipiell die Spaltung mittels Splinker-spezifischer Restriktionsnukleasen erfolgen. Durch diesen Prozess kann ausgehend von insgesamt 64 verschiedenen einzelsträngigen Linkern, die sich in drei aufeinanderfolgenden Nukleotiden unterscheiden, insgesamt 4.096 verschiedene doppelsträngige Splinker-Moleküle erzeugt werden, die sodann als Ausgangsbibliothek für ein Sloning-Verfahren in der beschriebenen Verfahrensweise verwendet werden können.

Beim erfindungsgemäßen Aufbau von Genfragmenten aus Anchor- und Splinkermolekülen mit einem Überhang von drei Nukleotiden ist es erforderlich, Anchor-Moleküle bzw. Splinker-Moleküle bereitzustellen, die zwar einen drei Nukleotid lagen Überhang aufweisen, jedoch die Erkennungssequenz für ein Restriktionsenzym vom Typ IIS tragen, das Überhänge mit einer Länge von ein, zwei, vier, fünf oder mehr Nukleotiden erzeugt. Dies ist notwendig, weil für die Durchführung des Sloning-Verfahrens immer mindestens zwei Restriktionsenzyme vom Typ IIS mit unterscheidbaren Erkennungssequenzen verwendet werden müssen, die darüber hinaus Überhänge der gleichen Länge erzeugen. Zum gegenwärtigen Zeitpunkt sind jedoch an Restriktionsenzymen vom Typ IIS, welche einen drei Nukleotid langen Überhang erzeugen, nur Isoschizomere bekannt, die die gleichen Sequenzen erkennen.. Eines dieser Restriktionsenzyme ist SapI. Vor dem Übergang in die Transpositionsphase (d.h. der Verknüpfung der parallel synthetisierten Teilfragmente, die ja jeweils paarweise mit verschiedenen Restriktionsenzymen geschnitten werden müssen) ist es bei Verwendung einer Bibliothek von Splinkern mit einem 3 Nukleotide langen Überhang also zunächst notwendig, Fragmente mit einem gleich langen Überhang (z.B. bestehend aus 4 Nukleotiden) zu schaffen.

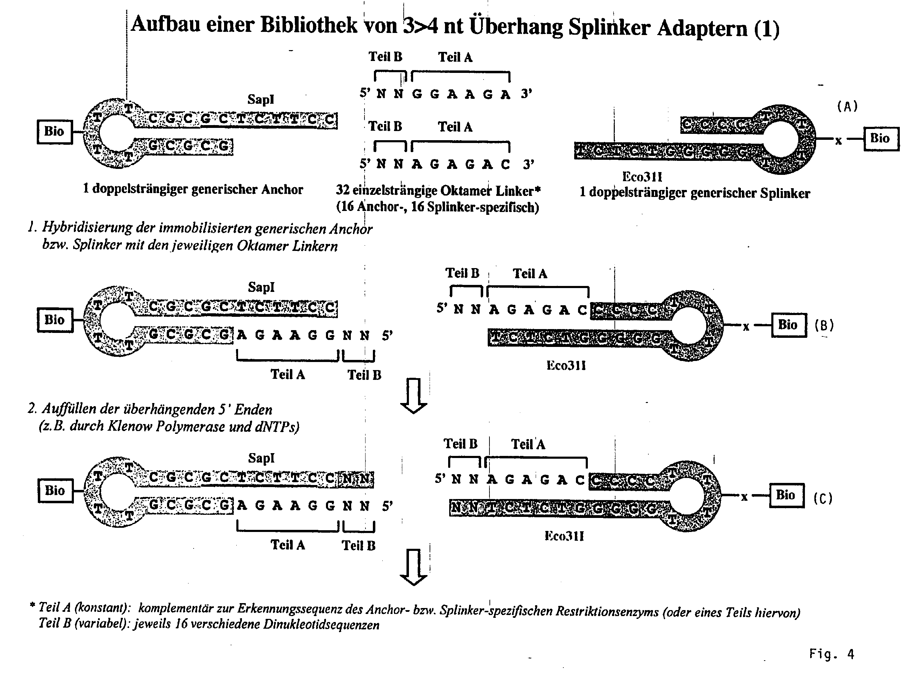

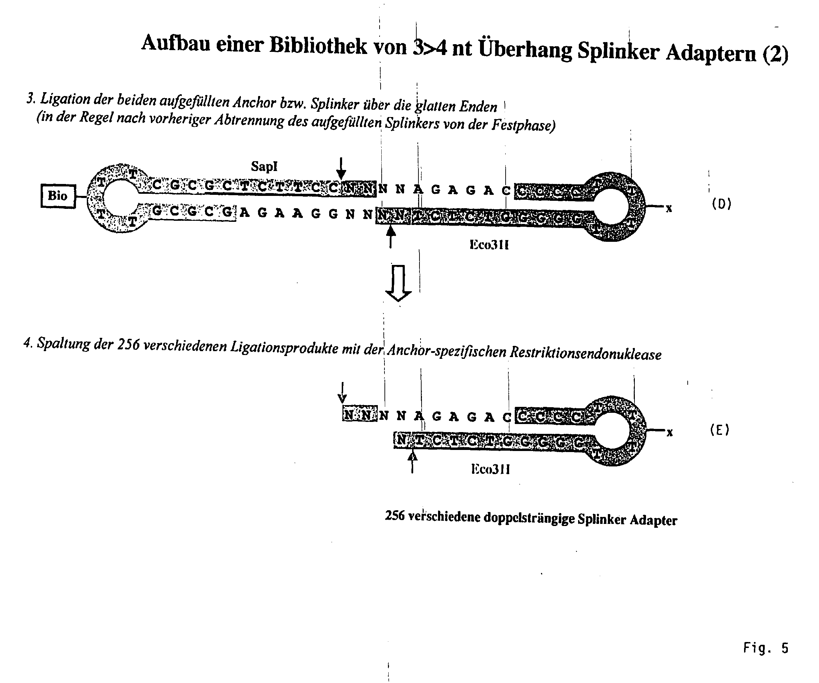

Die Figs. 4 und 5 zeigen ein Verfahren zum Aufbau einer Bibliothek, welche den Übergang von Splinker- bzw. Anchor-Molekülen mit einem drei Nukleotide langen Überhang zu solchen mit einem vier Nukleotide langen Überhang erlauben. Dabei wird grundsätzlich ähnlich vorgegangen, wie bei dem vorstehend beschriebenen Verfahren zum Aufbau einer Bibliothek aus Splinker- bzw. Anchor-Molekülen mit einem drei Nukleotid langen Überhang. Der wesentliche Unterschied besteht in der Ausgestaltung des Linker-Moleküls, welches, wie in (A) dargestellt, im vorliegenden Fall eine Länge von zwei Nukleotiden aufweist. Insoweit reichen 32 einzelsträngige Linker Moleküle aus, 16 Anchor-spezifische sowie 16 Splinker-spezifische, um eine vollständige Bibliothek, d.h. eine Bibliothek, die alle mögliche Sequenzen beinhaltet, bei denen sich die beiden letzten 5'-terminalen Positionen unterscheiden, aufzubauen. In Schritt (D) wird sodann mit der Anchor-spezifischen Restriktionsendonuklease geschnitten, im vorliegenden Falle SapI, was einen drei Nukleotid langen Überhang erzeugt. Mit diesem Verfahren können sodann die insgesamt 256 verschiedenen doppelsträngigen Moleküle erzeugt werden, die bei ihrer Verwendung den Übergang von Splinker- und Anchor-Molekülen mit einem drei Nukleotide langen Überhang zu solchen mit einem vier Nukleotide langen Überhang erlauben und daher auch als Splinker-Adaptoren bezeichnet werden

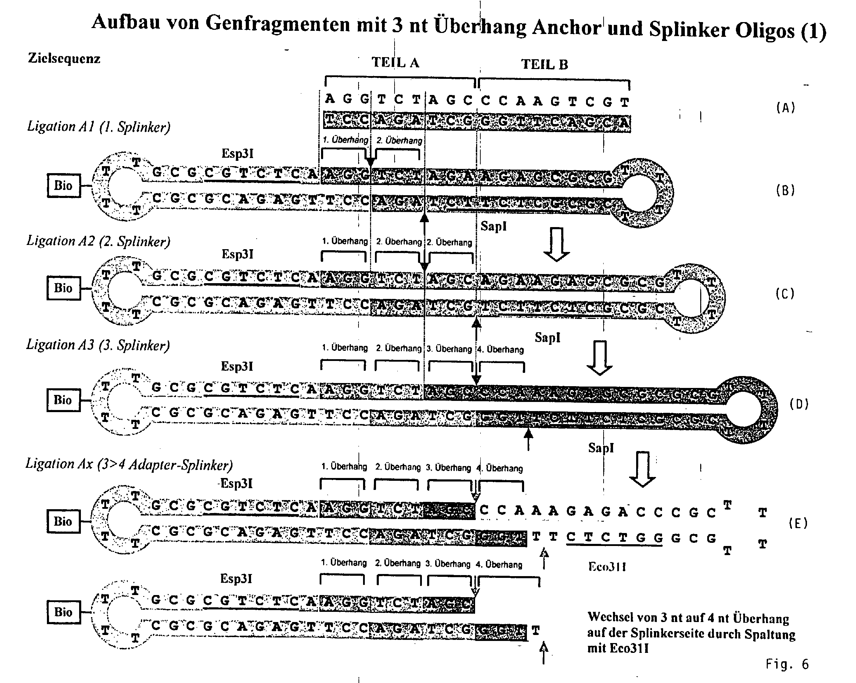

Auf der Grundlage der beiden vorstehend beschriebenen Bibliotheken und den Verfahren zu deren Herstellung kann sodann ein Aufbau von Genfragmenten aus Anchor- und Splinker-Molekülen mit einem drei Nukleotide langen Überhang erfolgen. Das entsprechende Verfahren ist in den Figuren 6 bis 8 dargestellt. Im Zusammenhang mit der Beschreibung der Figuren bezeichnet der Begriff des Anchors oder Anchor-Moleküls ein Oligonukleotid gemäß aa) bzw. ba) des Sloning-Verfahres bzw. der Begriff des Splinkers oder Splinker-Moleküls ein Oligonukleotid gemäß ab) bzw. bb) des Sloning-Verfahrens.

Geht man von der in (A) dargestellten zu synthetisierenden Sequenz aus (hierin auch als Zielsequenz bezeichnet), kann diese im vorliegenden Fall in die Teile A und B gegliedert werden. Der Teil A besteht ebenso wie der Teil B aus 9 Nukleotiden, die jeweils in drei Gruppen zu je drei Nukleotiden eingeteilt sind. Entsprechend kann ausgehend von einem an eine feste Matrix gekoppelten Anchor durch Ligation eines ersten Splinker-Moleküls die erste Dreiergruppe der Nukleotide auf das Anchor-Molekül übertragen werden. Durch Spaltung des Ligationsproduktes in Schritt (B) mittels des drei Nukleotide lange Überhänge erzeugenden Restriktionsenzyms vom Typ IIs (SapI) wird ein weiterer drei Nukleotide langer Überhang erzeugt, an den im Schritt (C) ein zweites Splinker-Molekül ligiert wird, welches anschließend durch Spaltung mit dem Splinker-spezifischen Restriktionsenzym abgetrennt wird. In gleicher Weise wird mit einem dritten Splinker-Molekül verfahren, wobei sodann das Ligationsprodukt gemäß (D) erhalten wird. In Schritt (E) wird nach Spaltung des Ligationsproduktes aus (D) nun ein Splinker-Adapter verwendet, der den Übergang von einem Überhang aus drei Nukleotiden zu einem Überhang aus vier Nukleotiden erlaubt. Wird nach Ligation dieses hierin auch als Adapter-Splinkers oder 3->4- Adapter-Linker bezeichneten Splinker-Moleküls mit dem Splinker-spezifischen Restriktionsenzyms gespalten, im vorliegenden Falle mit Eco31I, wird nun nicht mehr ein drei Nukleotide langer Überhang erzeugt, sondern ein vier Nukleotide langer Überhang und damit die Voraussetzungen für eine Transposition im Rahmen des Sloning-Verfahrens geschaffen, bei der zwei aufeinander abgestimmte Restriktionsenzyme vom Typ IIS verwendet werden.

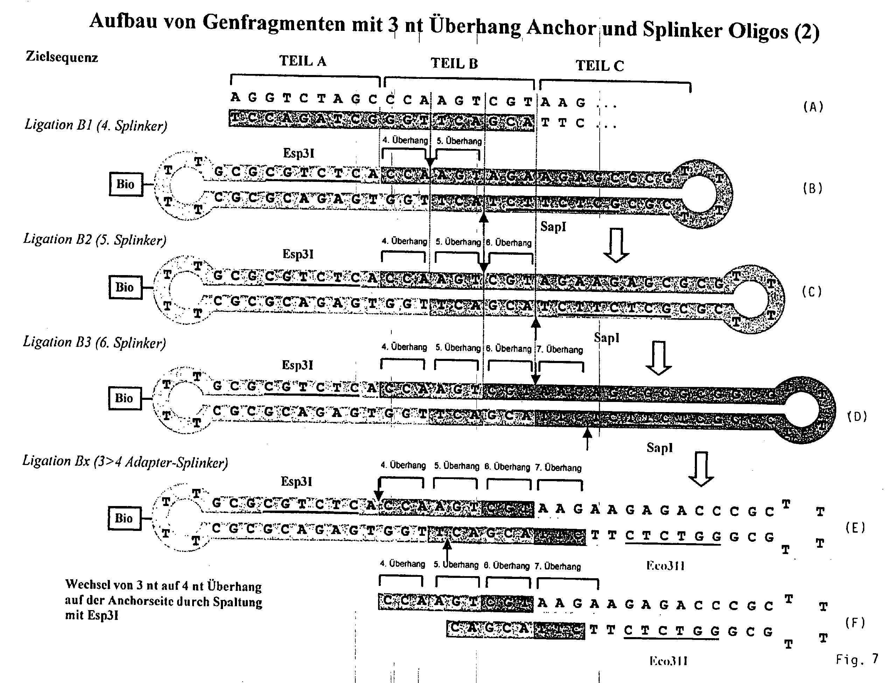

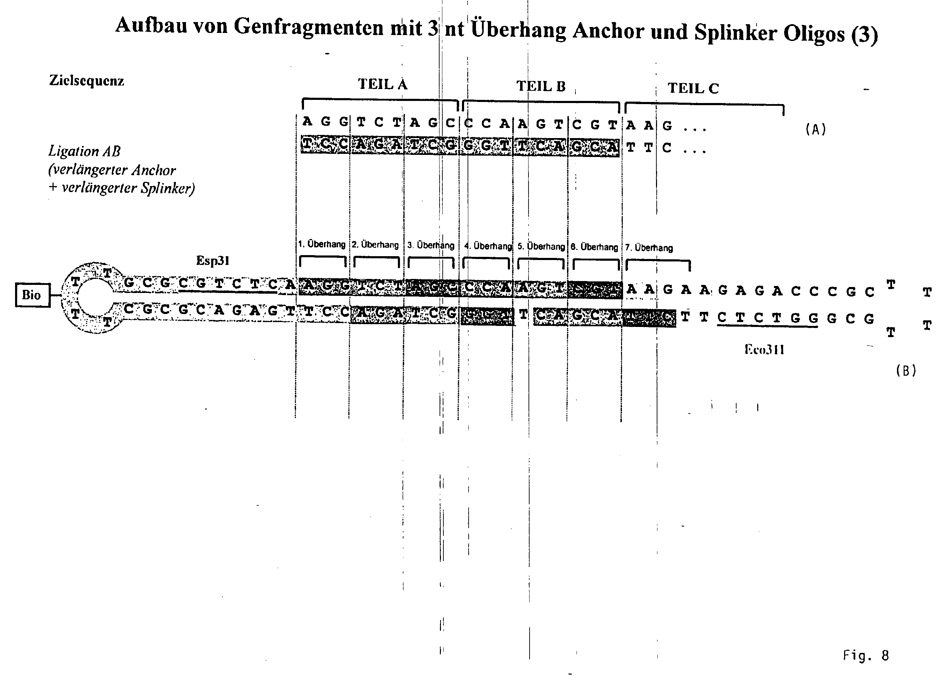

In den Schritten (B) bis (E) von Fig. 7 wird prinzipiell genauso vorgegangen, wie bei den Schritten (B) bis (E) von Fig. 6, wobei hier jedoch der in Schritt (B) verwendete Splinker einen 3 Nukleotid langen Überhang aufweist, der dem ersten Triplett von Teil (B) der Zielsequenz entspricht. Nachdem das dritte Triplett von Teil B durch einen entsprechendes Splinker-Molekül an das verlängerte Anchor-Molekül ligiert wurde, wird nochmals mit SapI geschnitten und sodann ein weiteres Splinker-Molekül verwendet, welches das erste Triplett von Teil C der Zielsequenz umfasst. Um auch hier die Voraussetzung für eine Transposition zu schaffen, wird sodann mit dem Anchor-spezifischen Restriktionsenzym vom Typ IIS, im vorliegenden Falle mit Esp3I, geschnitten und somit ein vier Nukleotide langer Überhang erzeugt. Die Zielsequenz aus Teil A und Teil B, wie in (A) von Fig. 8 dargestellt, wird nun dadurch erzeugt, dass das Spaltprodukt, d. h. das Anchor-Molekül mit einem vier Nukleotide langen Überhang aus Figur 6 (F) mit dem einen vier Nukleotide langen Überhang aufweisenden Splinker-Molekül aus Schritt (F) von Fig. 7 ligiert wird.

Die Figs. 9 bis Fig. 14 zeigen die wesentlichen Schritte des erfindungsgemäßen Verfahrens zur simultanen Herstellung verschiedener Genvarianten unter Anwendung des Sloning-Verfahrens. Im Zusammenhang mit dieser weiteren Ausführungsform des Sloning-Verfahrens ist anzumerken, dass hierbei der wesentliche Punkt darin zu sehen ist, dass ein korrekt verlängertes Anchor-Molekül oder ein korrekt verlängertes Splinker-Molekül auf mehrere Reaktionsansätze, bevorzugter Weise in verschiedenen Reaktionsgefäßen, aufgeteilt wird. Diese Aufteilung ist insbesondere dann nicht unproblematisch, wenn die Modifizierung der Anchor- bzw. Splinker-Moleküle eine Kopplung an eine feste Matrix erlauben, die eine Freisetzung der besagten Moleküle von derselben nicht erlauben, wie das beispielsweise bei der Verwendung nicht-spaltbarer Modifizierungen oder Modifizierungen, die zu einer sehr stabilen Wechselwirkung zwischen Nukleinsäure bzw. Modifikation und fester Matrix führt, der Fall ist.

Wie in den Fig. 9 bis Fig. 14 dargestellt, ist diese Ausführungsform des Sloning-Verfahrens im Wesentlichen durch die spezifische Ausgestaltung der Schritte aa) bis ag) bzw. ba) bis bg) gekennzeichnet. Im Zusammenhang mit der Beschreibung der Figuren 9 bis 14 bezeichnet der Begriff "Anchor" oder "Anchor-Molekül" ein Oligonukleotid gemäß aa) bzw. ba) des Sloning-Verfahres und der Begriff "Splinker" oder "Splinker-Molekül" ein Oligonukleotid gemäß ab) bzw. bb) des Sloning-Verfahrens.

Sofern freie Bindungsstellen der festen Matrix blockiert wurden, kann an Stelle eines normalen (nicht modifizierten) Splinker Oligonukleotids ein spezielles Splinker-Molekül an das verlängerte Anchor-Molekül ligiert werden. Dieses spezielle Splinker-Molekül enthält eine Modifikation, welche eine Kopplung an eine feste Matrix erlaubt. (A). In einem nächsten Schritt (B) erfolgt die Spaltung des so entstandenen Ligationsproduktes mit dem Anchor-spezifischen Restriktionsenzym vom Typ IIS. In Folge dieser Spaltung wird ein verlängertes Splinker-Molekül freigesetzt, welches eine Modifizierung trägt und in der Lösung des Reaktionsansatzes enthalten ist. Dieser Reaktionsansatz, genauer gesagt der flüssige Überstand davon, wird sodann in dem Umfang aliquotiert, wie dies erwünscht ist, insbesondere in dem Umfang, wie verschiedene Genvarianten erzeugt werden sollen. Sollen beispielsweise drei Genvarianten erzeugt werden, wie in den Figuren 9 bis 14 hierin beispielhaft veranschaulicht, erfolgt eine Aufteilung des flüssigen Überstandes in insgesamt drei Aliquots.

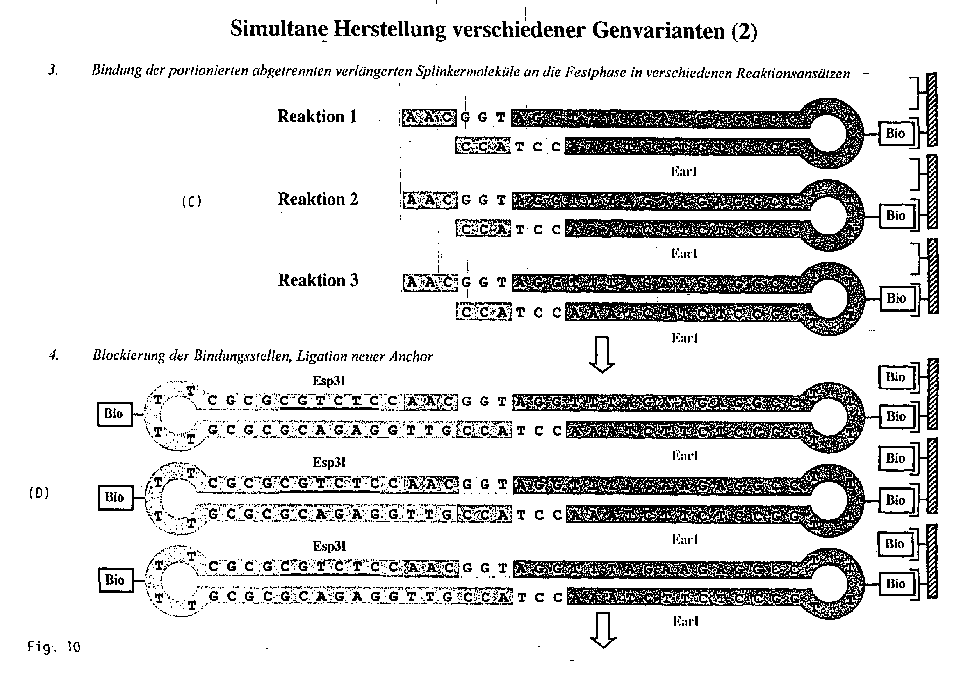

Ein jedes Aliquot, das ein abgetrenntes, verlängertes Splinker-Molekül mit einer Modifizierung enthält, wird in ein eigenes Reaktionsgefäß überführt, wobei in Folge der Modifizierung das verlängerte Splinker-Molekül an die feste Matrix gebunden wird (C). Die in Fig. 10 (C) mit Reaktion 1, Reaktion 2 und Reaktion 3 bezeichneten Abbildungen stellen die insgesamt drei hierin beispielhaft beschriebenen Reaktionsansätze dar.

Zu einem jeden Reaktionsansatz wird sodann ein Anchor-Molekül hinzugegeben, welches in Folge, der Komplementarität der überhängenden Enden mit dem an die feste Matrix gekoppelten Splinker-Molekül hybridisiert und anschließend unter Verwendung geeigneter Ligasen ligiert wird. Nachdem die Anchor-Moleküle selbst wiederum eine Modifizierung tragen, die eine Kopplung an eine feste Matrix erlauben, ist es erforderlich, dass vor Zugabe der Anchor-Moleküle die Bindungsstellen für diese Matrix blockiert werden, so dass eine Kopplung der hinzugegebenen Anchor-Moleküle an die feste Matrix unterbleibt. Dieser Blockierungsschritt ist dann nicht erforderlich, wenn die Modifizierung der Anchor-Moleküle eine Kopplung an die feste Matrix in dem Reaktionsansatz, in dem das an eine feste Oberfäche gekoppelte Splinker-Molekül enthalten ist, nicht erlaubt.

In einem nächsten Schritt wird sodann das Ligationsprodukt mit einer Splinker-spezifischen Restriktionsnuklease vom Typ IIS gespalten (E). Nachdem in Schritt (D) in Fig. 10 der Anchor zwar eine Modifizierung trägt, jedoch nicht an die feste Matrix koppeln kann, wird nach Spaltung des Ligationsproduktes der verlängerte Anchor im flüssigen Überstand des Reaktionsansatzes sein, wohingegen das Splinker-Molekül in Folge der Kopplung über die Modifizierung an die feste Matrix gebunden bleibt. Ein jeder der Überstände wird sodann in ein weiteres Reaktionsgefäß überführt, wobei der darin enthaltene verlängerte Anchor in Folge seiner Modifizierung an die Oberfläche gekoppelt wird (E).

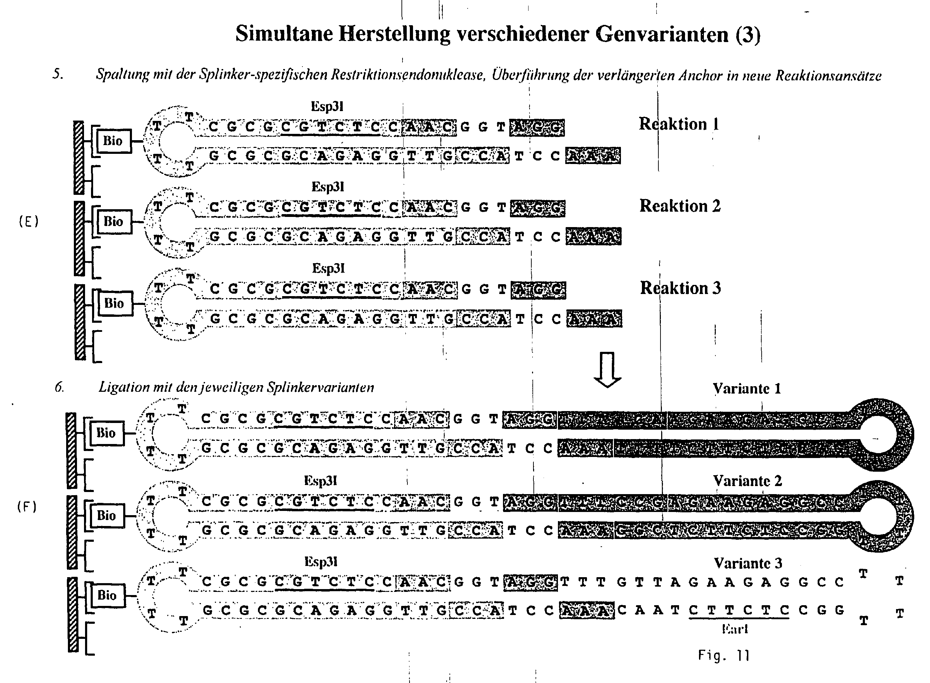

Zu dem solchermaßen immobilisierten Anchor-Molekülen werden zu einem jeden der entsprechenden Reaktionsansätze unterschiedliche Splinker-Moleküle hinzugegeben, wobei die Splinker-Moleküle sich in einem variablen Bereich unterscheiden, der sich dem 5'-überhängenden Ende anschließt. Bevorzugter Weise weist der sich anschließende variable Bereich eine Länge von 1 bis 9 Nukleotide auf, wobei 3 Nukleotide bevorzugt sind, da damit ein Codon für eine codierende Sequenz spezifisch bereitgestellt werden kann. In dem in Fig. 11 (F) dargestellten drei Reaktionsansätzen unterscheiden sich die Splinker an den Positionen 4 bis 6 (AGA, CCG bzw. GTT). Wird nun in einem nächsten Reaktionsansatz das solchermaßen erhaltene Ligationsprodukt aus Schritt (F) mit einer Splinker-spezifischen Restriktionsendonuklease gespalten, entstehen in den Reaktionsansätzen unterschiedlich verlängerte Anchor-Moleküle. Im Reaktionsansatz 1 umfasst die Variante sodann TTT, im Reaktionsansatz 2 GGC und im Reaktionsansatz 3 CAA (G). In einem nächsten Schritt werden sodann unterschiedliche Linker-Moleküle, deren Überhang komplementär zum Überhang der immobilisierten Anchor-Moleküle ist, hinzugesetzt. In Folge der Variabilität des Überhangs des einzelnen Anchor-Moleküls im Reaktionsansatz sind somit einem jeden Reaktionsansatz solche Splinker-Moleküle hinzuzugeben, die eine entsprechend komplementäre Sequenz aufweisen, so dass ein jeder Reaktionsansatz einen anderen Linker erfordert (Fig. 7 (H)). Bevorzugter Weise unterscheiden sich die verschiedenen Splinker-Moleküle nur in diesem Bereich. Anschließend erfolgt eine weitere Spaltung des in Schritt (H) erhaltenen Ligationsproduktes mittels einer Splinker-spezifischen Restriktionsnukleoase (J). In einem nächsten Schritt erfolgt sodann eine Ligation eines weiteren Splinker-Moleküls, das komplementär ist zu dem jeweiligen Überhang des verlängerten Anchor-Moleküls in einem jeden der drei Reaktionsansätze. Hier kann jeweils der gleiche Linker verwendet werden, da die in Schritt (J) erzeugten Überhänge des verlängerten Anchor-Moleküls eine identische Sequenz aufweisen. Bei dem in Schritt (J) hinzugefügten Splinker-Molekül kann es sich dabei um ein solches handeln, welches den Übergang von einem, wie in den Figuren 4 und 5 dargestellten, drei Nukleotide langen Überhang zu einem vier Nukleotide langen Überhang ermöglicht. Dieser Übergang unter Verwendung des Oligonukleotids, welches einen drei Nukleotide langen Überhang aufweist, dessen Erkennungsstellen für ein Restriktionsenzym vom Typ IIS bei Schneiden des Oligonukleotides mit demselben zu einem vier Nukleotide langen Überhang führt, wird hierin auch als Splinker-Adapter bezeichnet. In der hierin speziell beschriebenen Ausführungsform ist vorgesehen, dass dieser Splinker-Adapter eine Modifikation aufweist, die eine Kopplung an die Oberfläche einer festen Matrix erlaubt. In Abhängigkeit davon, ob das in Schritt (J) erhaltene Ligationsprodukt mit einem Splinker-spezifischen Restriktionsenzym vom Typ IIS gespalten wird, werden die in Schritt (K) dargestellten verlängerten, an eine feste Matrix gekoppelten Anchor-Moleküle in den verschiedenen Ansätzen erhalten werden, oder, bei Verwendung eines Anchor-spezifischen Restriktionsenzyms vom IIS die in (L) dargestellten verlängerten Splinker-Moleküle. Im ersteren Falle sind die Genvarianten auf der Anchor-Seite angeordnet, im zweiten Falle auf der Splinker-Seite. Die Ausgestaltung des Splinker-Moleküls mit einer Modifizierung liefert die Voraussetzung, dass das solchermaßen verlängerte Splinker-Molekül als Anchor-Molekül in einem Transpositionsschritt des Sloning-Verfahrens verwendet werden kann.

Obgleich anhand der Figuren 9 bis 14 lediglich die einmalige Einführung einer Genvariante beschrieben worden ist, ist es im Rahmen der vorliegenden Offenbarung, dass dieses mehrfach vorgenommen werden kann. Um nicht unnötig lange Splinker- bzw. Anchor-Moleküle zu erhalten und somit den Vorteil der Parallelsynthese, d.h. Durchführung von Transpositionen, aufzugeben, wird typischer Weise weniger als zehnmal, bevorzugter Weise weniger als fünfmal der Einbau einer Genvariante, wie vorstehend beschrieben, erfolgen.

Die Figs. 15 bis 17 zeigen die verschiedenen Schritte bei der Entfernung von nicht gespaltenen Fehlsequenzen gemäß der vorliegenden Erfindung. Im Zusammenhang mit der Beschreibung der Figuren 1 bis 3 bezeichnet der Begriff des Anchors oder Anchor-Moleküls ein-Oligonukleotid gemäß aa) bzw. ba) des Sloning-Verfahres und der Begriff des Splinkers oder Splinker-Moleküls ein Oligonukleotid gemäß ab) bzw. bb) des Sloning-Verfahrens.

Wie bei den hierin insgesamt offenbarten weiteren Ausführungsformen des als Sloning-Verfahren bezeichneten Verfahrens zur Herstellung von Nukleinsäuremolekülen durch Parallelsynthese, kann das Nukleinsäuremolekül, welches durch die Schritte aa) bis ag) hergestellt wird, mit dem Oligonukleotid, welches gemäß der Schritte ba) bis bg) hergestellt wird, miteinander verknüpft werden. Diese Verknüpfung wird hierin auch als Transposition bezeichnet.

Bei der Durchführung des Sloning-Verfahrens kann die Situation auftreten, dass im Rahmen der sequenziellen Addition von Splinker-Molekülen aus der Bibliothek, die hierin auch als Aufbausynthese bezeichnet wird, fehlerhafte Zwischenprodukte dergestalt entstehen, dass (beispielsweise durch unvollständiges Schneiden des für des Splinker-Molekül spezifischen Typ IIS Restriktionsenzyms) in dem Reaktionsansatz sowohl korrekt verlängerte, als auch unvollständig verlängerte Anchor-bzw. Splinker-Moleküle vorhanden sind. Das Vorhandensein derartiger unvollständig verlängerter Moleküle würde bei deren Verwendung als ein Element in der Transposition zu einer fehlerhaften Sequenz führen. Aus diesem Grunde besteht der Bedarf, das Sloning-Verfahren so auszubilden, dass sicher gestellt wird, dass nur korrekte Sequenzen, insbesondere auf der Ebene der Anchor-Moleküle, in der Transpositionphase, d. h. der parallelen Verknüpfung der aufgebauten Genfragmente, verwendet werden. Im Rahmen der vorliegenden Erfindung erfolgt dies dadurch, dass vor der letzten Ligation des Anchor-Moleküls mit einem Splinker, d.h. bevor das daraus erhaltene Ligationsprodukt transponiert wird, ein modifiziertes Splinker-Molekül verwendet wird, wobei die Modifikation darin besteht, dass der Splinker, grundsätzlich vergleichbar dem Anchor-Molekül, eine Modifizierung trägt, die eine Kopplung an eine feste Matrix erlaubt. Da es nicht möglich ist, Molekülen des Reaktionsansatzes, die im vorherigen Schritt nicht mit dem Splinker-spezifischen Restriktionsenzym vom Typ IIS gespalten wurden, ein derartiges modifiziertes Splinker-Molekül anzuligieren, sind nur die Ligationsprodukte des korrekt verlängerten Anchors mit dem jeweils letzten (modifizierten) Splinker-Molekül mit einer Modifizierung versehen. Die unvollständig verlängerten Anchor-Moleküle weisen als das Produkt aus einer vorherigen Ligation die darin verwendeten Splinker-Moleküle auf, die keine derartige Modifizierung tragen (siehe Fig. 1 (A)).

In einem weiteren Schritt (B) erfolgt die Spaltung der in dem Reaktionsansatz vorhandenen verlängerten Anchor-Moleküle mit dem Anchor-spezifischen Restriktionsenzym vom Typ IIS. Die nicht an die feste Matrix gekoppelten Spaltprodukte sind zum einen korrekt verlängerte Splinker-Moleküle, die eine Markierung tragen sowie inkomplett verlängerte Splinker-Moleküle, die keine Markierung tragen. (B). Die solchermaßen erhaltenen verlängerten Splinker-Moleküle werden bevorzugter Weise in einen neuen Reaktionsansatz bzw. neues Reaktionsgefäß überführt. Dieses Gefäß weist eine Oberfläche als feste Matrix auf, welche eine Kopplung mittels der an dem korrekt verlängerten Splinker-Molekül vorhandenen Modifizierung erlaubt. Dadurch kommt es zu einer Immobilisierung der korrekt verlängerten Splinker-Moleküle, wohingegen die nicht korrekt verlängerten Splinker-Moleküle, denen die eine Kopplung an die feste Matrix erlaubende Modifizierung fehlt, nicht an die feste Matrix binden. Durch einen oder mehrere Waschschritte werden die nicht korrekt verlängerten Splinker-Moleküle aus dem Reaktionsansatz entfernt. An der Matrix gebunden verbleibt das korrekt verlängerte Splinker-Molekül; das bei Verwendung von spaltbaren Modifikationen von dieser festen Matrix wieder entfernt werden kann und sodann mit dem ursprünglich verwendeten Anchor-Molekül ligiert werden kann (C) wobei die Sequenz des Anchormoleküls identisch ist mit der Sequenz des ursprünglich verwendeten Anchormoleküls.

Alternativ und für den Fall, dass die Modifikation keine Abspaltung des korrekt verlängerten Splinker-Moleküls erlaubt, kann zu dem Reaktionsansatz, der lediglich das immobilisierte, korrekt verlängerte Splinker-Molekül enthält, ein Anchor-Molekül hinzugegeben werden, welches zwar eine Modifizierung trägt, die eine Kopplung an eine feste Matrix erlaubt, jedoch in diesem Falle nicht an einer Oberfläche gebunden wird. Die Bindung des eine Modifizierung tragenden Anchor-Moleküls an die Oberfläche kann zum einen dadurch verhindert werden, dass der Anchor eine andere Modifizierung aufweist als die, die für die Kopplung des korrekt verlängerten Splinker-Moleküls an die Oberfläche verwendet wird. In dem Fall identischer Modifizierungen können vor Zugabe des Anchor-Moleküls die freien Bindungsstellen der Matrix durch Zugabe des die Modifizierung vermittelnden Moleküls abgesättigt oder blockiert werden. Dies kann bei Verwendung des Biotin-Streptavidin-Systems beispielsweise durch Zugabe von löslichem Biotin erfolgen. Dadurch bindet das Anchor-Moleküls an das an die feste Matrix gekoppelte, korrekt verlängerte Splinker-Molekül in Folge Hybridisierung bzw. Basenpaarung der überstehenden Enden, der sich eine Ligation unter Verwendung geeigneter Ligasen anschließt (D).

Das solchermaßen erhaltene Ligationsprodukt wird sodann mit einem Splinker-spezifischen Restriktionsenzym vom Typ IIS gespalten. Das dadurch erhaltene, im Überstand des Reaktionsgefäßes enthaltene Spaltprodukt wird in ein neues Reaktionsgefäß überführt, wobei hier das korrekt verlängerte Anchor-Molekül zuerst in Lösung vorliegt, aber in Folge der eine Kopplung an eine feste Matrix erlaubenden Modifizierung an die feste Matrix des neuen Reaktionsgefäßes immobilisiert (E).

In einem nächsten Schritt wird dem korrekt verlängerten, an die feste Matrix gekoppelten Anchor-Molekül ein geeignetes Splinker-Molekül hinzugegeben. Auf Grund der jeweils komplementären überhängenden Enden des korrekt verlängerten Anchor-Moleküls und des Splinker-Moleküls können diese miteinander hybridisieren. Das solchermaßen erhaltene Anlagerungsprodukt kann sodann mittels einer Ligaseaktivität ligiert werden (F).

In einem letzten Schritt (H) wird das solchermaßen erhaltene Ligationsprodukt mit einem Anchor-spezifischen Restriktionsenzym vom Typ IIS gespalten und ein korrekt verlängertes Splinker-Molekül in Lösung erhalten, welche sodann Gegenstand einer Transposition sein kann/können.

Zusammenfassend lässt sich feststellen, dass mittels dieser spezifischen Verfahrungsführung gewährleistet wird, dass störende, d. h. in ihrer Sequenz nicht korrekte Oligonukleotide, aus dem Reaktionsansatz entfernt werden können. Die Notwendigkeit der Entfernung nicht korrekt verlängerter Oligonukleotide ist maßgeblich für eine besonders gute Ausbeute und Korrektheit des zu synthetisierenden Oligonukleotids, welches im Rahmen des Sloning-Verfahrens als Anchor- oder Splinker-Molekül verwendet wird.

Prinzipiell kann dieses Verfahren auch nach jedem Syntheseschritt angewandt werden. In diesem Fall findet die eigentliche Gensynthese dann in Lösung statt. Inkorrekte Zwischenprodukte werden hingegen durch die Bindung an eine geeignete Festphase aus dem Reaktionsansatz entfernt. Fig. 18 bis 20 zeigen die verschiedenen Schritte einer solchen Gensynthese in Lösung, bei der es sich um eine weitere Ausführungsform des Sloning-Verfahrens handelt.

In Schritt (A) wird hierbei ein Anchor-Molekül, das in diesem Falle keine Modifizierung trägt, die eine Kopplung an eine feste Phase erlauben würde, mit einem Splinker-Molekül ligiert. In diesem Fall trägt das Splinker-Molekül eine Modifizierung, die an eine Kopplung an eine feste Matrix erlaubt. Diese Reaktion erfolgt in Lösung, d. h. weder das hinzugesetzte Splinker-Molekül, noch das Ligationsprodukt aus dem Anchor-Molekül und dem Splinker-Molekül ist zunächst an eine Festphase gebunden (A). Auf diese Weise wird eine permanente Selektion auf korrekt verlängerte und geschnittene Zwischenprodukte erreicht. Ein weiterer Vorteil dieser Vorgehensweise liegt darin begründet, dass sowohl Ligation- wie auch Restriktionsreaktionen in Lösung in der Regel mit einer höheren Effizienz ablaufen als an der Festphase.

Nach erfolgter Inaktivierung der Ligase wird mittels der Splinker-spezifischen Restriktionsendonuklease vom Typ IIS gespalten. In der Folge liegen im Reaktionsansatz ein verlängertes Anchor-Molekül sowie das Splinker-Molekül wiederum in Lösung vor. Die solchermaßen erhaltenen Spaltprodukte werden sodann in ein neues Reaktionsgefäß übergeführt, in dem in Folge des Vorhandenseins einer Modifizierung am Splinker-Molekül (im vorliegenden Falle ein Biotin) dieses an die feste Matrix bindet. Neben dem gespaltenen Splinker-Molekül sind in dem Reaktionsansatz des weiteren nicht geschnittene Ligationsprodukte aus Schritt (A) sowie nicht ligierte Splinker-Moleküle enthalten. Alle drei Splinker-Molekül-Derivate binden an die feste Phase, nicht jedoch das aus Schritt (B) erhaltene verlängerte Anchor-Molekül, das weiter in Lösung vorliegt (C). Der Überstand aus Schritt (C), d. h. das verlängerte Anchor-Molekül wird sodann in ein neues Reaktionsgefäß überführt und dort mit einem weiteren Splinker-Molekül umgesetzt, welches an seinen Enden komplementär ist zu dem Ende des verlängerten Anchor-Moleküls (E). Das neue Splinker-Molekül weist wiederum eine Modifizierung auf, die eine Kopplung an eine feste Matrix erlaubt.

Das solchermaßen erhaltene Ligationsprodukt wird nach Inaktivierung der Ligase mit der Splinker-spezifischen Restriktionsendonuklease vom Typ IIS gespalten und liefert damit ein weiter verlängertes Anchor-Molekül.

Diese Vorgehensweise kann grundsätzlich beliebig oft wiederholt werden. Der besondere Vorteil dieser Ausführungsform des Sloning-Verfahrens besteht darin, dass eine Aufreinigung eines im Sloning-Verfahren verwendbaren Oligonukleotides gewährleistet wird. Diese Vorgehensweise kann grundsätzlich auf eine jede Ebene des Sloning-Verfahrens angewandt werden.

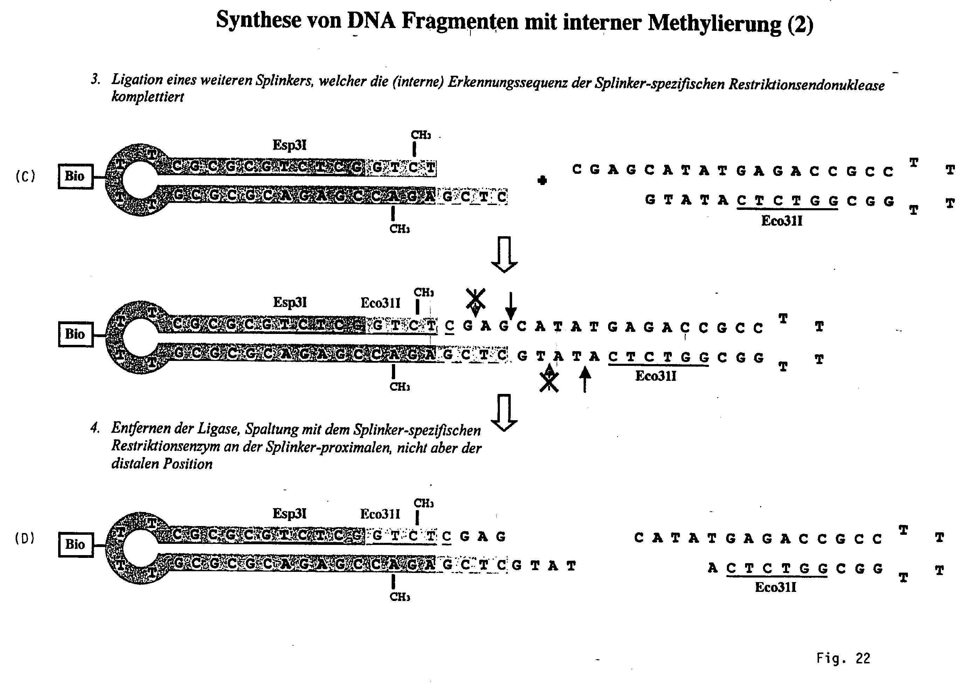

Die Figs. 21 und 22 zeigen die wesentlichen Schritte bei der Synthese von DNA-Fragmenten mit interner Methylierung gemäß der vorliegenden Erfindung, die Schritte Teil des Sloning-Verfahrens sein können. Insoweit wird hiermit eine weitere Ausführungsform des Sloning-Verfahrens offenbart.

Im Zusammenhang mit der Beschreibung der Figuren 21 und 22 bezeichnet der Begriff des Anchors oder Anchor-Moleküls ein Oligonukleotid gemäß aa) bzw. ba) des Sloning-Verfahres und der Begriff des Splinkers oder Splinker-Moleküls ein Oligonukleotid gemäß ab) bzw. bb) des Sloning-Verfahrens.

Im Rahmen der Synthese von Nukleinsäuremolekülen wie beispielsweise Genfragmenten unter Verwendung des Sloning-Verfahrens wurde beobachtet, dass einige Sequenzen nicht synthetisiert werden können, was darin begründet liegt, dass durch die Ligation eines Anchor-Moleküls sowie eines Splinker-Moleküls eine Erkennungsstelle für ein Restriktionsenzym ausgebildet wird, das dem Restriktionsenzym des Splinker-Moleküls bzw. dem Anchor-Molekül entspricht und in Folge dessen ein anderes Spaltungsereignis eintreten würde, als für die korrekte Verlängerung eines Anchor- bzw. Splinker-Moleküls erforderlich wäre.

Erfindungsgemäß wird diese Beschränkung der Verwendung des Sloning-Verfahrens dadurch umgangen, dass die durch die spezifische Kombination aus Anchor- und Splinkersequenz entstehenden zusätzlichen Erkennungsstellen für entweder das Anchor- oder das Splinker-spezifische Restriktionsenzym vom Typ IIS methyliert sind und damit nicht geschnitten werden können. Die für die Durchführung des Sloning Verfahrens notwendigen Erkennungssequenzen in den konstanten Bereichen der Splinker bzw. Anchor sind hingegen nicht methyliert und können daher nach wie vor gespalten werden.

Konkret wird dabei so vorgegangen, dass im Rahmen einer sequenziellen Ligation von teilweise methylierten Splinker-Molekülen aus einer Bibliothek vorgesehen ist, dass die 5'-Überhangsequenzen ((5'-> 3') GTC, CTC, TCT) und die folgenden 3'- Endsequenzen (AGA, ACG, GAC) methyliert sind. In Schritt (A) weist das eine eine Kopplung an eine feste Matrix erlaubende Modifizierung tragende Anchor-Molekül in seinem Überhang am Adenosin eine Methylierung auf. Das zu dem Anchor-Molekül komplementäre Splinker-Molekül weist in seinem Überhang ebenfalls eine Methylierung auf, im vorliegenden Falle am Cytosin. Dabei ist es im Rahmen der vorliegenden Erfindung, dass auch eine Methylierung in anderen Teilen als den Überhängen möglich ist, solange dadurch nicht eine der beiden notwendigen Erkennungsstellen für ein Restriktionsenzym vom Typ IIS funktional inaktiviert wird.

Nach Ligation der beiden Moleküle wird das in (A) dargestellte Ligationsprodukt erhalten, das nach Entfernen der Ligase mit der Splinker-spezifischen Restriktionsendonuklease vom Typ IIS gespalten wird. In Folge dieser Spaltung verlängert sich das Anchor-Molekül und weist nun in den beiden Strängen des Doppelstranges eine Methylierung auf. An das solchermaßen erhaltene, verlängerte Anchor-Molekül wird ein weiteres Splinker-Molekül ligiert, wobei das Splinker-Molekül dazu führt, dass in dem in Schritt (C) erhaltenen Ligationsprodukt neben der im Splinker-Molekülteil enthaltenen Splinker-spezifischen Restriktionsenzym-Erkennungsstelle eine weitere Erkennungsstelle für das Splinker-spezifische Restriktionsenzym gebildet wird. Wie in (C) dargestellt würde damit neben der im konkreten Beispiel angegebenen Erkennungsstelle für Eco31I im Splinker-Molekülteil im Ligationsbereich noch eine weitere Erkennungssequenz für Eco31I entstehen, mit der Folge, dass bei Verwendung von Eco31I, typischer Weise nach Entfernen der Ligase, drei Spaltprodukte entstünden. In Folge der Methylierung der zweiten, durch die Ligation des Splinker-Moleküls und des Anchor-Moleküls entstandenen Erkennungsstelle für Eco31I ist diese jedoch für das Splinker-spezifische Restriktionsenzym nicht zugänglich. Infolgedessen schneidet Eco31I dieses Ligationsprodukt lediglich Splinker-proximal, nicht aber Splinkerdistal. Demnach kann das Anchor-Molekül korrekt verlängert und für die weitere Aufbausynthese im Rahmen des Sloning-Verfahrens eingesetzt werden.

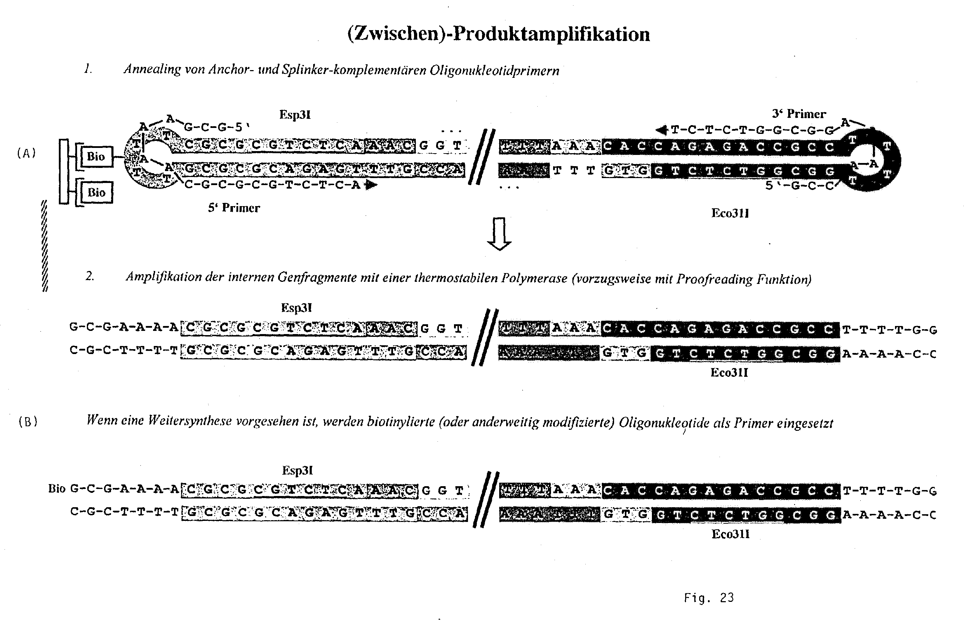

Fig. 23 zeigt ein Verfahren zur (Zwischen)- Produktamplifikation, wie es an beliebiger Stelle des Sloning-Verfahrens durchgeführt werden kann. Typischer Weise erfolgt die (Zwischen)-Produktamplifikation an Ligationsprodukten, die im Rahmen des Sloning-Verfahrens entstehen, so beispielsweise im Rahmen der sogenannten Aufbausynthese, (d.h. ein- oder mehrmaliges Durchlaufen der Schritte aa) bis ag) bzw. ba) bis bg)) oder auch im Rahmen der Transpositionen. Derartige (Zwischen)- Produktamplifikationsschritte - empfehlen sich insbesondere dann, wenn die Konzentration eines (Zwischen)- Produktes so niedrig geworden ist, dass eine effiziente Durchführung der folgenden Schritte gefährdet ist.

Die (Zwischen)-Produktamplifikation erfolgt durch das als solches im Stand der Technik bekannte Verfahren der Polymerasekettenreaktion. Dabei wird so vorgegangen, dass Anchor- und Splinker-komplementäre Oligonukleotidprimer an ein Ligationsprodukt, wie es im Rahmen des Sloning-Verfahrens anfällt, anelliert werden. Bevorzugter Weise sind die Primer dabei komplementär zu dem konstanten Teil des Anchor-Moleküls bzw. Splinker-Moleküls, oder eines Teils davon. Der Vorteil bei dieser Gestaltung der Primer ist der, dass damit ein Paar von Primern ausreicht, um unabhängig von der aufgebauten Nukleinsäure, d.h. der Zielsequenz oder einem Teil davon, die Amplifikation des Produktes bzw. eines Zwischenproduktes des Sloning-Verfahrens vornehmen zu können. Es ist jedoch auch möglich und im Rahmen der vorliegenden Erfindung, dass die Primer teilweise oder vollständig an einen Bereich des Anchor- oder Splinker-Moleküls binden, der der aufgebauten Nukleinsäure entspricht. Unter komplementär soll dabei hierin verstanden werden, dass eine Nukleinsäure an eine andere Nukleinsäure vermittels Basenpaarung in Wechselwirkung tritt. Es wird seitens der Fachleute anerkannt werden, dass komplementär nicht notwendigerweise eine vollständige Komplementarität bedeutet. Vielmehr kann eine oder mehrere Fehlbasenpaarungen enthalten sein und auch ein oder mehrere Nukleotide keine Basenpaarung aufweisen.

Bei der Auswahl der Primer ist zu beachten, dass diese bevorzugter Weise nicht selbstkomplementär sein sollten. In Folge dessen hybridisieren die bei diesem Verfahren verwendeten Primer bevorzugter Weise nur mit 3-4 Nukleotiden der Klammer (des konstanten doppelsträngigen Bereichs unmittelbar vor dem Loop), dem Loop-Bereich des Anchor- bzw. Splinker-Moleküls selbst sowie den folgenden Nukleotiden (maximal zum Ende des konstanten Teils des 5'-Überhanges des Anchor-Moleküls bzw. Splinker-Moleküls (A)). Nach Annelieren der Anchor- und Splinker-spezifischen Primer erfolgt eine Amplifikation der internen Genfragmente mit einer thermostabilen Polymerase, wobei diese Polymerase bevorzugter Weise eine Proofreading-Funktion aufweist. Typischer Weise werden die Primer in hohem Überschuss dem Reaktionsansatz zugesetzt. Für den Fall, dass eine Weitersynthese unter Verwendung des solchermaßen amplifizierten (Zwischen-) Produktes vorgesehen ist, werden bevorzugter Weise modifizierte Oligonukleotide als Primer eingesetzt, die eine Bindung der Oligonukleotide an eine feste Matrix erlauben. Das Ergebnis der (Zwischen)-Produktamplifikation weist keine Loop-Struktur mehr auf, die den Strang und Gegenstrang verbindet. Stattdessen liegt das amplifizierte Ligationsprodukt als Doppelstrangstruktur zweier Einzelstränge vor. Diese hierin auch als bipartite Struktur bezeichneten Moleküle können aber im Rahmen des Sloning-Verfahrens ebenfalls als Ausgangsprodukt verwendet werden.

Es ist im Rahmen der vorliegenden Erfindung, dass die verschiedenen Aspekte beliebig miteinander kombiniert werden können und somit eine Vielzahl von Ausführungsformen des Sloning-Verfahrens möglich sind.

Die in der vorangehenden Beschreibung, den Ansprüchen und den Zeichnungen offenbarten Merkmale der Erfindung können sowohl einzeln als auch in beliebiger Kombination zur Verwirklichung der Erfindung in ihren verschiedenen Ausführungsformen wesentlich sein.

## Patentansprüche

1. Verfahren zur Herstellung eines Nukleinsäuremoleküls umfassend die Schritte
a) Bereitstellen eines Oligonukleotids, das hergestellt ist durch die folgenden Schritte:
aa) Bereitstellen eines teilweise doppelsträngigen Oligonukleotids mit einem 5'-Überhang, das eine Erkennungssequenz für ein TypIIS Restriktionsenzym enthält, welches außerhalb seiner Erkennungssequenz schneidet, und eine Modifizierung trägt, die eine Kopplung an eine feste Matrix erlaubt, wobei der 5'-Überhang eine Länge von 3 Nukleotiden umfasst,
ab) Zugabe eines weiteren, mindestens teilweise doppelsträngigen Oligonukleotids mit einem 5'-Überhang und einer anderen Erkennungssequenz für ein TypIIS Restriktionsenzym, welches außerhalb seiner Erkennungssequenz schneidet, als in Schritt aa), wobei der 5'-Überhang eine Länge von 3 Nukleotiden umfasst,
ac) Ligation der Oligonukleotide aus Schritt aa) und ab) in der durch die Blockierung der nicht zu ligierenden Enden festgelegten Orientierung,
ad) Entfernen nicht verbrauchter Reaktanden sowie Enzyme,
ae) Spaltung des Ligationsprodukts aus Schritt ac) mit einem TypIIS Restriktionsenzym, welches außerhalb seiner Erkennungssequenz schneidet, wobei die Spaltung in der Nukleinsäuresequenz des Oligonukleotids aus Schritt ab) stattfindet,
af) Abtrennen des Reaktionsgemisches vom in Schritt ae) erhaltenen verlängerten Oligonukleotid aus Schritt aa),
b) Bereitstellen eines weiteren Oligonukleotids, das hergestellt ist durch die Schritte:
ba) Bereitstellen eines teilweise doppelsträngigen Oligonukleotids mit einem 5'-Überhang, das eine Erkennungssequenz für ein TypIIS Restriktionsenzym enthält, welches außerhalb seiner Erkennungssequenz schneidet, und eine Modifizierung trägt, die eine Kopplung an eine feste Matrix erlaubt, wobei der 5'-Überhang eine Länge von 3 Nukleotiden umfasst,
bb) Zugabe eines weiteren, mindestens teilweise doppelsträngigen Oligonukleotids mit einem 5'-Überhang und mit einer anderen Erkennungssequenz für ein TypIIS Restriktionsenzym, welches außerhalb seine Erkennungssequenz schneidet, als in Schritt ba), wobei der 5'-Überhang eine Länge von 3 Nukleotiden umfasst,
bc) Ligation der Oligonukleotide aus Schritt ba) und bb) in der durch die Blockierung der nicht zu ligierenden Enden festgelegten Orientierung,
bd) Entfernen nicht verbrauchter Reaktanden sowie Enzyme,
be) Spaltung des Ligationsproduktes aus Schritt bc) mit einem TypIIS Restriktionsenzym, welches außerhalb seiner Erkennungssequenz schneidet, wobei die Spaltung im Oligonukleotid in Schritt bb) stattfindet,
bf) Abtrennen des so verlängerten Nukleinsäuremoleküls vom Reaktionsgemisch,
c) Ligation der Oligonukleotide aus Schritt a) und b) in der durch die Blockierung der nicht zu ligierenden Enden festgelegten Orientierung,
d) Entfernen nicht verbrauchter Reaktanden sowie Enzyme,
e) Spaltung des Ligationsproduktes aus Schritt c) mit einem TypIIS Restriktionsenzym, welches außerhalb seiner Erkennungssequenz schneidet, wobei die Spaltung im Oligonukleotid aus Schritt a) oder b) stattfindet,
f) Abtrennen des so verlängerten Nukleinsäuremoleküls vom Reaktionsgemisch,
**dadurch gekennzeichnet, dass** das Oligonukleotid von Schritt ab) die Erkennungssequenz eines Restriktionsenzyms vom Typ IIS aufweist, das einen drei Nukleotide langen Überhang erzeugt, solange die Schritte ab) bis ae) wiederholt werden und das Oligonukleotid von Schritt ab) die Erkennungssequenz eines Restriktionsenzyms vom Typ IIS aufweist, das einen anderen als einen drei Nukleotid langen Überhang erzeugt, beim letzten Durchlaufen der Schritte ab) bis ae) und/oder
das Oligonukleotid von Schritt bb) die Erkennungssequenz eines Restriktionsenzyms vom Typ IIS aufweist, das einen drei Nukleotide langen Überhang erzeugt, solange die Schritte bb) bis be) wiederholt werden und das Oligonukleotid von Schritt bb) die Erkennungssequenz eines Restriktionsenzyms vom Typ IIS aufweist, das einen anderen als einen drei Nukleotid langen Überhang erzeugt, beim letzten Durchlaufen der Schritte bb) bis be).

2. Verfahren nach Anspruch 1, **dadurch gekennzeichnet, dass** im Anschluss an Schritt af) mindestens einmalig die Schritte ab) bis af) wiederholt werden und/oder im Anschluss an Schritt bf) mindestens einmalig die Schritte bb) bis be) wiederholt werden, wobei im Anschluss an die letzte Ligation in Schritt bc) und Entfernen nicht verbrauchter Reaktanden sowie Enzyme das Ligationsprodukt mit einem TypIIS Restriktionsenzym geschnitten wird, wobei die Spaltung in dem Oligonukleotid aus Schritt ba) stattfindet.

3. Verfahren zur Herstellung einer Gruppe von Nukleinsäuremolekülen umfassend die Schritte
a) Bereitstellen eines Oligonukleotids, das hergestellt ist durch die folgenden Schritte:
aa) Bereitstellen eines Oligonukleotids, das eine Erkennungssequenz für ein TypIIS Restriktionsenzym enthält, welches außerhalb seiner Erkennungssequenz schneidet, und eine Modifizierung trägt, die eine Kopplung an eine feste Matrix erlaubt,
Kopplung des Oligonukleotids an die feste Matrix
ab) Zugabe eines weiteren, mindestens teilweise doppelsträngigen Oligonukleotids mit einer anderen Erkennungssequenz für ein TypIIS Restriktionsenzym, welches außerhalb seiner Erkennungssequenz schneidet, als in Schritt aa),,
ac) Ligation der Oligonukleotide aus Schritt aa) und ab) in der durch die Blockierung der nicht zu ligierenden Enden festgelegten Orientierung,
ad) Entfernen nicht verbrauchter Reaktanden sowie Enzyme,
ae) Spaltung des Ligationsprodukts aus Schritt ac) mit einem TypIIS Restriktionsenzym, welches außerhalb seiner Erkennungssequenz schneidet, wobei die Spaltung in der Nukleinsäuresequenz des Oligonukleotids aus Schritt ab) stattfindet,
af) Abtrennen des Reaktionsgemisches vom in Schritt ae) erhaltenen verlängerten Oligonukleotid aus Schritt aa),
b) Bereitstellen eines weiteren Oligonukleotids, das hergestellt ist durch die Schritte:
ba) Bereitstellen eines Oligonukleotids, das eine Erkennungssequenz für ein TypIIS Restriktionsenzym enthält, welches außerhalb seiner Erkennungssequenz schneidet, und eine Modifizierung trägt, die eine Kopplung an eine feste Matrix erlaubt mit einem Ende an eine feste Matrix,
Kopplung des Oligonukleotids an die feste Matrix,
bb) Zugabe eines weiteren, mindestens teilweise doppelsträngigen Oligonukleotids mit einer anderen Erkennungssequenz für ein TypIIS Restriktionsenzym, welches außerhalb seine Erkennungssequenz schneidet, als in Schritt ba),
bc) Ligation der Oligonukleotide aus Schritt ba) und bb) in der durch die Blockierung der nicht zu ligierenden Enden festgelegten Orientierung,
bd) Entfernen nicht verbrauchter Reaktanden sowie Enzyme,
be) Spaltung des Ligationsproduktes aus Schritt bc) mit einem TypIIS Restriktionsenzym, welches außerhalb seiner Erkennungssequenz schneidet, wobei die Spaltung im Oligonukleotid in Schritt bb) stattfindet,
bf) Abtrennen des so verlängerten Nukleinsäuremoleküls vom Reaktionsgemisch,
c) Ligation der Oligonukleotide aus Schritt a) und b) in der durch die Blockierung der nicht zu ligierenden Enden festgelegten Orientierung,
d) Entfernen nicht verbrauchter Reaktanden sowie Enzyme,
e) Spaltung des Ligationsproduktes aus Schritt c) mit einem TypIIS Restriktionsenzym, welches außerhalb seiner Erkennungssequenz schneidet, wobei die Spaltung im Oligonukleotid aus Schritt a) oder b) stattfindet,
f) Abtrennen des so verlängerten Nukleinsäuremoleküls vom Reaktionsgemisch,
**dadurch gekennzeichnet, dass** beim letzten Wiederholen der Schritte ab) bis af) das in Schritt ab) zugegebene Oligonukleotid eine Modifizierung trägt, die eine Kopplung an eine feste Matrix erlaubt und
nach dem letzten Wiederholen der Schritte ab) bis af) als Schritt ah) das Ligationsprodukt aus Schritt ac) mit einem TypIIS Restriktionsenzym geschnitten wird, welches außerhalb seiner Erkennungssequenz schneidet, wobei die Spaltung in der Nukleinsäuresequenz des Oligonukleotids aus Schritt aa) stattfindet und das Spaltungsprodukt von dem an der festen Matrix gekoppelten Oligonukleotid freigesetzt wird
und das freigesetzte Spaltprodukt in mindestens zwei Reaktionsansätze aufgeteilt wird.

4. Verfahren nach Anspruch 3, **dadurch gekennzeichnet, dass** im Anschluss an Schritt af) mindestens einmalig die Schritte ab) bis af) wiederholt werden und/oder im Anschluss an Schritt bf) mindestens einmalig die Schritte bb) bis bf) wiederholt werden, wobei im Anschluss an die letzte Ligation in Schritt bc) und Entfernen nicht verbrauchter Reaktanden sowie Enzyme das Ligationsprodukt mit einem TypIIS Restriktionsenzym geschnitten wird, wobei die Spaltung in dem Oligonukleotid aus Schritt ba) stattfindet.

5. Verfahren nach Anspruch 3 oder 4, **dadurch gekennzeichnet, dass** das in Schritt ah) freigesetzte Spaltprodukt über die Modifizierung an eine feste Matrix gekoppelt wird und die keine Modifikation aufweisenden Spaltprodukte aus dem Reaktionsansatz entfernt werden.

6. Verfahren nach Anspruch 5, **dadurch gekennzeichnet, dass** in jedem der Reaktionsansätze zu dem an die feste Matrix gekoppelten Spaltprodukt aus Schritt ah) als Schritt ai) ein Oligonukleotid zugegeben wird, das eine Erkennungssequenz für ein TypIIS Restriktionsenzym enthält, welche verschieden ist von der Erkennungssequenz für ein Typ IIS Restriktionsenzym des Spaltproduktes aus Schritt ah), und eine Modifizierung trägt, die eine Kopplung an eine feste Matrix erlaubt, und als Schritt ak) eine Ligation des Spaltproduktes aus Schritt ah) mit dem Oligonukleotid erfolgt.

7. Verfahren nach Anspruch 6, **dadurch gekennzeichnet, dass** das Ligationsprodukt aus Schritt ak) mit einem TypIIS Restriktionsenzym geschnitten wird, das außerhalb seiner Erkennungssequenz schneidet, wobei die Spaltung in der Nukleinsäuresequenz des Spaltproduktes aus Schritt ah) erfolgt.

8. Verfahren nach Anspruch 6 oder 7, **dadurch gekennzeichnet, dass** das in Schritt ai) zugegebene Oligonukleotid in jedem Reaktionsansatz eine andere Sequenz aufweist.

9. Verfahren nach einem der Ansprüche 3 bis 8, **dadurch gekennzeichnet, dass** die den Reaktionsansätzen zugegebenen Oligonukleotide gleiche einzelsträngige Überhänge aufweisen.

10. Verfahren nach einem der Ansprüche 3 bis 9, **dadurch gekennzeichnet, dass** sich die Oligonukleotide in einem Bereich unterscheiden, der verschieden ist von dem einzelsträngigen Bereich, bevorzugterweise in einem Bereich unterscheiden, der eine Folge von Nukleotiden umfasst, die sich dem einzelsträngigen Bereich des Oligonukleotids anschließen.

11. Verfahren nach Anspruch 10, **dadurch gekennzeichnet, dass** die Folge von Nukleotiden eine Länge von 1 bis 10 Nukleotide, bevorzugter Weise 3 bis 6 Nukleotide umfasst.

12. Verfahren nach einem der Ansprüche 3 bis 11, **dadurch gekennzeichnet, dass** in einem Schritt al) eine Spaltung des in Schritt ai) zugegebenen Oligonukleotids erfolgt.

13. Verfahren nach Anspruch 12, **dadurch gekennzeichnet, dass** die Schritte ab) bis ak) und/oder al) zumindest einmal wiederholt werden.

14. Verfahren nach Anspruch 13, **dadurch gekennzeichnet, dass** nach dem letzten Wiederholen als Schritt am) die an die Festphasen gekoppelten Oligonukleotide mit einem weiteren Oligonukleotid gemäß ab) ligiert werden, wobei sich diese Oligonukleotide in den verschiedenen Reaktionsansätzen in der Sequenz des einzelsträngigen Überhanges und optional in der Sequenz des direkt angrenzenden doppelsträngigen Bereichs voneinander unterscheiden.

15. Verfahren zur Herstellung eines Nukleinsäuremoleküls umfassend die Schritte
a) Bereitstellen eines Oligonukleotids, das hergestellt ist durch die folgenden Schritte:
aa) Bereitstellen eines Oligonukleotids, das eine Erkennungssequenz für ein TypIIS Restriktionsenzym enthält, welches außerhalb seiner Erkennungssequenz schneidet, und eine Modifizierung trägt, die eine Kopplung an eine feste Matrix erlaubt,
Kopplung des Oligonukleotids an die feste Matrix
ab) Zugabe eines weiteren, mindestens teilweise doppelsträngigen Oligonukleotids mit einer anderen Erkennungssequenz für ein TypIIS Restriktionsenzym, welches außerhalb seiner Erkennungssequenz schneidet, als in Schritt aa),,
ac) Ligation der Oligonukleotide aus Schritt aa) und ab) in der durch die Blockierung der nicht zu ligierenden Enden festgelegten Orientierung,
ad) Entfernen nicht verbrauchter Reaktanden sowie Enzyme,
ae) Spaltung des Ligationsprodukts aus Schritt ac) mit einem TypIIS Restriktionsenzym, welches außerhalb seiner Erkennungssequenz schneidet, wobei die Spaltung in der Nukleinsäuresequenz des Oligonukleotids aus Schritt ab) stattfindet,
af) Abtrennen des Reaktionsgemisches vom in Schritt ae) erhaltenen verlängerten Oligonukleotid aus Schritt aa),
b) Bereitstellen eines weiteren Oligonukleotids, das hergestellt ist durch die Schritte:
ba) Bereitstellen eines Oligonukleotids, das eine Erkennungssequenz für ein TypIIS Restriktionsenzym enthält, welches außerhalb seiner Erkennungssequenz schneidet, und eine Modifizierung trägt, die eine Kopplung an eine feste Matrix erlaubt mit einem Ende an eine feste Matrix,
Kopplung des Oligonukleotids an die feste Matrix,
bb) Zugabe eines weiteren, mindestens teilweise doppelsträngigen Oligonukleotids mit einer anderen Erkennungssequenz für ein TypIIS Restriktionsenzym, welches außerhalb seiner Erkennungssequenz schneidet, als in Schritt ba),
bc) Ligation der Oligonukleotide aus Schritt ba) und bb) in der durch die Blockierung der nicht zu ligierenden Enden festgelegten Orientierung,
bd) Entfernen nicht verbrauchter Reaktanden sowie Enzyme,
be) Spaltung des Ligationsproduktes aus Schritt bc) mit einem TypIIS Restriktionsenzym, welches außerhalb seiner Erkennungssequenz schneidet, wobei die Spaltung im Oligonukleotid in Schritt bb) stattfindet,
bf) Abtrennen des so verlängerten Nukleinsäuremoleküls vom Reaktionsgemisch,
c) Ligation der Oligonukleotide aus Schritt a) und b) in der durch die Blockierung der nicht zu ligierenden Enden festgelegten Orientierung,
d) Entfernen nicht verbrauchter Reaktanden sowie Enzyme,
e) Spaltung des Ligationsproduktes aus Schritt c) mit einem TypIIS Restriktionsenzym, welches außerhalb seiner Erkennungssequenz schneidet, wobei die Spaltung im Oligonukleotid aus Schritt a) oder b) stattfindet,
f) Abtrennen des so verlängerten Nukleinsäuremoleküls vom Reaktionsgemisch,
**dadurch gekennzeichnet, dass** beim letzten Wiederholen der Schritte ab) bis af) das in Schritt ab) zugegebene Oligonukleotid eine Modifizierung trägt, die eine Kopplung an eine feste Matrix erlaubt.

16. Verfahren nach Anspruch 15, **dadurch gekennzeichnet, dass** im Anschluss an Schritt af) mindestens einmalig die Schritte ab) bis af) wiederholt werden und/oder im Anschluss an Schritt bf) mindestens einmalig die Schritte bb) bis bf) wiederholt werden, wobei im Anschluss an die letzte Ligation in Schritt bc) und Entfernen nicht verbrauchter Reaktanden sowie Enzyme das Ligationsprodukt mit einem TypIIS Restriktionsenzym geschnitten wird, wobei die Spaltung in dem Oligonukleotid aus Schritt ba) stattfindet,

17. Verfahren nach Anspruch 15 oder 16, **dadurch gekennzeichnet, dass** nach dem letzten Wiederholen der Schritte ab) bis af) als Schritt ah) das Ligationsprodukt aus Schritt ac) mit einem TypIIS Restriktionsenzym geschnitten wird, welches außerhalb seiner Erkennungssequenz schneidet, wobei die Spaltung im Oligonukleotid aus Schritt aa) stattfindet und das Spaltprodukt von dem an der festen Matrix gekoppelten Oligonukleotid freigesetzt wird.

18. Verfahren nach Anspruch 17, **dadurch gekennzeichnet, dass** das in Schritt ah) freigesetzte Spaltungsprodukt in einem neuen Reaktionsgefäß über die Modifizierung an eine feste Matrix gekoppelt wird und die keine Modifikation aufweisenden Spaltungsprodukte aus dem die feste Matrix und das in Schritt ah) freigesetzte Spaltprodukt enthaltenden Reaktionsansatz entfernt werden.

19. Verfahren nach Anspruch 18, **dadurch gekennzeichnet, dass** zu dem an die feste Matrix gekoppelten Spaltprodukt aus Schritt ah) ein Oligonukleotid zugegeben wird, das eine Erkennungssequenz für ein TypIIS Restriktionsenzym enthält, welche verschieden ist von der Erkennungssequenz für ein Typ IIS Restriktionsenzym, des Spaltproduktes aus Schritt ah) und eine Modifizierung trägt, die eine Kopplung an eine feste Matrix erlaubt, und als Schritt ai) eine Ligation des Spaltproduktes aus Schritt ah) mit dem Oligonukleotid erfolgt.

20. Verfahren nach Anspruch 19, **dadurch gekennzeichnet, dass** das Ligationsprodukt aus Schritt ai) mit einem TypIIS Restriktionsenzym geschnitten wird, das außerhalb seiner Erkennungssequenz schneidet, wobei die Spaltung in der Nukleinsäuresequenz des Spaltproduktes aus Schritt ah) erfolgt.

21. Verfahren nach einem der Ansprüche 15 bis 20, **dadurch gekennzeichnet, dass** die Schritte unter Verwendung eines oder mehrerer modifizierter Oligonukleotide gemäß Schritt ab) wiederholt wird.

22. Verfahren zur Herstellung eines Nukleinsäuremoleküls umfassend die Schritte
a) Bereitstellen eines Oligonukleotids, das hergestellt ist durch die folgenden Schritte:
aa) Bereitstellen eines Oligonukleotids, das eine Erkennungssequenz für ein TypIIS Restriktionsenzym enthält, welches außerhalb seiner Erkennungssequenz schneidet,
ab) Zugabe eines weiteren, mindestens teilweise doppelsträngigen Oligonukleotids mit einer anderen Erkennungssequenz für ein TypIIS Restriktionsenzym, welches außerhalb seiner Erkennungssequenz schneidet, als in Schritt aa) und eine Modifizierung trägt, die eine Kopplung an eine feste Matrix erlaubt,
ac) Ligation der Oligonukleotide aus Schritt aa) und ab) in der durch die Blockierung der nicht zu ligierenden Enden festgelegten Orientierung,
ad) Spaltung des Ligationsprodukts aus Schritt ac) mit einem TypIIS Restriktionsenzym, welches außerhalb seiner Erkennungssequenz schneidet, wobei die Spaltung in der Nukleinsäuresequenz des Oligonukleotids aus Schritt ab) stattfindet,
af) Abtrennen des keine Modifizierung tragenden Spaltproduktes aus Schritt ae) des Reaktionsgemisches,
b) Bereitstellen eines weiteren Oligonukleotids, das hergestellt ist durch die Schritte:
ba) Bereitstellen eines Oligonukleotids, das eine Erkennungssequenz für ein TypIIS Restriktionsenzym enthält,
bb) Zugabe eines weiteren, mindestens teilweise doppelsträngigen Oligonukleotids mit einer anderen Erkennungssequenz für ein TypIIS Restriktionsenzym, welches außerhalb seine Erkennungssequenz schneidet, als in Schritt ba) und eine Modifizierung trägt, die eine Kopplung an eine feste Matrix erlaubt,
bc) Ligation der Oligonukleotide aus Schritt ba) und bb) in der durch die Blockierung der nicht zu ligierenden Enden festgelegten Orientierung,
be) Spaltung des Ligationsproduktes aus Schritt bc) mit einem TypIIS Restriktionsenzym, welches außerhalb seine Erkennungssequenz schneidet, wobei die Spaltung im Oligonukleotid in Schritt bb) stattfindet,
bf) Abtrennen des keine Modifizierung tragenden Spaltproduktes aus Schritt be) des Reaktionsgemisches,
c) Ligation der Oligonukleotide aus Schritt a) und b) in der durch die Blockierung der nicht zu ligierenden Enden festgelegten Orientierung in Lösung,
d) Entfernen und/oder Inaktivieren nicht verbrauchter Reaktanden sowie Enzyme,
e) Spaltung des Ligationsproduktes aus Schritt c) mit einem TypIIS Restriktionsenzym, welches außerhalb seiner Erkennungssequenz schneidet, wobei die Spaltung im Oligonukleotid aus Schritt a) oder b) stattfindet,
f) Abtrennen des so verlängerten Nukleinsäuremoleküls vom Reaktionsgemisch.

23. Verfahren nach Anspruch 22, **dadurch gekennzeichnet, dass** im Anschluss an Schritt ac) als Schritt ad) nicht verbrauchte Reaktanden sowie Enzyme entfernt werden und/oder im Anschluss an Schritt af) mindestens einmalig die Schritte ab) bis af) wiederholt werden und/oder dass im Anschluss an Schritt bf) mindestens einmalig die Schritte bb) bis bf) wiederholt werden, wobei im Anschluss an die letzte Ligation in Schritt bc) und Entfernen und/oder Inaktivieren nicht verbrauchter Reaktanden sowie Enzyme das Ligationsprodukt mit einem TypIIS Restriktionsenzym geschnitten wird, wobei die Spaltung in dem Oligonukleotid aus Schritt ba) stattfindet.

24. Verfahren nach Anspruch 22 oder 23, weiter umfassend den Schritt, dass die keine Modifizierung tragenden Spaltprodukte in einen neuen Reaktionsansatz überführt werden.

25. Verfahren nach einem der Ansprüche 22 bis 24, wobei nach Schritt bc) der Schritt bd) folgt bestehend aus :
bd) Entfernen und/oder Inaktivieren nicht vebrauchter Reaktanden sowie Enzyme.

26. Verfahren nach einem der Ansprüche 1 bis 25 des Weiteren enthaltend ein Verfahren zur Amplifikation des enstehenden Ligationsproduktes, **gekennzeichnet durch** die Schritte:
a) Bereitstellen eines Ligationsproduktes;
b) Bereitstellen eines für das Oligonukleotid gemäß Schritt aa) und/oder ab) zumindest teilweise komplementären Primers,
c) Bereitstellen eines für das Oligonukleotid gemäß Schritt ab) und/oder bb) zumindest teilweise komplementären Primers,
d) Hybridisieren zumindest eines der Primer mit dem Ligationsprodukt;
e) Durchführen einer Polymerasekettenreaktion unter Verwendung des an das Ligationsprodukt hybridisierten Primers;

27. Verfahren nach Anspruch 26, **dadurch gekennzeichnet, dass** die Polymerasekettenreaktion gleichzeitig mit dem für das Oligonukleotid gemäß Schritt aa) und/oder ab) des Sloning-Verfahrens zumindest teilweise komplementären Primer und dem für das Oligonukleotid gemäß Schritt ab) und/oder bb) des Sloning-Verfahrens zumindest teilweise komplementären Primer durchgeführt wird.

28. Verfahren nach Anspruch 26 oder 27, **dadurch gekennzeichnet, dass** zumindest einer der Primer eine Modifizierung trägt, die eine Kopplung an eine feste Matrix erlaubt.

## Claims

1. A method for the production of a nucleic acid molecule comprising the steps
a) providing an oligonucleotide produced by the following steps:
aa) providing a partially double-stranded oligonucleotide with a 5'-overhand, containing a recognition site for a type IIS restriction enzyme which cuts outside of its recognition site, with the oligonucleotide carrying a modification which allows coupling to a solid matrix, whereby the 5'-overhang has a length of 3 nucleotides,
ab) addition of a further, at least partially double-stranded oligonucleotide with a 5'-overhang and a recognition site for a type IIS restriction enzyme which cuts outside of its recognition site, the recognition site being different from the one in step aa), whereby the 5'-overhang has a length of 3 nucleotides,
ac) ligation of the oligonucleotides from steps aa) and ab) in the orientation defined by the blocking of the ends not to be ligated,
ad) removing unconsumed reactants as well as enzymes,
ae) cleavage of the ligation product from step ac) with a type IIS restriction enzyme which cuts outside of its recognition site, whereby the cleavage occurs in the nucleic acid sequence of the oligonucleotide from step ab),
af) separating the reaction mixture from the oligonucleotide from step aa) which has been elongated and obtained in step ae),
b) providing a further oligonucleotide produced by the steps:
ba) providing a partially double-stranded oligonucleotide with a 5'-overhand, containing a recognition site for a type IIS restriction enzyme which cuts outside of its recognition site, with the oligonucleotide carrying a modification which allows coupling to a solid matrix, whereby the 5'-overhang has a length of 3 nucleotides,
bb) addition of a further, at least partially double-stranded oligonucleotide with a 5'-overhang and with a recognition site for a type IIS restriction enzyme which cuts outside of its recognition site, the recognition site being different from the one in step ba), whereby the 5'-overhang has a length of 3 nucleotides,
bc) ligation of the oligonucleotides from steps ba) and bb) in the orientation defined by the blocking of the ends not to be ligated,
bd) removing unconsumed reactants as well as enzymes,
be) cleavage of the ligation product from step bc) with a type IIS restriction enzyme, which cuts outside of its recognition site, whereby the cleavage occurs in the oligonucleotide in step bb),
bf) separating the thus elongated oligonucleotide from the reaction mixture,
c) ligation of the oligonucleotides from steps a) and b) in the orientation defined by the blocking of the ends not to be ligated,
d) removing unconsumed reactants as well as enzymes,
e) cleavage of the ligation product from step c) with a type IIS restriction enzyme which cuts outside of its recognition site, whereby the cleavage occurs in the oligonucleotide from steps a) or b),
f) separating the thus elongated nucleic acid molecule from the reaction mixture,
**characterized in that** the oligonucleotide from step ab) has the recognition site for a type IIS restriction enzyme which generates an overhang three nucleotides in length as long as steps ab) to ae) are repeated and the oligonucleotide from step ab) has the recognition site of a type IIS restriction enzyme which generates an overhang other than an overhang three nucleotides in length, in the last cycle of the steps ab) to ae) and/or
the oligonucleotide from step bb) has the recognition site for a type IIS restriction enzyme which generates an overhang three nucleotides in length, as long as steps bb) to be) are repeated and the oligonucleotide from step bb) has the recognition site of a type IIS restriction enzyme, which produces an overhang other than an overhang three nucleotides in length, in the last cycle of the steps bb) to be).

2. The method according to claim 1, **characterized in that**, following step af), steps ab) to af) are repeated at least once and/or, following step bf), steps bb) to be) are repeated at least once, whereby, following the final ligation in step bc) and the removing of unconsumed reactants as well as enzymes, the ligation product is cut with a type IIS restriction enzyme, whereby the cleavage occurs in the oligonucleotide from step ba).

3. A method for the production of a group of nucleic acid molecules comprising the steps
a) providing an oligonucleotide produced by the following steps:
aa) providing an oligonucleotide containing a recognition site for a type IIS restriction enzyme which cuts outside of its recognition site, with the oligonucleotide carrying a modification which allows coupling to a solid matrix,
coupling the oligonucleotide to the solid matrix,
ab) addition of a further, at least partially double-stranded oligonucleotide with a recognition site for a type IIS restriction enzyme which cuts outside of its recognition site, the recognition site being different from the one in step aa),
ac) ligation of the oligonucleotides from steps aa) and ab) in the orientation defined by the blocking of the ends not to be ligated,
ad) removing unconsumed reactants as well as enzymes,
ae) cleavage of the ligation product from step ac) with a type IIS restriction enzyme which cuts outside of its recognition site, whereby the cleavage occurs in the nucleic acid sequence of the oligonucleotide from step ab),
af) separating the reaction mixture from the oligonucleotide from step aa) which has been elongated and obtained in step ae),
b) providing a further oligonucleotide produced by the steps:
ba) providing an oligonucleotide containing a recognition site for a type IIS restriction enzyme which cuts outside of its recognition site, with the oligonucleotide carrying a modification which allows coupling to a solid matrix, with one end to a solid matrix,
coupling of the oligonucleotide to the solid matrix,
bb) addition of a further, at least partially double-stranded oligonucleotide with a recognition site for a type IIS restriction enzyme which cuts outside of its recognition site, the recognition site being different from the one in step ba),
bc) ligation of the oligonucleotides from steps ba) and bb) in the orientation defined by the blocking of the ends not to be ligated,
bd) removing unconsumed reactants as well as enzymes,
be) cleavage of the ligation product from step bc) with a type IIS restriction enzyme which cuts outside of its recognition site, whereby the cleavage occurs in the oligonucleotide in step bb),
bf) separating the thus elongated oligonucleotide from the reaction mixture,
c) ligation of the oligonucleotides from steps a) and b) in the orientation defined by the blocking of the ends not to be ligated,
d) removing unconsumed reactants as well as enzymes,
e) cleavage of the ligation product from step c) with a type IIS restriction enzyme, which cuts outside of its recognition site, whereby the cleavage occurs in the oligonucleotide from steps a) or b),
f) separating the thus elongated nucleic acid molecule from the reaction mixture,
**characterized in that** in the last repetition of steps ab) to af) the oligonucleotide added in step ab) carries a modification, which allows coupling to a solid matrix and
after the last repetition of steps ab) to af), as step ah), the ligation product from step ac) is cut with a type IIS restriction enzyme which cuts outside of its recognition site, whereby the cleavage occurs in the nucleic acid sequence of the oligonucleotide from step aa) and the cleavage product is released from the oligonucleotide coupled to the solid matrix,
and the released cleavage product is divided into at least two reactions.

4. The method according to claim 3, **characterized in that**, following step af), steps ab) to af) are repeated at least once and/or, following step bf), steps bb) to bf) are repeated at least once, whereby, following the final ligation in step bc) and the removing of unconsumed reactants as well as enzymes, the ligation product is cut with a type IIS restriction enzyme, whereby the cleavage occurs in the oligonucleotide from step ba).

5. The method according to claims 3 or 4, **characterized in that** the cleavage product released in step ah) is coupled to a solid matrix via the modification, and that the cleavage products that do not contain the modification are removed from the reaction.

6. The method according to claim 5, **characterized in that**, as step ai), in each of the reactions an oligonucleotide is added to the cleavage product coupled to the solid matrix from step ah), whereby the oligonucleotide contains a recognition site for a type IIS restriction enzyme which is different from the recognition site of a type IIS restriction enzyme of the cleavage product from step ah), with the oligonucleotide carrying a modification which allows coupling to a solid matrix, and, as step ak), the cleavage product from step ah) is ligated with the oligonucleotide.

7. The method according to claim 6, **characterized in that** the ligation product from step ak) is cut with a type IIS restriction enzyme which cuts outside of its recognition site, whereby the cleavage occurs in the nucleic acid sequence of the cleavage product from step ah).

8. The method according to claim 6 or 7, **characterized in that** the oligonucleotide added in step ai) has a different sequence in each reaction.

9. The method according to any of claims 3 to 8, **characterized in that** the oligonucleotides added to the reactions have identical single-stranded overhangs.

10. The method according to any of claims 3 to 9, **characterized in that** the oligonucleotides differ in a region that is different from the single-stranded region, preferably in a region comprising a sequence of nucleotides following the single-stranded region of the oligonucleotide.

11. The method according to claim 10, **characterized in that** the sequence of nucleotides has a length of 1 to 10 nucleotides, preferably 3 to 6 nucleotides.

12. The method according to any of claims 3 to 11, **characterized in that**, in a step al), the oligonucleotide added in step ai) is cleaved.

13. The method according to claim 12, **characterized in that** the steps ab) to ak) and/or al) are repeated at least once.

14. The method according to claim 13, **characterized in that**, after the last repetition, the oligonucleotides coupled to the solid phases are ligated, as step am), with a further oligonucleotide according to ab), whereby these oligonucleotides in the different reactions differ from each other in the sequence of the single-stranded overhang and optionally in the sequence of the directly abutting double-stranded region.

15. A method for the production of a nucleic acid molecule comprising the steps
a) providing an oligonucleotide produced by the following steps:
aa) providing an oligonucleotide containing a recognition site for a type IIS restriction enzyme which cuts outside of its recognition site, with the oligonucleotide carrying a modification, which allows coupling to a solid matrix,
coupling the oligonucleotide to the solid matrix,
ab) addition of a further, at least partially double-stranded oligonucleotide with a recognition site for a type IIS restriction enzyme which cuts outside of its recognition site, the recognition site being different from the one in step aa),
ac) ligation of the oligonucleotides from steps aa) and ab) in the orientation defined by the blocking of the ends not to be ligated,
ad) removing unconsumed reactants as well as enzymes,
ae) cleavage of the ligation product from step ac) with a type IIS restriction enzyme which cuts outside of its recognition site, whereby the cleavage occurs in the nucleic acid sequence of the oligonucleotide from step ab),
af) separating the reaction mixture from the oligonucleotide from step aa) which has been elongated and obtained in step ae),
b) providing a further oligonucleotide produced by the steps:
ba) providing an oligonucleotide containing a recognition site for a type IIS restriction enzyme which cuts outside of its recognition site, with the oligonucleotide carrying a modification, which allows coupling to a solid matrix, with one end to the solid matrix,
coupling of the oligonucleotide to the solid matrix,
bb) addition of a further, at least partially double-stranded oligonucleotide with a recognition site for a type IIS restriction enzyme which cuts outside of its recognition site, the recognition site being different from the one in step ba),
bc) ligation of the oligonucleotides from steps ba) and bb) in the orientation defined by the blocking of the ends not to be ligated,
bd) removing unconsumed reactants as well as enzymes,
be) cleavage of the ligation product from step bc) with a type IIS restriction enzyme which cuts outside of its recognition site, whereby the cleavage occurs in the oligonucleotide from step bb),
bf) separating the thus elongated nucleic acid molecule from the reaction mixture,
c) ligation of the oligonucleotides from steps a) and b) in the orientation defined by the blocking of the ends not to be ligated,
d) removing unconsumed reactants as well as enzymes,
e) cleavage of the ligation product from step c) with a type IIS restriction enzyme, which cuts outside of its recognition site, whereby the cleavage occurs in the oligonucleotide from steps a) or b),
f) separating the thus elongated nucleic acid molecule from the reaction mixture,
**characterized in that**, in the last repetition of steps ab) to af), the oligonucleotide added in step ab) carries a modification which allows coupling to a solid matrix.

16. The method according to claim 15, **characterized in that**, following step af), steps ab) to af) are repeated at least once and/or, following step bf), steps bb) to bf) are repeated at least once, whereby, following the final ligation in step bc) and the removing of unconsumed reactants as well as enzymes, the ligation product is cut with a type IIS restriction enzyme, whereby the cleavage occurs in the oligonucleotide from step ba).

17. The method according to claims 15 or 16, **characterized in that**, after the last repetition of steps ab) to af), the ligation product from step ac) is cut, as step ah), with a type IIS restriction enzyme which cuts outside of its recognition site, whereby the cleavage occurs in the oligonucleotide from step aa) and the cleavage product is released from the oligonucleotide coupled to the solid matrix.

18. The method according to claim 17, **characterized in that** the cleavage product released in step ah) is coupled, in a new reaction vessel, to a solid matrix via the modification, and the cleavage products that do not contain a modification are removed from the reaction containing the solid matrix and the cleavage product released in step ah).

19. The method according to claim 18, **characterized in that**, to the cleavage product coupled to the solid matrix from step ah), an oligonucleotide is added which contains a recognition site for a type IIS restriction enzyme that is different from the recognition site for a type IIS restriction enzyme of the cleavage product from step ah), the oligonucleotide carrying a modification which allows coupling to a solid matrix, and that, as step ai), the cleavage product from step ah) is ligated with the oligonucleotide.

20. The method according to claim 19, **characterized in that** the ligation product from step ai) is cut with a type IIS restriction enzyme which cuts outside of its recognition site, whereby the cleavage occurs in the nucleic acid sequence of the cleavage product from step ah).

21. The method according to any one of claims 15 to 20, **characterized in that** the steps are repeated using one or more modified oligonucleotides according to step ab).

22. A method for the production of a nucleic acid molecule comprising the steps
a) providing an oligonucleotide produced by the following steps:
aa) providing an oligonucleotide containing a recognition site for a type IIS restriction enzyme which cuts outside of its recognition site,
ab) addition of a further, at least partially double-stranded oligonucleotide with a recognition site for a type IIS restriction enzyme which cuts outside of its recognition site, the recognition site being different from the one in step aa), with the oligonucleotide carrying a modification allowing coupling to a solid matrix,
ac) ligation of the oligonucleotides from steps aa) and ab) in the orientation defined by the blocking of the ends not to be ligated,
ad) cleavage of the ligation product from step ac) with a type IIS restriction enzyme which cuts outside of its recognition site, whereby the cleavage occurs in the nucleic acid sequence of the oligonucleotide from step ab),
af) separating from the reaction mixture the cleavage product from step ae) that does not carry a modification,
b) providing a further oligonucleotide produced by the steps:
ba) providing an oligonucleotide containing a recognition site for a type IIS restriction enzyme,
bb) addition of a further, at least partially double-stranded oligonucleotide with a recognition site for a type IIS restriction enzyme which cuts outside of its recognition site, the recognition site being different from the one in step ba), with the oligonucleotide carrying a modification which allows coupling to a solid matrix,
bc) ligation of the oligonucleotides from steps ba) and bb) in the orientation defined by the blocking of the ends not to be ligated,
be) cleavage of the ligation product from step bc) with a type IIS restriction enzyme which cuts outside of its recognition site, whereby the cleavage occurs in the oligonucleotide from step bb),
bf) separating from the reaction mixture the cleavage product from step be) that does not carry a modification,
c) ligation of the oligonucleotides from steps a) and b) in the orientation defined by the blocking of the ends not to be ligated,
d) removing and/or inactivating unconsumed reactants as well as enzymes,
e) cleavage of the ligation product from step c) with a type IIS restriction enzyme which cuts outside of its recognition site, whereby the cleavage occurs in the oligonucleotide from steps a) or b),
f) separating the thus elongated nucleic acid molecule from the reaction mixture,

23. The method according to claim 22, **characterized in that**, following step ac), as step ad), unconsumed reactants as well as enzymes are removed and/or, following step af), steps ab) to af) are repeated at least once and/or, following step bf), steps bb) to bf) are repeated at least once, whereby, following the final ligation in step bc) and the removing and/or inactivating of unconsumed reactants as well as enzymes, the ligation product is cut using a type IIS restriction enzyme, whereby the cleavage occurs in the oligonucleotide from step ba).

24. The method according to claims 22 or 23, further comprising the step of transferring cleavage products that do not contain a modification to a new reaction.

25. The method according to any of claims 22 to 24, whereby step bd) follows step bc), consisting of:
bd) Removing and/or inactivating unconsumed reactants as well as enzymes.

26. The method according to any of claims 1 to 25, further comprising a method for the amplification of a ligation product being generated, **characterized by** the steps:
a) providing a ligation product;
b) providing a primer which is at least partially complementary to the oligonucleotide according to steps aa) and/or ab),
c) providing a primer which is at least partially complementary to the oligonucleotide according to steps ab) and/or bb),
d) hybridizing at least one of the primers with the ligation product;
e) performing a polymerase chain reaction using the primer hybridized to the ligation product.

27. The method according to claim 26, **characterized in that** the polymerase chain reaction is carried out simultaneously with the primer which is at least partially complementary to the oligonucleotide according to steps aa) and/or ab) of the Sloning method and the primer which is at least partially complementary to the oligonucleotide according to steps ab) and/or bb) of the Sloning method.

28. The method according to claim 26 or 27, **characterized in that** at least one of the primers carries a modification which allows coupling to a solid matrix.

## Revendications

1. Procédé de production d'une molécule d'acide nucléique comprenant les étapes consistant à
a) mettre à disposition un oligonucléotide qui est produit par les étapes suivantes :
aa) mise à disposition d'un oligonucléotide en partie à double brin avec un prolongement en 5' qui contient une séquence d'identification d'une enzyme de restriction de type IIS qui clive en dehors de sa séquence d'identification, et
porte une modification qui permet un couplage à une matrice solide, le prolongement 5' comprenant une longueur de 3 nucléotides,
ab) addition d'un autre oligonucléotide au moins en partie à double brin comportant un prolongement en 5' et une autre séquence d'identification d'une enzyme de restriction de type IIS qui clive en dehors de sa séquence d'identification, comme à l'étape aa), le prolongement 5' comprenant une longueur de 3 nucléotides,
ac) ligature des oligonucléotides des étapes aa) et ab) dans l'orientation déterminée par le blocage des extrémités qui ne sont pas à lier,
ad) élimination des réactifs non utilisés ainsi que de l'enzyme,
ae) clivage du produit de ligature de l'étape ac) avec une enzyme de restriction de type IIS qui clive en dehors de sa séquence d'identification, le clivage s'effectuant dans la séquence d'acide nucléique de l'oligonucléotide de l'étape ab)
af) séparation du mélange réactionnel de l'oligonucléotide prolongé de l'étape aa) obtenu à l'étape ae),
b) mettre à disposition un autre oligonucléotide qui est produit par les étapes de:
ba) mise à disposition d'un oligonucléotide en partie à double brin avec un prolongement en 5' qui contient une séquence d'identification d'une enzyme de restriction de type IIS qui clive en dehors de sa séquence d'identification, et porte une modification qui permet un couplage à une matrice solide, le prolongement 5' comprenant une longueur de 3 nucléotides,
bb) addition d'un autre oligonucléotide au moins en partie à double brin comportant un prolongement en 5' et une autre séquence d'identification d'une enzyme de restriction de type IIS qui clive en dehors de sa séquence d'identification, comme à l'étape ba), le prolongement 5' comprenant une longueur de 3 nucléotides,
bc) ligature des oligonucléotides des étapes ba) et bb) dans l'orientation déterminée par le blocage des extrémités qui ne sont pas à lier,
bd) élimination des réactifs non utilisés ainsi que de l'enzyme,
be) clivage du produit de ligature de l'étape bc) avec une enzyme de restriction de type IIS qui clive en dehors de sa séquence d'identification, le clivage s'effectuant dans l'oligonucléotide de l'étape bb)
bf) séparation de la molécule d'acide nucléique ainsi prolongée du mélange réactionnel,
c) lier les oligonucléotides de l'étape a) et b) dans l'orientation déterminée par le blocage des extrémités qui ne sont pas à lier,
d) éliminer les réactifs non utilisés ainsi que les enzymes,
e) cliver le produit de ligature de l'étape c) avec une enzyme de restriction de type IIS qui clive en dehors de sa séquence d'identification, le clivage étant réalisé dans l'oligonucléotide de l'étape a) ou b),
f) séparer la molécule d'acide nucléique ainsi prolongée du mélange réactionnel, **caractérisé en ce que** l'oligonucléotide de l'étape ab) présente la séquence d'identification d'une enzyme de restriction de type IIS qui génère un prolongement d'une longueur de trois nucléotides tant que les étapes ab) à ae) sont répétées et l'oligonucléotide de l'étape ab) présente la séquence d'identification d'une enzyme de restriction de type IIS qui génère un autre prolongement d'une longueur de trois nucléotides lors de la dernière réalisation des étapes ab) à ae) et/ou
l'oligonucléotide de l'étape bb) présente la séquence d'identification d'une enzyme de restriction de type IIS qui génère un prolongement d'une longueur de trois nucléotides tant que les étapes bb) à be) sont répétées et
l'oligonucléotide de l'étape bb) présente la séquence d'identification d'une enzyme de restriction de type IIS qui génère un autre prolongement d'une longueur de trois nucléotides lors de la dernière réalisation des étapes bb) à be).

2. Procédé selon la revendication 1, **caractérisé en ce que**, à la suite de l'étape af) les étapes ab) à af) sont répétées au moins une fois et/ou à la suite de l'étape bf) les étapes bb) à be) sont répétées au moins une fois, à la fin de la dernière ligature de l'étape c) et de l'élimination des réactifs non utilisés et de l'enzyme, le produit de ligature étant clivé avec une enzyme de restriction de type IIS, le clivage étant réalisé dans l'oligonucléotide de l'étape ba).

3. Procédé de production d'un groupe de molécules d'acide nucléique comprenant les étapes consistant à
a) mettre à disposition un oligonucléotide qui est produit par les étapes suivantes :
aa) mise à disposition d'un oligonucléotide qui contient une séquence d'identification d'une enzyme de restriction de type IIS qui clive en dehors de sa séquence d'identification, et porte une modification qui permet un couplage à une matrice solide,
couplage de l'oligonucléotide à la matrice solide
ab) addition d'un autre oligonucléotide au moins en partie à double brin avec une autre séquence d'identification d'une enzyme de restriction de type IIS qui clive en dehors de sa séquence d'identification, comme à l'étape aa),
ac) ligature des oligonucléotides des étapes aa) et ab) dans l'orientation déterminée par le blocage des extrémités qui ne sont pas à lier,
ad) élimination des réactifs non utilisés ainsi que de l'enzyme,
ae) clivage du produit de ligature de l'étape ac) avec une enzyme de restriction de type IIS qui clive en dehors de sa séquence d'identification, le clivage s'effectuant dans la séquence d'acide nucléique de l'oligonucléotide de l'étape ab)
af) séparation du mélange réactionnel de l'oligonucléotide prolongé de l'étape aa) obtenu à l'étape ae),
b) mettre à disposition un autre oligonucléotide qui est produit par les étapes de:
ba) mise à disposition d'un oligonucléotide qui contient une séquence d'identification d'une enzyme de restriction de type IIS qui clive en dehors de sa séquence d'identification, et porte une modification qui permet un couplage à une matrice solide, avec une extrémité à une matrice solide,
couplage de l'oligonucléotide à la matrice solide,
bb) addition d'un autre oligonucléotide au moins en partie à double brin avec une autre séquence d'identification d'une enzyme de restriction de type IIS qui clive en dehors de sa séquence d'identification, comme à l'étape ba),
bc) ligature des oligonucléotides des étapes ba) et bb) dans l'orientation déterminée par le blocage des extrémités qui ne sont pas à lier,
bd) élimination des réactifs non utilisés ainsi que de l'enzyme,
be) clivage du produit de ligature de l'étape bc) avec une enzyme de restriction de type IIS qui clive en dehors de sa séquence d'identification, le clivage s'effectuant dans l'oligonucléotide de l'étape bb)
bf) séparation de la molécule d'acide nucléique ainsi prolongée du mélange réactionnel,
c) lier les oligonucléotides de l'étape a) et b) dans l'orientation déterminée par le blocage des extrémités qui ne sont pas à lier,
d) éliminer les réactifs non utilisés ainsi que les enzymes,
e) cliver le produit de ligature de l'étape c) avec une enzyme de restriction de type IIS qui clive en dehors de sa séquence d'identification, le clivage étant réalisé dans l'oligonucléotide de l'étape a) ou b),
f) séparer la molécule d'acide nucléique ainsi prolongée du mélange réactionnel,
**caractérisé en ce que** lors de la dernière répétition des étapes ab) à af) l'oligonucléotide de l'étape ab) porte une modification qui permet un couplage à une matrice solide et
après la dernière répétition des étapes ab) à af) en tant qu'étape ah), le produit de ligature de l'étape ac) est clivé avec une enzyme de restriction de type IIS qui clive en dehors de sa séquence d'identification, le clivage étant réalisé dans la séquence d'acide nucléique de l'oligonucléotide de l'étape aa) et le produit de ligature étant libéré de l'oligonucléotide couplé à la matrice solide,
et le produit de clivage libéré étant réparti dans au moins deux préparations réactionnelles.

4. Procédé selon la revendication 3, **caractérisé en ce que**, à la suite de l'étape af) les étapes ab) à af) sont répétées au moins une fois et/ou à la suite de l'étape bf) les étapes bb) à bf) sont répétées au moins une fois, à la fin de la dernière ligature de l'étape bc) et de l'élimination des réactifs non utilisés et de l'enzyme, le produit de ligature étant clivé avec une enzyme de restriction de type IIS, le clivage étant réalisé dans l'oligonucléotide de l'étape ba).

5. Procédé selon la revendication 3 ou 4, **caractérisé en ce que** le produit de clivage libéré à l'étape ah) est couplé par la modification à une matrice solide et les produits de clivage ne présentant pas de modification sont éliminés de la préparation réactionnelle.

6. Procédé selon la revendication 5, **caractérisé en ce que**, dans chacune des préparations réactionnelles, un oligonucléotide qui contient une séquence d'identification d'une enzyme de restriction de type IIS qui est différente de la séquence d'identification d'une enzyme de restriction de type IIS du produit de clivage de l'étape ah) est ajouté au produit de clivage couplé à la matrice solide de l'étape ah) en tant qu'étape ai), et porte une modification qui permet un couplage à une matrice solide et en tant qu'étape ak), une ligature du produit de clivage de l'étape ah) étant réalisée avec l'oligonucléotide.

7. Procédé selon la revendication 6, **caractérisé en ce que** le produit de ligature de l'étape ak) est clivé avec une enzyme de restriction de type IIS qui clive en dehors de sa séquence de reconnaissance, le clivage étant réalisé dans la séquence d'acide nucléique du produit de clivage de l'étape ah).

8. Procédé selon la revendication 6 ou 7, **caractérisé en ce que** l'oligonucléotide ajouté à l'étape ai) présente une autre séquence dans chaque préparation réactionnelle.

9. Procédé selon l'une quelconque des revendications 3 à 8, **caractérisé en ce que** les oligonucléotides ajoutés dans les préparations réactionnelles présentent des prolongement à brin simples identiques.

10. Procédé selon l'une quelconque des revendications 3 à 9, **caractérisé en ce que** les oligonucléotides se distinguent dans une région qui est différente de la région à brin simple, de préférence se distinguent dans une région qui présente une suite de nucléotides qui s'adjoint à la région à brin simple de l'oligonucléotide.

11. Procédé selon la revendication 10, **caractérisé en ce que** la suite de nucléotides présente une longueur de 1 à 10 nucléotides, de préférence de 3 à 6 nucléotides.

12. Procédé selon l'une quelconque des revendications 3 à 11, **caractérisé en ce que** dans une étape al) s'effectue un clivage de l'oligonucléotide ajouté à l'étape ai).

13. Procédé selon la revendication 12, **caractérisé en ce que** les étapes ab) à ak) et/ou al) sont répétées au moins une fois.

14. Procédé selon la revendication 13, **caractérisé en ce qu'**après la dernière répétition en tant qu'étape am), les oligonucléotides couplés aux phases solides sont liés avec un autre oligonucléotide selon l'étape ab), ces oligonucléotides se distinguant les uns des autres dans les différentes préparations réactionnelles dans la séquence du prolongement à brin simple et éventuellement dans la séquence de la région directement adjacente à double brin.

15. Procédé de production d'une molécule d'acide nucléique comprenant les étapes consistant à :
a) mettre à disposition un oligonucléotide qui est produit par les étapes suivantes :
aa) mise à disposition d'un oligonucléotide qui contient une séquence d'identification d'une enzyme de restriction de type IIS qui clive en dehors de sa séquence d'identification, et porte une modification qui permet un couplage à une matrice solide,
couplage de l'oligonucléotide à la matrice solide
ab) addition d'un autre oligonucléotide au moins en partie à double brin avec une autre séquence d'identification d'une enzyme de restriction de type IIS qui clive en dehors de sa séquence d'identification, comme à l'étape aa),
ac) ligature des oligonucléotides des étapes aa) et ab) dans l'orientation déterminée par le blocage des extrémités qui ne sont pas à lier,
ad) élimination des réactifs non utilisés ainsi que de l'enzyme,
ae) clivage du produit de ligature de l'étape ac) avec une enzyme de restriction de type IIS qui clive en dehors de sa séquence d'identification, le clivage s'effectuant dans la séquence d'acide nucléique de l'oligonucléotide de l'étape ab)
af) séparation du mélange réactionnel de l'oligonucléotide prolongé de l'étape aa) obtenu à l'étape ae),
b) mettre à disposition un autre oligonucléotide qui est produit par les étapes de:
ba) mise à disposition d'un oligonucléotide qui contient une séquence d'identification d'une enzyme de restriction de type IIS qui clive en dehors de sa séquence d'identification, et porte une modification qui permet un couplage à une matrice solide, avec une extrémité à une matrice solide
couplage de l'oligonucléotide à la matrice solide,
bb) addition d'un autre oligonucléotide au moins en partie à double brin avec une autre séquence d'identification d'une enzyme de restriction de type IIS qui clive en dehors de sa séquence d'identification, comme à l'étape ba),
bc) ligature des oligonucléotides des étapes ba) et bb) dans l'orientation déterminée par le blocage des extrémités qui ne sont pas à lier,
bd) élimination des réactifs non utilisés ainsi que de l'enzyme,
be) clivage du produit de ligature de l'étape bc) avec une enzyme de restriction de type IIS qui clive en dehors de sa séquence d'identification, le clivage s'effectuant dans l'oligonucléotide de l'étape bb),
bf) séparation de la molécule d'acide nucléique ainsi prolongée du mélange réactionnel,
c) lier les oligonucléotides de l'étape a) et b) dans l'orientation déterminée par le blocage des extrémités qui ne sont pas à lier,
d) éliminer les réactifs non utilisés ainsi que les enzymes,
e) cliver le produit de ligature de l'étape c) avec une enzyme de restriction de type IIS qui clive en dehors de sa séquence d'identification, le clivage étant réalisé dans l'oligonucléotide de l'étape a) ou b),
f) séparer la molécule d'acide nucléique ainsi prolongée du mélange réactionnel,
**caractérisé en ce que** lors de la dernière répétition des étapes ab) à af), l'oligonucléotide ajouté à l'étape ab) porte une modification qui permet un couplage à une matrice solide.

16. Procédé selon la revendication 15, **caractérisé en ce que**, à la suite de l'étape af) les étapes ab) à af) sont répétées au moins une fois et/ou à la suite de l'étape bf) les étapes bb) à bf) sont répétées au moins une fois, à la fin de la dernière ligature de l'étape bc) et de l'élimination des réactifs non utilisés et de l'enzyme, le produit de ligature étant clivé avec une enzyme de restriction de type IIS, le clivage étant réalisé dans l'oligonucléotide de l'étape ba).

17. Procédé selon la revendication 15 ou 16, **caractérisé en ce que**, après la dernière répétition des étapes ab) à af) en tant qu'étape ah), le produit de ligature de l'étape ac) est clivé avec une enzyme de restriction de type IIS qui clive en dehors de sa séquence d'identification, le clivage étant réalisé dans l'oligonucléotide de l'étape aa) et le produit de ligature étant libéré de l'oligonucléotide couplé à la matrice solide.

18. Procédé selon la revendication 17, **caractérisé en ce que** le produit de clivage libéré à l'étape ah) est couplé dans un nouveau récipient réactionnel par la modification à une matrice solide et les produits de clivage ne présentant pas de modification sont éliminés de la préparation réactionnelle contenant la matrice solide et le produit de clivage libéré à l'étape ah).

19. Procédé selon la revendication 18, **caractérisé en ce que** l'on ajoute au produit de clivage couplé à la matrice solide de l'étape ah), un oligonucléotide qui contient une séquence d'identification d'une enzyme de restriction de type IIS qui est différente de la séquence d'identification d'une enzyme de restriction de type IIS du produit de clivage de l'étape ah) et porte une modification qui permet un couplage à une matrice solide et en tant qu'étape ai), une ligature du produit de clivage de l'étape ah) avec l'oligonucléotide est réalisée.

20. Procédé selon la revendication 19, **caractérisé en ce que** le produit de ligature de l'étape ai) est clivé avec une enzyme de restriction de type IIS qui clive en dehors de sa séquence de reconnaissance, le clivage étant réalisé dans la séquence d'acide nucléique du produit de clivage de l'étape ah).

21. Procédé selon l'une quelconque des revendications 15 à 20, **caractérisé en ce que** les étapes sont répétées en utilisant un ou plusieurs oligonucléotides modifiés selon l'étape ab).

22. Procédé de production d'une molécule d'acide nucléique comprenant les étapes consistant à :
a) mettre à disposition un oligonucléotide qui est produit par les étapes suivantes :
aa) mise à disposition d'un oligonucléotide qui contient une séquence d'identification d'une enzyme de restriction de type IIS qui clive en dehors de sa séquence d'identification,
ab) addition d'un autre oligonucléotide au moins en partie à double brin avec une autre séquence d'identification d'une enzyme de restriction de type IIS qui clive en dehors de sa séquence d'identification, comme à l'étape aa), et porte une modification qui permet un couplage à une matrice solide,
ac) ligature des oligonucléotides des étapes aa) et ab) dans l'orientation déterminée par le blocage des extrémités qui ne sont pas à lier,
ad) clivage du produit de ligature de l'étape ac) avec une enzyme de restriction de type IIS qui clive en dehors de sa séquence d'identification, le clivage s'effectuant dans la séquence d'acide nucléique de l'oligonucléotide de l'étape ab)
af) séparation du produit de clivage de l'étape ae) qui ne porte pas de modification,
b) mettre à disposition un autre oligonucléotide qui est produit par les étapes de:
ba) mise à disposition d'un oligonucléotide qui contient une séquence d'identification d'une enzyme de restriction de type IIS,
bb) addition d'un autre oligonucléotide au moins en partie à double brin avec une autre séquence d'identification d'une enzyme de restriction de type IIS qui clive en dehors de sa séquence d'identification, comme à l'étape ba), et porte une modification qui permet un couplage à une matrice solide,
bc) ligature des oligonucléotides des étapes ba) et bb) dans l'orientation déterminée par le blocage des extrémités qui ne sont pas à lier,
be) clivage du produit de ligature de l'étape bc) avec une enzyme de restriction de type IIS qui clive en dehors de sa séquence d'identification, le clivage s'effectuant dans l'oligonucléotide de l'étape bb),
bf) séparation du produit de clivage qui ne porte pas de modification de l'étape be) du mélange réactionnel,
c) lier les oligonucléotides de l'étape a) et b) dans l'orientation déterminée par le blocage des extrémités qui ne sont pas à lier,
d) éliminer et/ou inactivation des réactifs non utilisés ainsi que des enzymes,
e) cliver le produit de ligature de l'étape c) avec une enzyme de restriction de type IIS qui clive en dehors de sa séquence d'identification, le clivage étant réalisé dans l'oligonucléotide de l'étape a) ou b),
f) séparer la molécule d'acide nucléique ainsi prolongée du mélange réactionnel.

23. Procédé selon la revendication 22, **caractérisé en ce que** à la suite de l'étape ac) en tant qu'étape ad), les réactifs non utilisés et l'enzyme sont éliminés et/ou à la suite de l'étape af) les étapes ab) à af) sont répétées au moins une fois et/ou à la suite de l'étape bf) les étapes bb à bf sont répétées, au moins une fois à la fin de la dernière ligature de l'étape bc) et de l'élimination et/ou de l'inactivation des réactifs non utilisés et de l'enzyme, le produit de ligature étant clivé avec une enzyme de restriction de type IIS, le clivage étant réalisé dans l'oligonucléotide de l'étape ba).

24. Procédé selon la revendication 22 ou 23, comprenant en outre l'étape selon laquelle les produits de clivage qui ne portent pas de modification sont transférés dans une nouvelle préparation réactionnelle.

25. Procédé selon l'une quelconque des revendications 22 à 24, dans lequel après l'étape bc) l'étape bd suit, comprenant :
bd) l'élimination et/ou l'inactivation des réactifs non utilisés et de l'enzyme.

26. Procédé selon l'une quelconque des revendications 1 à 25 comprenant en outre un procédé d'amplification du produit de ligature obtenu, **caractérisé par** les étapes de
a) mise à disposition d'un produit de ligature ;
b) mise à disposition d'une amorce au moins en partie complémentaire pour l'oligonucléotide selon l'étape aa) et/ou ab),
c) mise à disposition d'une amorce au moins en partie complémentaire pour l'oligonucléotide selon l'étape ab) et/ou l'étape bb),
d) hybridation d'au moins l'une des amorces avec le produit de ligature ;
e) réalisation d'une réaction de polymérase en utilisant l'amorce hybridée au produit de ligature.

27. Procédé selon la revendication 26, **caractérisé en ce que** la réaction en chaîne polymérase est réalisée en même temps avec l'amorce au moins complémentaire pour l'oligonucléotide selon l'étape aa) et/ou ab) du procédé de sloning et avec l'amorce au moins complémentaire pour l'oligonucléotide du procédé de sloning selon l'étape ab) et/ou bb).

28. Procédé selon la revendication 26 ou 27, **caractérisé en ce que** au moins l'une des amorces porte une modification qui permet un couplage à une matrice solide.
